# EUROPEAN PATENT SPECIFICATION

(11) **EP 3 094 726 B1**
(45) Date of publication and mention of the grant of the patent: **12.12.2018**
(21) Application number: 15704071.8
(22) Date of filing: 13.01.2015
(51) Int. Cl.: C12N 15/113, A61K 39/395, A61K 39/00, C07K 16/18

(54) **BIOLOGICAL MATERIALS AND THERAPEUTIC USES THEREOF**
BIOLOGISCHE MATERIALIEN UND THERAPEUTISCHE VERWENDUNG DAVON
MATÉRIELS BIOLOGIQUES ET LEURS UTILISATIONS THÉRAPEUTIQUES

(30) Priority: 13.01.2014 GB 201400517
(43) Date of publication of application: 23.11.2016
(73) Proprietor: Imperial Innovations Ltd, London SW7 2PG (GB)
(72) Inventor: MIDWOOD, Kim Suzanne, Chesham Buckinghamshire HP5 2JF (GB); VENABLES, Patrick John, Epsom Surrey KT19 8LE (GB)
(74) Representative: Sterling IP
(86) International application number: PCT/GB2015/050053
(87) International publication number: WO 2015/104564

(56) References cited:
- WO-A1-2008/092894
- WO-A1-2010/103289
- WO-A1-2010/103289
- CALVO A ET AL: "Identification of VEGF-regulated genes associated with increased lung metastatic potential: functional involvement of tenascin-C in tumor growth and lung metastasis", September 2008 (2008-09), ONCOGENE, VOL. 27, NR. 40, PAGE(S) 5373-5384, XP002741771, ISSN: 0950-9232 page 5376, right-hand column, paragraph 3 page 5383, right-hand column, paragraph 1 figure 3d

## Description

The present invention relates to citrullinated tenascin-C and its activity in chronic inflammation. There is also provided modulators of citrullinated tenascin-C and its biological activity.

Inflammation is the complex biological response of tissues to harmful stimuli, such as pathogens, tissue damage, or irritants. It is a protective attempt by the tissue to remove the injurious stimuli as well as initiate the healing process for the tissue. Abnormalities associated with inflammation comprise a large, unrelated group of disorders which underlie a variety of human diseases (inflammatory disorders). Examples of diseases with an inflammatory aspect include (but are not limited to) asthma, autoimmune disease, glomerulonephritis, allergy (hypersensitivities), inflammatory bowel diseases, reperfusion injury, rheumatoid arthritis and transplant rejection.

In particular, chronic inflammation is a debilitating and serious condition associated with many of the above diseases and is characterised by persistent inflammation and/or altered immune responses such as in autoimmune disease.

Rheumatoid arthritis (RA) is a typical example of, though by no means the only, chronic inflammatory condition. RA is characterized by synovial inflammation and destruction of joint cartilage and bone mediated by persistent synthesis of pro-inflammatory cytokines and matrix metalloproteinases (MMPs). Biological compounds that suppress the synthesis of inflammatory cytokines such as TNFα and IL-6 are successful at treating RA in the short-term. However, repeated treatments are required, which renders this an expensive therapeutic approach, and does not provide long-term remission. Furthermore, total systemic suppression of cytokine function is not without inherent problems such as increased infectious risk. Thus, despite advances in care, there remains an unmet need for an economical mode of treatment of chronic inflammatory that is efficacious over the long term (Smolen (2006) and Williams (2007)).

The mechanisms that underpin disease chronicity remain unclear and the factor(s) that drive the prolonged expression of inflammatory and destructive mediators are currently unknown.

Toll-like receptors (TLRs) play a key role in driving the production of inflammatory mediators in RA and blockade of TLR function may be of significant clinical benefit (reviewed in Brentano (2005) and O'Neill (2002)). This receptor family forms an integral part of the immune system. TLRs mediate host defence against infection and injury by recognising both pathogen-associated molecular patterns (PAMPs) and damage-associated molecular patterns (DAMPs) (Matzinger (2002)). DAMPs are endogenous pro-inflammatory molecules generated upon tissue injury and include intracellular molecules released from damaged or necrotic cells, fragments of extracellular matrix (ECM) molecules or ECM molecules up regulated upon injury (reviewed in Bianchi (2007) and Gordon (2002)).

Upon activation, TLRs promote both innate and adaptive immune responses including stimulation of expression of pro-inflammatory cytokines and MMPs (Medzhitov (2002)). TLRs are expressed at high levels in synovial tissue from RA patients (Radstake (2004), Roelofs (2005), Sacre (2007), and (Sacre (2008) and mice with targeted deletions or loss of function mutations in TLR4 are protected from experimental arthritis (Choe (2003) and Lee (2005). Furthermore, inhibitors of TLR4 can reduce destructive arthritis in mice (Abdollahi-Roodsaz (2007)) and a putative TLR4 inhibitor improved symptoms in 15 out of 23 patients with moderate to severe RA in a preliminary phase I trial (Vanags (2006). However, it is unclear which TLR ligand(s) are involved in disease pathogenesis.

Tenascin-C is an ECM glycoprotein that is associated with tissue injury and wound repair. Tenascin-C is expressed specifically at during active tissue remodelling during embryogenesis, being first observed during gastrulation and somite formation. In later stages of development expression is restricted to sites of branching morphogenesis of mammary gland and the lung, in the developing skeleton, cardiovascular system and in connective tissues at sites of epithelial to mesenchymal transformation. Expression is down regulated once these processes cease and before embryogenesis is complete (Jones (2000)).

Tenascin-C is not normally expressed in healthy adult tissue but, in adults, is specifically and transiently up-regulated during acute inflammation and persistently expressed in chronic inflammation (reviewed in Chiquet-Ehrismann (2003)). Immunohistochemical studies show that little tenascin-C is expressed in normal human joints but levels are greatly increased in RA synovia, in areas of inflammation and fibrosis, specifically below the synovial lining, in the invading pannus and around blood vessels (Cutolo (1992), MacCachren (1992) and Salter (1993)). There is also a significant increase in tenascin-C levels in synovial fluid from RA patients (Chevalier (1994) and Hasegawa (2007)) and in RA cartilage (Salter (1993) and Chevalier (1994)).

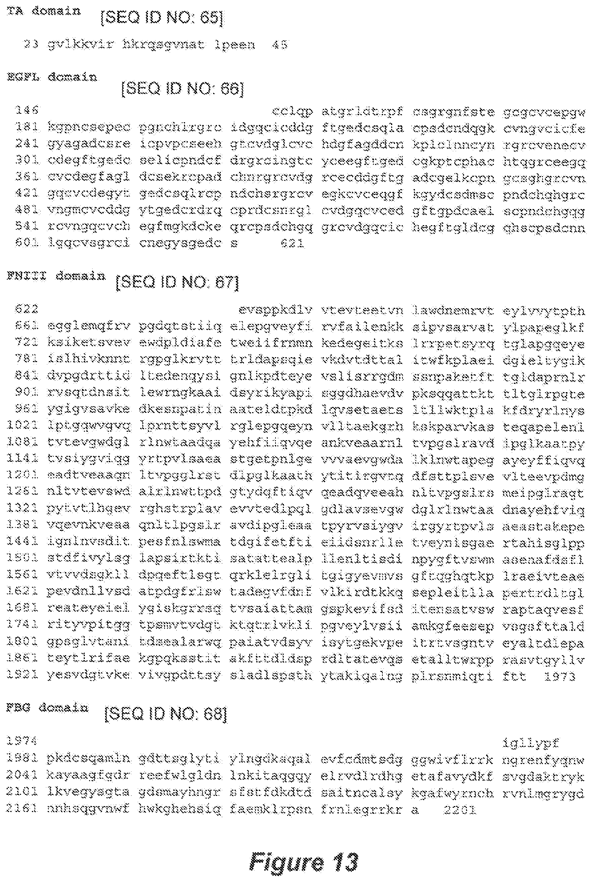

Tenascin-C is a large hexameric protein of 1.5 million Da. Each chain comprises different domains, including an assembly domain (TA), EGF-like repeats (EGF-L), fibronectin type III-like repeats (TNIII) and a fibrinogen-like globe (FBG) (reviewed in Orend (2005)). The sequences of tenascin-C and its domains are shown in Figure 13.

The inventors have shown previously that tenascin-C is an endogenous TLR4 ligand that it is required for destructive joint inflammation observed in arthritis and is involved in the prolonging of the inflammatory response characterising the chronic inflammatory condition. In particular, tenascin-C has been shown to be an endogenous activator of TLR4 and demonstrated that this molecule is required for destructive joint inflammation (WO 2010/103289).

In WO 2010/103289, a role for tenascin-C in mediating an immune response in the joint was demonstrated by induction of joint inflammation upon intra-articular injection of the FBG domain of tenascin-C in mice *in vivo.* Moreover, acute joint inflammation induced by zymosan was not as prolonged in tenascin-C deficient mice. Both the wild type and tenascin-C null mice responded to acute inflammation induction by zymosan equally, demonstrating that tenascin-C does not appear to be involved in the initiation of inflammation. However, the less persistent synovitis exhibited by tenascin-C null mice indicated a role in the maintenance of joint inflammation. The importance of tenascin-C in prolonging joint inflammation was underscored by the observation that targeted deletion of tenascin-C protected mice from sustained erosive joint inflammation during arthritis induced by immunization with mBSA.

Tenascin-C was shown to be capable of activating cells in the joint and the primary active domain of tenascin-C has been mapped to the fibrinogen-like globe (FBG), a 227 amino acid (26.9 kDa) globular domain at the C terminal of the molecule (Siri (1991)).

Addition of FBG to synovial membrane cultures from RA patients enhanced the spontaneous release of pro-inflammatory cytokines. It also stimulated synthesis of TNF-α, IL-6 and IL-8 in primary human macrophages and IL-6 in RA synovial fibroblasts via activation of TLR4 and MyD88 dependent signalling pathways.

It has been shown that, as in the case of LPS, TLR4 expression is necessary for induction of cytokine synthesis by FBG. However, unlike LPS, neither CD14 nor MD-2 appears to be required for TLR-4 activation. CD14 is dispensable for activation of TLR4 by other ligands. It is not required for TLR4 to respond to lipid A in a MyD88 dependent manner (Jiang (2005)), fibronectin EDA can activate mast cells even in the absence of CD14 (Gondokaryono (2007)) and hyaluronic acid activation of human monocytic THP-1 cells requires a complex of TLR4, CD44 and MD-2, but not CD14 (Taylor (2007)).

Formation of distinct receptor complexes by each TLR4 ligand may facilitate recruitment of different intracellular adapter/signalling molecules. This may account for the differential cellular responses we observe with FBG and LPS, for example lack of IL-8 induction by FBG in RA synovial fibroblasts. Similarly, hyaluronic acid activation of the TLR4 and CD44 complex induces a pattern of gene expression in mouse alveolar macrophage cell lines that is different to LPS (Taylor (2007)). That FBG induces IL-8 synthesis in human macrophages, suggests cell type specific ligand recognition and/or signalling occurs.

The tightly regulated pattern of expression of tenascin-C makes it an attractive target for treating chronic inflammation. It is predominantly absent from healthy adults, however expression is specifically induced upon tissue injury. During acute inflammation tenascin-C is transiently expressed: induction often precedes inflammation and both mRNA and protein are absent from the tissue by the time inflammation is resolved (reviewed in Chiquet-Ehrismann (2003)).

Persistent expression of tenascin-C has now been shown to be associated with chronic inflammation. In addition to RA, increased tenascin-C levels are observed in other autoimmune diseases including multiple sclerosis (Gutowski (1999)) and Sjogrens disease (Amin (2001)), and in non-healing wounds and diabetic and venous ulcers (Loots (1998)). *De novo* synthesis of tenascin-C correlates well with the intensity of inflammation in diseases of the oral mucosa and plasma levels of tenascin-C are a reliable indicator for the activity of inflammatory bowel diseases before and after medication or surgery (reviewed in Chiquet-Ehrismann (2003)).

WO 2010/103289 described agents for modulation of a chronic inflammatory response wherein the agent modulates the biological activity of tenascin-C and their use in treating conditions associated with chronic inflammation. However, there remains an ongoing need for new and improved treatments for such conditions.

The inventors have now shown that, surprisingly, tenascin-C can be citrullinated *in vitro* and that citrullinated tenascin-C is preferentially found in patients with a chronic inflammatory condition.

Citrullination results from the conversion of arginine residues to citrulline, mediated by peptidyl arginine deiminases (PADs). This post translational modification occurs physiologically in the skin and central nervous system and pathologically at sites of inflammation. The change significantly affects protein conformation, ionic interactions and susceptibility to proteolytic cleavage. Citrullination also creates new epitopes that lead to the generation of specific antibodies. Antibodies to citrullinated proteins (ACPAs) specifically recognise these citrullinated protein antigens. In addition, epitopes created by citrullination may also determine protein binding to HLA DR, T cell receptors and specific ligands within the innate immune system (67).

ACPAs have been found in RA patients and this is one way of diagnosing this disease. More recently, these antibodies have also been shown to actively promote disease. However, the proteins that these antibodies recognise are not well described. Until now, the only citrullinated antigens to have been identified in RA synovial fluid, be epitope-mapped and the specificity of their antibodies reproducibly confirmed in several laboratories (67) are fibrinogen, type II collagen, vimentin and α-enolase

The inventors previously identified citrullinated α-enolase as an auto-antigen in RA and demonstrated its importance both in both diagnosis and aetiology (68-74). Recent data from other groups suggest that ACPAs and their antigens also actively contribute to disease pathogenesis. For example, citrullinated fibrinogen-containing immune complexes enhance experimental murine arthritis (75, 76) and synergistically promote cytokine synthesis by activation of toll-like receptor 4 (TLR4) and Fcγ receptors in human monocytes (77, 78).

It has long been known that fibrinogen is citrullinated and that antibodies to this form of fibrinogen are present in RA patients. Sokolove (2011) (16) shows that fibrinogen when citrullinated was a better activator of TLR4 than native fibrinogen and that citrullinated fibrinogen formed complexes with antibodies that activated inflammation via synergistic TLR-Fcy receptor signalling. In this way modification of this protein exacerbates inflammation in RA.

The inventors previously identified the pro-inflammatory glycoprotein tenascin-C as an endogenous activator of innate and adaptive immune responses and showed that its expression is required for chronic joint inflammation *in vivo* (79, 80). Furthermore, high levels of tenascin-C were found in RA synovium (81) and serum (82).

However, it has not previously been considered that tenascin-C may be citrullinated, nor that such citrullination might modulate the pro-inflammatory activity of tenascin-C or that citrullinated tenascin-C might be an autoantigen.

The inventors have now identified that full length tenascin-C, as well as its individual domains, including (but not only) the FBG domain, can be post translationally modified by citrullination *in vitro.* The inventors show that citrullinated FBG is better at stimulating cytokine (e.g. TNFα) synthesis by primary human macrophages than native FBG. The inventors have found that only RA patients, and not normal healthy controls, possess antibodies that recognise citrullinated tenascin-C and that serum from RA patients and normal healthy controls does not react with native or non citrullinated tenascin-C. The inventors also show that along with the FBG domain, other domains of tenascin-C are citrullinated in RA patients.

This is the first finding that tenascin-C can be citrullinated and the first demonstration that this modification of tenascin-C is relevant in RA. The inventors also show that citrullination acts to enhance the inflammatory capacity of tenascin-C providing at least three new major mechanisms by which this protein drives inflammation in RA. The pro-inflammatory effect of the citrullinated antigen, i.e. tenascin-C, is a finding of major significance, because it shows that both antibody (e.g. via Fcγ receptor signalling) and antigen (e.g. by TLR signalling) components of ACPA-containing tenascin-C immune complexes are pro-inflammatory. Thus citrullinated tenascin-C alone, autoantibodies to citrullinated tenascin-C alone or citrullinated tenascin-C-antibody complexes may drive inflammation in disease.

Even more surprisingly, the inventors have found that the amino acids in fibrinogen that are citrullinated are located in regions that are not homologous to the FBG domain of tenascin-C. In other words, amino acids that are citrullinated and targets of ACPA in FBG-C are not homologues to regions in fibrinogen. This is surprising as one might expect the same sequences to be modified in each protein.

Therefore, this is the first time that it has been shown that domains of tenascin-C, including the FBG domain are citrullinated in RA and that antibodies against citrullinated tenascin-C are found preferentially in RA patients

In a first aspect of the invention there is provided an agent for modulation of a chronic inflammatory response wherein the agent modulates the biological activity of citrullinated tenascin-C; wherein the agent is an antibody or antigen binding fragment thereof which binds specifically to citrullinated tenascin-C.

By "biological activity" of citrullinated tenascin-C we include: 1) the effect of citrullinated tenascin-C via TLRs or other receptors; and/or 2) the effect of antibodies that recognise citrullinated tenascin-C activating Fcγ receptors or other receptors; and/or 3) the effect of signalling by tenascin-ACPA immune complexes together, for example that might synergistically activate TLR and Fcy or other receptors.

In preferred embodiments, the modulation of the biological activity of citrullinated tenascin-C includes one/or more of: 1) the modulation of citrullinated tenascin-C's action on TLRs (e.g. TLR4) and/or other receptors acting alone and in complex with TLRs (e.g. integrins); 2) the modulation of the generation of ACPAs against citrullinated tenascin-C and their activation of Fcy receptors; and 3) the modulation of tenascin-C's action via forming complexes with ACPA antibodies and the resultant signalling via Fcγ/TLR receptor complexes or other receptor complexes. The modulation of the biological activity of citrullinated tenascin-C may include two/or more of these or, in some embodiments, all of these.

"Citrullinated tenascin-C" is intended to include tenascin-C which has been modified to any extent at any position by the post-translational process of citrullination, that is, the conversion of arginine residues to citrulline. "Citrullinated tenascin-C" also includes one or more fragments of citrullinated tenascin-C. It is therefore intended that the agents of the invention may modulate the biological activity of one or more fragments of citrullinated tenascin-C. "Citrullinated tenascin-C" may include tenascin-C which has been citrullinated at one or more specific residue(s), for example, wherein the specific residue(s) may be selected from any of the group comprising residues 50, 51, 55, 72, 120, 169, 173, 209, 214, 219, 220, 222; or combinations thereof (residue numbers as determined from SEQ ID NO: 70). Alternatively, the tenascin-C may be citrullinated at one or more specific residue(s) wherein the specific residue(s) may be selected from any of the group comprising residues 55, 72, 120, 169, 173, 209, 214, 219, and 220; or combinations thereof (residue numbers as determined from SEQ ID NO: 70). The specific citrullinated residues may comprise CIT55, CIT209, CIT214, CIT219, and/or CIT220. The specific citrullinated residue may comprise CIT50. The specific citrullinated residue may comprise CIT51. The specific citrullinated residue may comprise CIT55. The specific citrullinated residue may comprise CIT209. The specific citrullinated residue may comprise CIT214. The specific citrullinated residue may comprise CIT219. The specific citrullinated residue may comprise CIT220. In another embodiment, the specific citrullinated residues may comprise CIT209 and/or CIT214. Alternatively, the specific citrullinated residues may comprise CIT219 and/or CIT220. The specific citrullinated residue may comprise CIT222.

The agent of the first aspect of the invention may modulate the biological activity of citrullinated tenascin-C by altering one or more of the physical properties of citrullinated tenascin-C, altering the binding properties of citrullinated tenascin-C and/or alternating the antigenicity of citrullinated tenascin-C.

By altering the antigenicity of citrullinated tenascin-C it is meant that the number of antibodies that are made against the citrullinated version is increased or decreased. Citrullination makes proteins more antigenic and these antibodies are important in driving the pathogenesis of RA. Preferably the antigenicity of tenascin-C is reduced.

By altering the physical properties of citrullinated tenascin-C we include altering one or more of the following properties of citrullinated tenascin-C: (i) the charge; (ii) folding patterns (such as enhancing a more fibrillar structure); and (iii) the exposure or concealment of cryptic domains, modules or sequences of citrullinated tenascin-C.

The agent of the first aspect of the invention may also modulate the biological activity of citrullinated tenascin-C by altering the level of citrullination of citrullinated tenascin-C, or by altering the ratio of citrullinated tenascin-C to non-citrullinated tenascin-C.

The overall level of citrullination of tenascin might be altered (i.e. more or fewer arginine residues are modified). The level of citrullination might be reduced over the whole of tenascin-C or only over one or more specific fragments or domains (e.g. the FBG domain). Preferably the level of citrullination is reduced.

The individual domains of tenascin-C at which citrullination may be altered include one or more of the assembly domain (TA domain), the EGF-L repeats (EGF-L domain), the fibronectin type III-like repeats (TNIII domain) and the FBG domain. In a preferred embodiment it is the citrullination of the fibronectin type III-like repeats and/or the FBG domain that is altered. In a most preferred embodiment, it is the citrullination of the FBG domain that is altered. Preferably the alteration of citrullination is a decrease in citrullination.

Similarly, the ratio of citrullination of one or more specific domains of tenascin-C (e.g. the FBG domain) to one or more other domains of tenascin-C might be altered. Alternatively, the ratio of citrullination of one or more specific residues of tenascin-C (e.g. one or more of residues 55, 209, 214, 219, 220, 50, 51, 72, 120, 169, 173 and 222 (residue numbers as determined from SEQ ID NO: 70)) to one or more other residues (e.g. one or more of residues 55, 209, 214, 219, 220, 50, 51, 72, 120, 169, 173 and 222) of tenascin-C might be altered.

The level of citrullination might be reduced by altering one or more specific citrullinated residue(s) of tenascin-C, or fragment thereof, to a non-citrullinated form of the residue(s), wherein the specific citrullinated residue(s) may be selected from any of the group comprising residues 55, 72, 120, 169, 173, 209, 214, 219, and 220 (residue numbers as determined from SEQ ID NO: 70); or combinations thereof; or wherein the specific citrullinated residue(s) may be selected from any of the group comprising residues 50, 51, 55, 72, 120, 169, 173, 209, 214, 219, 220 and 222; or combinations thereof.

The specific citrullinated residues may comprise one or more of CIT55, CIT209, CIT214, CIT219 and CIT220. The specific citrullinated residue may comprise CIT50. The specific citrullinated residue may comprise CIT51. The specific citrullinated residue may comprise CIT55. The specific citrullinated residue may comprise CIT209. The specific citrullinated residue may comprise CIT214. The specific citrullinated residue may comprise CIT219. The specific citrullinated residue may comprise CIT220. In another embodiment, the specific citrullinated residues may comprise CIT209 and/or CIT214. Alternatively, the specific citrullinated residues may comprise CIT219 and/or CIT220. The specific citrullinated residue may comprise CIT222.

The specific citrullinated residues may be, or be found in, an epitope and may be detected in such an epitope. For example, the epitope may comprise at least one citrullinated residue selected from CIT55, CIT209, CIT214, CIT219 and CIT220; or combinations thereof. The epitope may comprise at least one citrullinated residue selected from CIT50, CIT51, CIT55, CIT209, CIT214, CIT219, CIT220 and CIT222; or combinations thereof. The epitope may comprise citrullinated residue CIT50. The epitope may comprise citrullinated residue CIT51. The epitope may comprise citrullinated residue CIT55. The epitope may comprise citrullinated residue CIT209. The epitope may comprise citrullinated residue CIT214. The epitope may comprise citrullinated residue CIT219. The epitope may comprise citrullinated residue CIT220. The epitope may comprise citrullinated residue CIT222.

Levels of citrullination might be reduced by preventing citrullination in the first place (e.g. by using PAD inhibitors), by reducing tenascin expression in general, by increasing native tenascin expression, and/or by administering arginine null mutants of tenascin-C)

Such agents may be identified using methods well known in the art, such as:
(a) by determining the effect of a test agent on levels of expression of citrullinated tenascin-C, for example by western blotting, or related hybridisation techniques;
(b) by determining the effect of a test agent on levels of citrullinated tenascin-C protein, for example by immunoassays using anti- citrullinated tenascin-C antibodies;
(c) by determining the effect of a test agent on a functional marker or result of citrullinated tenascin-C activity, for example via the methods of the examples;
(d) by using a change in charge or size e.g. when migrating on SDSPAGE to show citrullination; and/or
(e) by using mass spectrometry and proteomic approaches to quantify the ratio of citrullination as well as identify specific arginine residues that are modified.

The agent of the first aspect of the invention may down-regulate the biological activity of citrullinated tenascin-C.

The agent of the first aspect of the invention may up-regulate the biological activity of citrullinated tenascin-C. The desirability of up-regulating activity of immune and inflammatory molecules and cells is relevant to the production of therapies for compromised immune and inflammatory patients and in the development of vaccines. (see Harandi (2009)).

The agent of the first aspect of the invention may be an inhibitor of transcription of tenascin-C.

The agent of the first aspect of the invention may be an inhibitor of translation of tenascin-C.

The agent of the first aspect of the invention may be an inhibitor of citrullination of tenascin-C. Inhibition of citrullination may be achieved, for example, in the same manner as in which the levels of citrullination may be reduced (e.g. by inhibiting peptidyl arginine deiminases (PADs)).

The agent of the first aspect of the invention may be an inhibitor of the binding properties of citrullinated tenascin-C. For example, the agent may alter the conformation of citrullinated tenascin-C such that it is no longer able to bind to its receptor.

The agent of the first aspect of the invention may be a competitive binding inhibitor of citrullinated tenascin-C. It will be appreciated by persons skilled in the art that the agent may also inhibit the biological activity of citrullinated tenascin-C by blocking citrullinated tenascin-C receptor function either directly (by acting as a citrullinated tenascin-C receptor antagonist) or indirectly (by binding intermediary or assisting molecules).

The agent of the first aspect of the invention may be an antagonist of the TLR-4 receptor and/or the Fcγ receptor and/or any other receptor, or receptors in complex.

It will be appreciated by persons skilled in the art that inhibition of the biological activity of citrullinated tenascin-C by an agent of the invention may be in whole or in part. For example, the agent may inhibit the biological activity of citrullinated tenascin-C by at least 10%, preferably at least 20%, 30%, 40%, 50%, 60%, 70%, 80% or 90%, and most preferably by 100% compared to the biological activity of citrullinated tenascin-C on inflammatory cells which have not been exposed to the agent.

The FBG domain has been shown to be predominantly involved in the interaction of tenascin-C with its target in relation to the persistence of chronic inflammation. The FBG domain in particular has also been shown to be citrullinated. An additional or alternative domain of citrullinated tenascin-C is the fibronectin type III-like repeats. The citrullinated tenascin-C or one or more fragments of citrullinated tenascin-C may be citrullinated at one or more specific residue(s) wherein the specific residue(s) may be selected from any of the group comprising residues 50, 51, 55, 72, 120, 169, 173, 209, 214, 219, 220, 222; or combinations thereof (residue numbers as determined from SEQ ID NO: 70). Alternatively, the citrullinated tenascin-C or one or more fragments of citrullinated tenascin-C may be citrullinated at one or more specific residue(s) wherein the specific residue(s) may be selected from any of the group comprising residues 55, 72, 120, 169, 173, 209, 214, 219, and 220; or combinations thereof (residue numbers as determined from SEQ ID NO: 70). The specific citrullinated residues may comprise CIT55, CIT209, CIT214, CIT219, and/or CIT220. The specific citrullinated residue may comprise CIT50. The specific citrullinated residue may comprise CIT51. The specific citrullinated residue may comprise CIT55. The specific citrullinated residue may comprise CIT209. The specific citrullinated residue may comprise CIT214. The specific citrullinated residue may comprise CIT219. The specific citrullinated residue may comprise CIT220. In another embodiment, the specific citrullinated residues may comprise CIT209 and/or CIT214. Alternatively, the specific citrullinated residues may comprise CIT219 and/or CIT220. The specific citrullinated residue may comprise CIT222.

The specific citrullinated residues may be, or be found in, an epitope and may be detected in such an epitope. For example, the epitope may comprise at least one citrullinated residue selected from CIT55, CIT209, CIT214, CIT219 and CIT220; or combinations thereof. The epitope may comprise at least one citrullinated residue selected from CIT50, CIT51, CIT55, CIT209, CIT214, CIT219, CIT220 and CIT222; or combinations thereof. The epitope may comprise citrullinated residue CIT50. The epitope may comprise citrullinated residue CIT51. The epitope may comprise citrullinated residue CIT55. The epitope may comprise citrullinated residue CIT209. The epitope may comprise citrullinated residue CIT214. The epitope may comprise citrullinated residue CIT219. The epitope may comprise citrullinated residue CIT220. The epitope may comprise citrullinated residue CIT222.

In an example where the agent is a compound with binding affinity for citrullinated tenascin-C, the compound may bind substantially reversibly or substantially irreversibly to an active site of citrullinated tenascin-C. In a further example, the compound may bind to a portion of citrullinated tenascin-C that is not the active site so as to interfere with the binding of the citrullinated tenascin-C to a ligand or receptor. In a still further example, the compound may bind to a portion of citrullinated tenascin-C so as to decrease the proteins activity by an allosteric effect. This allosteric effect may be an allosteric effect that is involved in the natural regulation of the activity of citrullinated tenascin-C, for example in the activation of the citrullinated tenascin-C by an "upstream activator". Alternatively, the compound may bind to an immune complex of citrullinated tenascin-C and an ACPA.

Methods for detecting interactions between a test compound and citrullinated tenascin-C are well known in the art. For example ultrafiltration with ion spray mass spectroscopy/HPLC methods or other physical and analytical methods may be used. In addition, Fluorescence Energy Resonance Transfer (FRET) methods may be used, in which binding of two fluorescent labelled entities may be measured by measuring the interaction of the fluorescent labels when in close proximity to each other.

Alternative methods of detecting binding of a polypeptide to macromolecules, for example DNA, RNA, proteins and phospholipids, include a surface plasmon resonance assay, for example as described in Plant et al., 1995, Analyt Biochem 226(2), 342-348. Methods may make use of a polypeptide that is labelled, for example with a radioactive or fluorescent label.

A further method of identifying a compound that is capable of binding to the polypeptide is one where the polypeptide is exposed to the compound and any binding of the compound to the said polypeptide is detected and/or measured. The binding constant for the binding of the compound to the polypeptide may be determined. Suitable methods for detecting and/or measuring (quantifying) the binding of a compound to a polypeptide are well known to those skilled in the art and may be performed, for example, using a method capable of high throughput operation, for example a chip-based method. New technology, called VLSIPS™, has enabled the production of extremely small chips that contain hundreds of thousands or more of different molecular probes. These biological chips or arrays have probes arranged in arrays, each probe assigned a specific location. Biological chips have been produced in which each location has a scale of, for example, ten microns. The chips can be used to determine whether target molecules interact with any of the probes on the chip. After exposing the array to target molecules under selected test conditions, scanning devices can examine each location in the array and determine whether a target molecule has interacted with the probe at that location.

Another method of identifying compounds with binding affinity for citrullinated tenascin-C is the yeast two-hybrid system, where the polypeptides of the invention can be used to "capture" proteins that bind citrullinated tenascin-C. The yeast two-hybrid system is described in Fields & Song, Nature 340:245-246 (1989).

In a further example, the agent is a compound which has ligand-binding capacity for citrullinated tenascin-C.

The agent of the first aspect of the invention is an antibody or antigen-binding fragment thereof. The antigen-binding fragment may be selected from the group consisting of Fv fragments (*e.g*. single chain Fv and disulphide-bonded Fv), Fab-like fragments (*e.g*. Fab fragments, Fab' fragments and F(ab)2 fragments), single variable domains (*e.g*. V_{H} and V_{L} domains) and domain antibodies (dAbs, including single and dual formats [*i.e.* dAb-linker-dAb]).

The antibody or antigen-binding fragment thereof binds specifically to citrullinated tenascin-C (i.e. it does not bind to non-citrullinated tenascin-C).

The antibody may preferably bind specifically to the FBG domain of citrullinated tenascin-C that activates TLR4, and/or the antibody may preferably bind to an alternative domain of citrullinated tenascin-C. One or more of those domains in particular may be citrullinated.

The advantages of using antibody fragments, rather than whole antibodies, are several-fold. The smaller size of the fragments may lead to improved pharmacological properties, such as better penetration of solid tissue. Moreover, antigen-binding fragments such as Fab, Fv, ScFv and dAb antibody fragments can be expressed in and secreted from *E. coli*, thus allowing the facile production of large amounts of the said fragments.

Also included within the scope of the invention are modified versions of antibodies and an antigen-binding fragments thereof, *e.g*. modified by the covalent attachment of polyethylene glycol or other suitable polymer.

Methods of generating antibodies and antibody fragments are well known in the art. For example, antibodies may be generated via any one of several methods which employ induction of *in vivo* production of antibody molecules, screening of immunoglobulin libraries (Orlandi. et al, 1989. Proc. Natl. Acad. Sci. U.S.A. 86:3833-3837; Winter et al., 1991, Nature 349:293-299) or generation of monoclonal antibody molecules by cell lines in culture. These include, but are not limited to, the hybridoma technique, the human B-cell hybridoma technique, and the Epstein-Barr virus (EBV)-hybridoma technique (Kohler et al., 1975. Nature 256:4950497; Kozbor et al., 1985. J. Immunol. Methods 81:31-42; Cote et al., 1983. Proc. Natl. Acad. Sci. USA 80:2026-2030; Cole et al., 1984. Mol. Cell. Biol. 62:109-120).

Suitable monoclonal antibodies to selected antigens may be prepared by known techniques, for example those disclosed in *"*Monoclonal Antibodies: A manual of techniques", H Zola (CRC Press, 1988) and in *"*Monoclonal Hybridoma Antibodies: Techniques and Applications", J G R Hurrell (CRC Press, 1982).

Antibody fragments can be obtained using methods well known in the art (see, for example, Harlow & Lane, 1988, "Antibodies: A Laboratory Manual", Cold Spring Harbor Laboratory, New York). For example, antibody fragments according to the present invention can be prepared by proteolytic hydrolysis of the antibody or by expression in *E. coli* or mammalian cells (*e.g.* Chinese hamster ovary cell culture or other protein expression systems) of DNA encoding the fragment. Alternatively, antibody fragments can be obtained by pepsin or papain digestion of whole antibodies by conventional methods.

It will be appreciated by persons skilled in the art that for human therapy or diagnostics, humanised antibodies are preferably used. Humanised forms of non-human (e.g. murine) antibodies are genetically engineered chimaeric antibodies or antibody fragments having preferably minimal-portions derived from non-human antibodies. Humanised antibodies include antibodies in which complementary determining regions of a human antibody (recipient antibody) are replaced by residues from a complementary determining region of a non human species (donor antibody) such as mouse, rat of rabbit having the desired functionality. In some instances, Fv framework residues of the human antibody are replaced by corresponding non-human residues. Humanised antibodies may also comprise residues which are found neither in the recipient antibody nor in the imported complementarity determining region or framework sequences. In general, the humanised antibody will comprise substantially all of at least one, and typically two, variable domains, in which all or substantially all of the complementarity determining regions correspond to those of a non human antibody and all, or substantially all, of the framework regions correspond to those of a relevant human consensus sequence. Humanised antibodies optimally also include at least a portion of an antibody constant region, such as an Fc region, typically derived from a human antibody (see, for example, Jones et al., 1986. Nature 321:522-525; Riechmann et al., 1988, Nature 332:323-329; Presta, 1992, Curr. Op. Struct. Biol. 2:593-596).

Methods for humanising non-human antibodies are well known in the art. Generally, the humanised antibody has one or more amino acid residues introduced into it from a source which is non-human. These non-human amino acid residues, often referred to as imported residues, are typically taken from an imported variable domain. Humanisation can be essentially performed as described (see, for example, Jones et al., 1986, Nature 321:522-525; Reichmann et al., 1988. Nature 332:323-327; Verhoeyen et al., 1988, Science 239:1534-1536I; US 4,816,567) by substituting human complementarity determining regions with corresponding rodent complementarity determining regions. Accordingly, such humanised antibodies are chimaeric antibodies, wherein substantially less than an intact human variable domain has been substituted by the corresponding sequence from a non-human species. In practice, humanised antibodies may be typically human antibodies in which some complementarity determining region residues and possibly some framework residues are substituted by residues from analogous sites in rodent antibodies.

Human antibodies can also be identified using various techniques known in the art, including phage display libraries (see, for example, Hoogenboom & Winter, 1991, J. Mol. Biol. 227:381; Marks et al., 1991, J. Mol. Biol. 222:581; Cole et al., 1985, In: Monoclonal antibodies and Cancer Therapy, Alan R. Liss, pp. 77; Boerner et al., 1991. J. Immunol. 147:86-95).

Once suitable antibodies are obtained, they may be tested for activity, for example by ELISA.

The agent of the first aspect of the invention may be an antibody or antigen-binding fragment thereof which has specificity for Toll Like Receptor 4 (TLR4) or co-receptors of Toll Like Receptor 4, or Fcγ receptor, or tenascin-C or a domain thereof of any of these.

Co-receptors to primary receptors, such as TLR4, assist with binding of a signalling molecule to the primary receptor in order to facilitate ligand recognition and binding and initiate/maintain the biological process resulting from receptor binding.

The agent of the first aspect of the invention may be an agent which has binding affinity for the FBG domain of citrullinated tenascin-C.

The agent of the first aspect of the invention may be an antibody or antigen-binding fragment thereof which has specificity for the FBG domain of citrullinated tenascin-C.

The agent of the first aspect of the invention may be an agent which has binding affinity for one or more of residues 50, 51, 55, 72, 120, 169, 173, 209, 214, 219, 220 and 222 as numbered in SEQ ID NO: 70 of citrullinated tenascin-C, or which has binding affinity for one or more of residues 55, 72, 120, 169, 173, 209, 214, 219, and 220 as numbered in SEQ ID NO: 70 of citrullinated tenascin-C, or which has binding affinity for one or more of residues 55, 209, 214, 219, and 220 as numbered in SEQ ID NO: 70 of citrullinated tenascin-C. The agent of the first aspect of the invention may be an agent which has binding affinity for residue 55. The agent of the first aspect of the invention may be an agent which has binding affinity for residue 209. The agent of the first aspect of the invention may be an agent which has binding affinity for residue 214. The agent of the first aspect of the invention may be an agent which has binding affinity for residue 219. The agent of the first aspect of the invention may be an agent which has binding affinity for residue 220.

The agent of the first aspect of the invention may be an antibody or antigen-binding fragment thereof which has specificity for one or more of residues 50, 51, 55, 72, 120, 169, 173, 209, 214, 219, 220 and 222 as numbered in SEQ ID NO: 70 of citrullinated tenascin-C, or which has specificity for one or more of residues 55, 72, 120, 169, 173, 209, 214, 219, and 220 as numbered in SEQ ID NO: 70 of citrullinated tenascin-C, or which has specificity for one or more of residues 55, 209, 214, 219, and 220 as numbered in SEQ ID NO: 70 of citrullinated tenascin-C. The agent of the first aspect of the invention may be an agent which has specificity for residue 55. The agent of the first aspect of the invention may be an agent which has specificity for residue 209. The agent of the first aspect of the invention may be an agent which has specificity for residue 214. The agent of the first aspect of the invention may be an agent which has specificity for residue 219. The agent of the first aspect of the invention may be an agent which has specificity for residue 220.

The agent of the first aspect of the invention may be an agent which modulates the biological activity of the FBG domain of citrullinated tenascin-C. The agent may modulate the biological activity of the FBG domain in addition to that of other domains of citrullinated tenascin-C, or the agent may modulate the biological activity of only the FBG domain of citrullinated tenascin-C.

The agent of the first aspect of the invention may modulate citrullinated tenascin-C which is citrullinated at the FBG domain. The citrullinated tenascin-C that is modulated may, therefore, be citrullinated at the FBG domain in addition to one or more (or all) other domains of tenascin-C. The citrullinated tenascin-C may also be citrullinated at only the FBG domain. Alternatively, the citrullinated tenascin-C may be citrullinated only at one or more domains other than the FBG domain. In other words, the citrullinated tenascin-C may not be citrullinated FBG domain but be citrullinated at one or more or all other domains of tenascin-C. A preferred example of a non-FBG domain of tenascin-C that may be citrullinated is the fibronectin type III-like repeats.

The agent of the first aspect of the invention may modulate citrullinated tenascin-C which is citrullinated at one or more specific residue(s) wherein the specific residue(s) may be selected from any of the group comprising residues 50, 51, 55, 72, 120, 169, 173, 209, 214, 219, 220, 222; or combinations thereof (residue numbers as determined from SEQ ID NO: 70). Alternatively, the citrullinated tenascin-C or one or more fragments of citrullinated tenascin-C may be citrullinated at one or more specific residue(s) wherein the specific residue(s) may be selected from any of the group comprising residues 55, 72, 120, 169, 173, 209, 214, 219, and 220; or combinations thereof (residue numbers as determined from SEQ ID NO: 70). The specific citrullinated residues may comprise CIT55, CIT209, CIT214, CIT219, and/or CIT220. The specific citrullinated residue may comprise CIT50. The specific citrullinated residue may comprise CIT51. The specific citrullinated residue may comprise CIT55. The specific citrullinated residue may comprise CIT209. The specific citrullinated residue may comprise CIT214. The specific citrullinated residue may comprise CIT219. The specific citrullinated residue may comprise CIT220. In another embodiment, the specific citrullinated residues may comprise CIT209 and/or CIT214. Alternatively, the specific citrullinated residues may comprise CIT219 and/or CIT220. The specific citrullinated residue may comprise CIT222.

The specific citrullinated residues may be, or be found in, an epitope and may be detected in such an epitope. For example, the epitope may comprise at least one citrullinated residue selected from CIT55, CIT209, CIT214, CIT219 and CIT220; or combinations thereof. The epitope may comprise at least one citrullinated residue selected from CIT50, CIT51, CIT55, CIT209, CIT214, CIT219, CIT220 and CIT222; or combinations thereof. The epitope may comprise citrullinated residue CIT50. The epitope may comprise citrullinated residue CIT51. The epitope may comprise citrullinated residue CIT55. The epitope may comprise citrullinated residue CIT209. The epitope may comprise citrullinated residue CIT214. The epitope may comprise citrullinated residue CIT219. The epitope may comprise citrullinated residue CIT220. The epitope may comprise citrullinated residue CIT222.

The agent of the first aspect of the invention may modulate the biological activity of autoantibodies with binding specificity for citrullinated tenascin-C. In preferred embodiments the agent binds the autoantibodies with binding specificity for citrullinated tenascin either alone and/or when the autoantibody is part of a complex with citrullinated tenascin-C.

Autoantibodies with binding specificity for citrullinated tenascin-C may have binding specificity for one or more of residues 50, 51, 55, 72, 120, 169, 173, 209, 214, 219, 220 and 222 as numbered in SEQ ID NO: 70 of citrullinated tenascin-C, or may have binding specificity for one or more of residues 55, 72, 120, 169, 173, 209, 214, 219, and 220 as numbered in SEQ ID NO: 70 of citrullinated tenascin-C, or may have binding specificity for one or more of residues 55, 209, 214, 219, and 220 as numbered in SEQ ID NO: 70 of citrullinated tenascin-C.

A method of identifying an agent that modulates the activity of citrullinated tenascin-C comprising the steps of:
(i) providing one or more a candidate agents;
(ii) contacting one or more cells with citrullinated tenascin-C and the one or more candidate agents;
(iii) contacting one or more cells with citrullinated tenascin-C and no candidate agent;
(iv) determining whether said candidate agent modulates the effect of citrullinated tenascin-C on the one or more cells in step (ii) in comparison to the cell(s) of control step (iii).

Methods of determining whether the candidate agent modulates the effect of citrullinated tenascin-C can be carried out using the methods of the examples.

Tenascin-C binding to TLR4 or any other appropriate receptor can be tested by solid phase binding assay, surface plasmon resonance, analytical gel filtration +/- inhibitor. The activity of the tenascin-C/ACPA complex can be accounted for by using the same method as for FBG alone and test for stimulation of cytokines by cells +/- inhibitor.

The method may result in the activity of citrullinated tenascin-C being up-regulated.

The method may result in the activity of citrullinated tenascin-C being down-regulated.

The method may include the cells of steps (ii) and (iii) (described above) expressing Toll-like receptor 4 (TLR4) and/or Fcγ receptor.

The method may have the one or more cells selected from the group consisting of inflammatory cells, fibroblasts, fibroblast like cells (including RA synovial fibroblasts, also known as synoviocytes), mouse embryonic fibroblasts, human embryonic kidney cells.

The inflammatory cells may be selected from the group consisting of macrophages, dendritic cells, monocytes, lymphocytes, monocyte like cells and macrophage like cells.

The chronic inflammation may be associated with any condition associated with inappropriate inflammation. Such conditions include, but are not limited to, rheumatoid arthritis (RA), autoimmune conditions, inflammatory bowel diseases (including Crohn's disease and ulcerative colitis), non-healing wounds, multiple sclerosis, cancer, atherosclerosis, sjogrens disease, diabetes, lupus erythrematosus (including systemic lupus erythrematosus), asthma, fibrotic diseases (including liver cirrhosis), pulmonary fibrosis, UV damage, psoriasis, ankylosing spondylitis and cardiovascular disease.

Of particular, but non-exclusive interest, the chronic inflammation is associated with rheumatoid arthritis (RA).

The agent may down-regulate the chronic inflammatory response.

The agent may up-regulate the chronic inflammatory response.

In the first aspect of the invention the chronic inflammation may be associated with any condition associated with inappropriate inflammation. Such conditions include, but are not limited to, rheumatoid arthritis (RA), autoimmune conditions, inflammatory bowel diseases (including Crohn's disease and ulcerative colitis), non-healing wounds, multiple sclerosis, cancer, atherosclerosis, sjogrens disease, diabetes, lupus erythrematosus (including systemic lupus erythrematosus), asthma, fibrotic diseases (including liver cirrhosis), pulmonary fibrosis, UV damage, psoriasis, ankylosing spondylitis and cardiovascular disease.

In a second aspect of the invention there is provided a composition comprising an agent as defined in the first aspect of the invention and a pharmaceutically acceptable carrier, excipient and/or diluent.

It will be appreciated by persons skilled in the art that such an effective amount of the agent or formulation thereof may be delivered as a single bolus dose (*i.e.* acute administration) or, more preferably, as a series of doses over time (*i.e.* chronic administration).

The agents of the invention can be formulated at various concentrations, depending on the efficacy/toxicity of the compound being used and the indication for which it is being used. Preferably, the formulation comprises the agent of the invention at a concentration of between 0.1 µM and 1 mM, more preferably between 1 µM and 100 µM, between 5 µM and 50 µM, between 10 µM and 50 µM, between 20 µM and 40 µM and most preferably about 30 µM. For *in vitro* applications, formulations may comprise a lower concentration of a compound of the invention, for example between 0.0025 µM and 1 µM.

It will be appreciated by persons skilled in the art that the agents of the invention will generally be administered in admixture with a suitable pharmaceutical excipient diluent or carrier selected with regard to the intended route of administration and standard pharmaceutical practice (for example, see Remington: The Science and Practice of Pharmacy, 19th edition, 1995, Ed. Alfonso Gennaro, Mack Publishing Company, Pennsylvania, USA).

For example, the agents of the invention can be administered orally, buccally or sublingually in the form of tablets, capsules, ovules, elixirs, solutions or suspensions, which may contain flavouring or colouring agents, for immediate-, delayed- or controlled-release applications. The agents of invention may also be administered via intracavernosal injection.

Such tablets may contain excipients such as microcrystalline cellulose, lactose, sodium citrate, calcium carbonate, dibasic calcium phosphate and glycine, disintegrants such as starch (preferably corn, potato or tapioca starch), sodium starch glycollate, croscarmellose sodium and certain complex silicates, and granulation binders such as polyvinylpyrrolidone, hydroxypropylmethylcellulose (HPMC), hydroxy-propylcellulose (HPC), sucrose, gelatin and acacia. Additionally, lubricating agents such as magnesium stearate, stearic acid, glyceryl behenate and talc may be included.

Solid compositions of a similar type may also be employed as fillers in gelatin capsules. Preferred excipients in this regard include lactose, starch, cellulose, milk sugar or high molecular weight polyethylene glycols. For aqueous suspensions and/or elixirs, the compounds of the invention may be combined with various sweetening or flavouring agents, colouring matter or dyes, with emulsifying and/or suspending agents and with diluents such as water, ethanol, propylene glycol and glycerin, and combinations thereof.

The agents of the invention can also be administered parenterally, for example, intravenously, intra-articularly, intra-arterially, intraperitoneally, intra-thecally, intraventricularly, intrasternally, intracranially, intra-muscularly or subcutaneously, or they may be administered by infusion techniques. They are best used in the form of a sterile aqueous solution which may contain other substances, for example, enough salts or glucose to make the solution isotonic with blood. The aqueous solutions should be suitably buffered (preferably to a pH of from 3 to 9), if necessary. The preparation of suitable parenteral formulations under sterile conditions is readily accomplished by standard pharmaceutical techniques well known to those skilled in the art.

Formulations suitable for parenteral administration include aqueous and non-aqueous sterile injection solutions which may contain anti-oxidants, buffers, bacteriostats and solutes which render the formulation isotonic with the blood of the intended recipient; and aqueous and non-aqueous sterile suspensions which may include suspending agents and thickening agents. The formulations may be presented in unit-dose or multi-dose containers, for example sealed ampoules and vials, and may be stored in a freeze-dried (lyophilised) condition requiring only the addition of the sterile liquid carrier, for example water for injections, immediately prior to use. Extemporaneous injection solutions and suspensions may be prepared from sterile powders, granules and tablets of the kind previously described.

For oral and parenteral administration to human patients, the daily dosage level of the agents of the invention will usually be from 1 to 1000 mg per adult (*i.e.* from about 0.015 to 15 mg/kg), administered in single or divided doses.

The agents of the invention can also be administered intranasally or by inhalation and are conveniently delivered in the form of a dry powder inhaler or an aerosol spray presentation from a pressurised container, pump, spray or nebuliser with the use of a suitable propellant, *e.g.* dichlorodifluoromethane, trichlorofluoro-methane, dichlorotetrafluoro-ethane, a hydrofluoroalkane such as 1,1,1,2-tetrafluoroethane (HFA 134A3 or 1,1,1,2,3,3,3-heptafluoropropane (HFA 227EA3), carbon dioxide or other suitable gas. In the case of a pressurised aerosol, the dosage unit may be determined by providing a valve to deliver a metered amount. The pressurised container, pump, spray or nebuliser may contain a solution or suspension of the active compound, *e.g.* using a mixture of ethanol and the propellant as the solvent, which may additionally contain a lubricant, *e.g.* sorbitan trioleate. Capsules and cartridges (made, for example, from gelatin) for use in an inhaler or insufflator may be formulated to contain a powder mix of a compound of the invention and a suitable powder base such as lactose or starch.

Aerosol or dry powder formulations are preferably arranged so that each metered dose or 'puff' contains at least 1 mg of a compound of the invention for delivery to the patient. It will be appreciated that the overall daily dose with an aerosol will vary from patient to patient, and may be administered in a single dose or, more usually, in divided doses throughout the day.

Alternatively, the agents of the invention can be administered in the form of a suppository or pessary, or they may be applied topically in the form of a lotion, solution, cream, ointment or dusting powder. The compounds of the invention may also be transdermally administered, for example, by the use of a skin patch. They may also be administered by the ocular route.

For ophthalmic use, the agents of the invention can be formulated as micronised suspensions in isotonic, pH adjusted, sterile saline, or, preferably, as solutions in isotonic, pH adjusted, sterile saline, optionally in combination with a preservative such as a benzylalkonium chloride. Alternatively, they may be formulated in an ointment such as petrolatum.

For application topically to the skin, the agents of the invention can be formulated as a suitable ointment containing the active compound suspended or dissolved in, for example, a mixture with one or more of the following: mineral oil, liquid petrolatum, white petrolatum, propylene glycol, polyoxyethylene polyoxypropylene compound, emulsifying wax and water. Alternatively, they can be formulated as a suitable lotion or cream, suspended or dissolved in, for example, a mixture of one or more of the following: mineral oil, sorbitan monostearate, a polyethylene glycol, liquid paraffin, polysorbate 60, cetyl esters wax, cetearyl alcohol, 2-octyldodecanol, benzyl alcohol and water.

Formulations suitable for topical administration in the mouth include lozenges comprising the active ingredient in a flavoured basis, usually sucrose and acacia or tragacanth; pastilles comprising the active ingredient in an inert basis such as gelatin and glycerin, or sucrose and acacia; and mouth-washes comprising the active ingredient in a suitable liquid carrier.

Where the agent is a polypeptide, it may be preferable to use a sustained-release drug delivery system, such as a microspheres. These are designed specifically to reduce the frequency of injections. An example of such a system is Nutropin Depot which encapsulates recombinant human growth hormone (rhGH) in biodegradable microspheres that, once injected, release rhGH slowly over a sustained period.

Alternatively, polypeptide agents of the present invention can be administered by a surgically implanted device that releases the drug directly to the required site.

Electroporation therapy (EPT) systems can also be employed for the administration of proteins and polypeptides. A device which delivers a pulsed electric field to cells increases the permeability of the cell membranes to the drug, resulting in a significant enhancement of intracellular drug delivery.

Proteins and polypeptides can also be delivered by electroincorporation (EI). EI occurs when small particles of up to 30 microns in diameter on the surface of the skin experience electrical pulses identical or similar to those used in electroporation. In EI, these particles are driven through the stratum corneum and into deeper layers of the skin. The particles can be loaded or coated with drugs or genes or can simply act as "bullets" that generate pores in the skin through which the drugs can enter.

An alternative method of protein and polypeptide delivery is the thermo-sensitive ReGel injectable. Below body temperature, ReGel is an injectable liquid while at body temperature it immediately forms a gel reservoir that slowly erodes and dissolves into known, safe, biodegradable polymers. The active drug is delivered over time as the biopolymers dissolve.

Protein and polypeptide pharmaceuticals can also be delivered orally. One such system employs a natural process for oral uptake of vitamin B12 in the body to co-deliver proteins and polypeptides. By riding the vitamin B12 uptake system, the protein or polypeptide can move through the intestinal wall. Complexes are produced between vitamin B12 analogues and the drug that retain both significant affinity for intrinsic factor (IF) in the vitamin B12 portion of the complex and significant bioactivity of the drug portion of the complex.

Methods for administering oligonucleotide or polynucleotide agents of the invention are also well know in the art (see Dass, 2002, *J Pharm Pharmacol.* **54**(1):3-27; Dass, 2001, *Drug Deliv.* **8**(4):191-213; Lebedeva *et al*., 2000, *Eur J Pharm Biopharm.* **50**(1):101-19; Pierce *et al*., 2005, *Mini Rev Med Chem.* **5**(1):41-55; *Lysik & Wu-Pong,* 2003, *J Pharm Sci.* 2003 **2**(8):1559-73; Dass, 2004, *Biotechnol Appl Biochem.* **40**(Pt 2):113-22; Medina, 2004, *Curr Pharm Des.* 10(24):2981-9.

The composition of the second aspect of the invention may further comprise at least one other agent.

Such a further agent may be an anti-inflammatory agent which includes but is not limited to non-steroidal anti-inflammatory agent (NSAID), a disease modifying anti-rheumatic drug (DMARD), a statin (including HMG-CoA reductase inhibitors such as simvastatin), a biological agent (biologicals), a steroid, an immunosuppressive agent, a salicylate and/or a microbicidal agent. Non-steroidal anti-inflammatory agents include anti-metabolite agents (such as methotrexate) and anti-inflammatory gold agents (including gold sodium thiomalate, aurothiomalate or gold salts, such as auranofin). Biologicals include anti-TNF agents (including adalimumab, etanercept, infliximab, anti-IL-1 reagents, anti-IL-6 reagents, anti-B cell reagents (retoximab), anti-T cell reagents (anti-CD4 antibodies), anti-IL-15 reagents, anti-CLTA4 reagents, anti-RAGE reagents), antibodies, soluble receptors, receptor binding proteins, cytokine binding proteins, mutant proteins with altered or attenuated functions, RNAi, polynucleotide aptemers, antisense oligonucleotides or omega 3 fatty acids. Steroids (also know as corticosteroids) include cortisone, prednisolone or dexamethasone. Immunosuppressive agents include cylcosporin, FK506, rapamycin, mycophenolic acid. Salicylates include aspirin, sodium salicylate, choline salicylate and magnesium salicylate. Microbicidal agents include quinine and chloroquine. For example, the agent may be administered in combination with one or more of an NSAID, DMARD, or immunosuppressant

In a third aspect of the invention there is provided an agent or composition as defined in the first and second aspects of the invention for use as a medicament.

In a fourth aspect of the invention there is provided an agent or composition as defined in the first and second aspects of the invention for use in the treatment of a chronic inflammatory condition.

In preferred embodiments, the agent or composition as defined in the first, second, third and fourth aspect of the invention relate to treatment of a chronic inflammatory condition wherein the chronic inflammatory condition is in a subject possessing citrullinated tenascin-C and/or autoantibodies with specificity for citrullinated tenascin-C.

The citrullinated tenascin-C and/or autoantibodies possessed by the subject may be found in sample of blood, synovial fluid and/or joint tissue derived from the subject.

By "possessing" citrullinated tenascin-C and/or autoantibodies with specificity for citrullinated tenascin-C it is meant that any citrullinated tenascin-C and/or one or more fragments of citrullinated tenascin-C and/or autoantibodies with specificity for citrullinated tenascin-C and/or for one or more fragments of citrullinated tenascin-C are detected in the sample.

Subjects can be identified as possessing citrullinated tenascin-C and/or one or more fragments of citrullinated tenascin-C by use an antibody specific to citrullinated tenascin-C that does not bind native tenascin-C and then immunoassaying e.g. by ELISA and/or western blot. For either serum or tissue/cells this is preferably a sandwich ELISA, i.e. one citrullinated tenascin-C (citTNC) Ab used as capture, then adding serum/tissue lysate as suitable dilution, then a second, different citTNC antibody to detect. A second antibody can be labelled for detection or indirect detection - as in any standard ELISA protocol. In the event that two different suitable citTNC antibodies cannot be found, the method uses a direct ELISA made up of serum/tissue lysate on plates and the antibody added directly to this. Alternatively, a non-citrullinated-TNC antibody may be used for capture, and a citrullinated-TNC antibody may be used for detection, or vice versa. This also allows ratio of native and citrullinated TNC may be determined.

Alternatively, tenascin-C may be immune precipitated and then western blotting or mass spectrometry may be used to determine if the tenascin-C is citrullinated.

Subjects can be identified as possessing autoantibodies having specificity for citrullinated tenascin-C and/or one or more fragments of citrullinated tenascin-C by western blotting with RA serum as in the examples.

Alternatively, the residues that are citrullinated in TNC can be better defined and then create a peptide assay (either western blot as above, and/or an ELISA approach not possible with large proteins). Only the peptide which is citrullinated is created and, as a control, a non citrullinated peptide. The plate is coated with peptide and RA serum applied and used to detect antibody (see Lundberg (2008) for details of the methods). The citrullinated tenascin-C peptide for use in the assay may comprise any tenascin-C peptide comprising a citrullinated residue selected from any of the group comprising residues 50, 51, 55, 72, 120, 169, 173, 209, 214, 219, 220 and 222; or combinations thereof (residue numbers as determined from SEQ ID NO: 70). The citrullinated tenascin-C peptide for use in the assay may comprise any tenascin-C peptide comprising a citrullinated residue selected from any of the group comprising residues 55, 72, 120, 169, 173, 209, 214, 219, and 220; or combinations thereof. The peptide may comprise citrullinated residues CIT55, CIT209, CIT214, CIT219, and/or CIT220. In another example, the peptide may comprise citrullinated residue CIT50. In another example, the peptide may comprise citrullinated residue CIT51. In another example, the peptide may comprise citrullinated residue CIT55. The peptide may comprise citrullinated residue CIT209. The peptide may comprise citrullinated residue CIT214. The peptide may comprise citrullinated residue CIT219. The peptide may comprise citrullinated residue CIT220. Alternatively, the peptide may comprise citrullinated residue CIT209 and/or CIT214. Alternatively, the peptide may comprise citrullinated residue CIT219 and/or CIT220. Alternatively, the peptide may comprise citrullinated residue CIT222. Combinations of the above peptides may be used in a pool. An equivalent non-citrullinated peptide of the same sequence may be used as the assay control.

The citrullinated tenascin-C peptide, or fragment thereof, for use in a peptide assay may be in a looped or cyclic formation, for example to aid antibody recognition.

The citrullinated tenascin-C peptide, or fragment thereof, for use in a peptide assay may comprise a sequence of part of SEQ ID NO: 70 having one or more citrullinated residues selected from CIT50, CIT51, CIT55, CIT72, CIT120, CIT169, CIT173, CIT209, CIT214, CIT219, CIT220 and CIT222; or combinations thereof. Alternatively, the citrullinated tenascin-C peptide, or fragment thereof, for use in a peptide assay may comprise a sequence of part of SEQ ID NO: 70 having one or more citrullinated residues selected from CIT55, CIT72, CIT120, CIT169, CIT173, CIT209, CIT214, CIT219, and CIT220; or combinations thereof. The citrullinated tenascin-C peptide, or fragment thereof, for use in a peptide assay may comprise a sequence of part of SEQ ID NO: 70 having citrullinated residue CIT50. The citrullinated tenascin-C peptide, or fragment thereof, for use in a peptide assay may comprise a sequence of part of SEQ ID NO: 70 having citrullinated residue CIT51. The citrullinated tenascin-C peptide, or fragment thereof, for use in a peptide assay may comprise a sequence of part of SEQ ID NO: 70 having citrullinated residue CIT55. The citrullinated tenascin-C peptide, or fragment thereof, for use in a peptide assay may comprise a sequence of part of SEQ ID NO: 70 having citrullinated residue CIT72. The citrullinated tenascin-C peptide, or fragment thereof, for use in a peptide assay may comprise a sequence of part of SEQ ID NO: 70 having citrullinated residue CIT120. The citrullinated tenascin-C peptide, or fragment thereof, for use in a peptide assay may comprise a sequence of part of SEQ ID NO: 70 having citrullinated residue CIT169. The citrullinated tenascin-C peptide, or fragment thereof, for use in a peptide assay may comprise a sequence of part of SEQ ID NO: 70 having citrullinated residue CIT173. The citrullinated tenascin-C peptide, or fragment thereof, for use in a peptide assay may comprise a sequence of part of SEQ ID NO: 70 having citrullinated residue CIT209. The citrullinated tenascin-C peptide, or fragment thereof, for use in a peptide assay may comprise a sequence of part of SEQ ID NO: 70 having citrullinated residue CIT214. The citrullinated tenascin-C peptide, or fragment thereof, for use in a peptide assay may comprise a sequence of part of SEQ ID NO: 70 having citrullinated residue CIT219. The citrullinated tenascin-C peptide, or fragment thereof, for use in a peptide assay may comprise a sequence of part of SEQ ID NO: 70 having citrullinated residue CIT220. The citrullinated tenascin-C peptide, or fragment thereof, for use in a peptide assay may comprise a sequence of part of SEQ ID NO: 70 having citrullinated residue CIT222. Two or more different fragments of SEQ ID NO: 70 may be used, which have different citrullinated residues relative to each other. Reference to fragments or sequences of SEQ ID NO: 70 may also include variants thereof. For example, a variant may have a sequence from SEQ ID NO: 70 plus additional amino acid residues or modifications.

The citrullinated tenascin-C peptide or the sequence of part of SEQ ID NO: 70 may comprise between about 5 and about 25 consecutive amino acid residues. The citrullinated tenascin-C peptide or the sequence of part of SEQ ID NO: 70 may comprise between about 10 and about 25 consecutive amino acid residues. The citrullinated tenascin-C peptide or the sequence of part of SEQ ID NO: 70 may comprise between about 15 and about 25 consecutive amino acid residues. The citrullinated tenascin-C peptide or the sequence of part of SEQ ID NO: 70 may comprise between about 18 and about 22 consecutive amino acid residues. The citrullinated tenascin-C peptide or the sequence of part of SEQ ID NO: 70 may comprise between about 8 and about 20 consecutive amino acid residues.

For example, the citrullinated tenascin-C peptide for use in a peptide assay may comprise a peptide sequence selected from any of the group comprising SEQ ID NO: 55, SEQ ID NO: 57, SEQ ID NO: 59, SEQ ID NO: 61, SEQ ID NO: 63 and SEQ ID NO: 64; or variants thereof and/or combinations thereof. The citrullinated tenascin-C peptide for use in a peptide assay may comprise a peptide sequence of SEQ ID NO: 55 or a variant thereof. The citrullinated tenascin-C peptide for use in a peptide assay may comprise a peptide sequence of SEQ ID NO: 57 or a variant thereof. The citrullinated tenascin-C peptide for use in a peptide assay may comprise a peptide sequence of SEQ ID NO: 59 or a variant thereof. The citrullinated tenascin-C peptide for use in a peptide assay may comprise a peptide sequence of SEQ ID NO: 61 or a variant thereof. The citrullinated tenascin-C peptide for use in a peptide assay may comprise a peptide sequence of SEQ ID NO: 63 or a variant thereof. The citrullinated tenascin-C peptide for use in a peptide assay may comprise a peptide sequence of SEQ ID NO: 64 or a variant thereof.

The presence, and optionally the level, of the citrullinated tenascin-C and/or one or more fragments of citrullinated tenascin-C in a sample may be determined by any suitable assay, which may comprise the use of any of the group comprising immunoassays, spectrometry, western blot, ELISA, immunoprecipitation, slot or dot blot assay, isoelectric focussing, SDS-PAGE and antibody microarray immunohistological staining, radio immuno assay (RIA), fluoroimmunoassay, an immunoassay using an avidin-biotin or streptoavidin-biotin system, etc or combinations thereof. These methods are well known to persons skilled in the art.

The agent, or composition as defined in the first, second, third or fourth aspects of the invention may relate to treatment of a chronic inflammatory condition wherein the condition is associated with any condition associated with inappropriate inflammation. Such conditions include, but are not limited to, rheumatoid arthritis (RA), autoimmune conditions, inflammatory bowel diseases (including Crohn's disease and ulcerative colitis), non-healing wounds, multiple sclerosis, cancer, atherosclerosis, sjogrens disease, diabetes, lupus erythrematosus (including systemic lupus erythrematosus), asthma, fibrotic diseases (including liver cirrhosis), pulmonary fibrosis, UV damage, psoriasis, ankylosing spondylitis and cardiovascular disease.

A method of determining the appropriate treatment for a subject having a chronic inflammatory condition based on the presence or absence of: (i) citrullinated tenascin-C and/or one or more fragments of citrullinated tenascin-C; and/or (ii) autoantibodies having specificity for citrullinated tenascin-C and/or for one or more fragments of citrullinated tenascin-C

In one example the method comprises the steps of:
(i) providing a sample derived from the subject;
(ii) testing the sample for the presence of citrullinated tenascin-C and/or one or more fragments of citrullinated tenascin-C and/or autoantibodies having specificity for citrullinated tenascin-C and/or for one or more fragments of citrullinated tenascin-C, wherein the presence or absence of citrullinated tenascin-C and/or one or more fragments of citrullinated tenascin-C and/or autoantibodies having specificity for citrullinated tenascin-C and/or for one or more fragments of citrullinated tenascin-C determines the appropriate treatment.

In an alternative example the method comprises testing a sample from the subject for the presence of citrullinated tenascin-C and/or one or more fragments of citrullinated tenascin-C and/or autoantibodies having specificity for citrullinated tenascin-C and/or for one or more fragments of citrullinated tenascin-C, wherein the presence or absence of citrullinated tenascin-C and/or one or more fragments of citrullinated tenascin-C and/or autoantibodies having specificity for citrullinated tenascin-C and/or for one or more fragments of citrullinated tenascin-C determines the appropriate treatment.

For example, the sample is a sample of blood, synovial fluid and/or joint tissue derived from the subject.

In one example the presence of citrullinated tenascin-C and/or one or more fragments of citrullinated tenascin-C and/or autoantibodies having specificity for citrullinated tenascin-C and/or for one or more fragments of citrullinated tenascin-C determines that the subject should be administered an effective amount of an agent or composition as defined herein.

In some examples, the method is for determining the appropriate treatment for a subject having an inflammatory disorder.

In one example, the method comprises the steps of:
(i) providing a sample derived from the subject; and
(ii) testing the sample for the presence of (i) citrullinated tenascin-C and/or one or more fragments of citrullinated tenascin-C; and/or (ii) autoantibodies with specificity for citrullinated tenascin-C and/or one or more fragments of citrullinated tenascin-C, wherein the presence or absence of (i) citrullinated tenascin-C and/or one or more fragments of citrullinated tenascin-C; and/or (ii) autoantibodies with specificity for citrullinated tenascin-C and/or one or more fragments of citrullinated tenascin-C, indicates the appropriate treatment.

In a preferred example the presence of (i) citrullinated tenascin-C and/or one or more fragments of citrullinated tenascin-C; and/or (ii) autoantibodies with specificity for citrullinated tenascin-C and/or one or more fragments of citrullinated tenascin-C, determines that the subject should be administered an effective amount of an agent or composition, the agent or composition may be one or more of anti-TNF drug; an anti-IL17 therapy; a T-cell co-stimulation modulator (such as Orencia™ - abatacept): an interleukin-6 (IL-6) inhibitor (such as Actemra™ - tocilizumab); an anti-CD20 antibody (such as Rituxan™ - rituxumab; a B cell activating factor (such as anti-BAFF); an inhibitor of janus kinase (JAK) (such as Tofacitinib™); an inhibitor of spleen tyrosine kinase (Syk) (such as Fostamatinib™); antiTNC antibodies or antibodies to citrullinated tenascin-C domains, and an agent that modulates the biological activity of citrullinated and/or non-citrullinated tenascin-C.
Citrullinated tenascin-C and/or one or more fragments of citrullinated tenascin-C may be used in a method of determining the appropriate treatment for a subject having a chronic inflammatory condition.

An autoantibody having specificity for citrullinated tenascin-C and/or for one or more fragments of citrullinated tenascin-C may be used in a method of determining the appropriate treatment for a subject having a chronic inflammatory condition.

Treatment is the administering of an effective amount of an agent or composition as defined in the first, second, third and fourth aspects of the invention. In preferred embodiments, the presence of citrullinated tenascin-C and/or one or more fragments of citrullinated tenascin-C and/or autoantibodies having specificity for citrullinated tenascin-C and/or for one or more fragments of citrullinated tenascin-C determines the appropriate treatment that should be administered.

The method, citrullinated tenascin-C, one or more fragments of citrullinated tenascin-C or autoantibody as defined herein may relate to determining the appropriate treatment of a chronic inflammatory condition wherein the condition is associated with any condition associated with inappropriate inflammation. Such conditions include, but are not limited to, rheumatoid arthritis (RA), autoimmune conditions, inflammatory bowel diseases (including Crohn's disease and ulcerative colitis), non-healing wounds, multiple sclerosis, cancer, atherosclerosis, sjogrens disease, diabetes, lupus erythrematosus (including systemic lupus erythrematosus), asthma, fibrotic diseases (including liver cirrhosis), pulmonary fibrosis, UV damage, psoriasis, ankylosing spondylitis and cardiovascular disease.

Detecting the presence or absence, or the level, of citrullinated tenascin-C and/or one or more fragments of citrullinated tenascin-C may comprise the detection of tenascin-C, or fragments thereof, comprising one or more specific citrullinated residue(s). The specific citrullinated residue(s) may be selected from any of the group comprising residues 50, 51, 55, 72, 120, 169, 173, 209, 214, 219, 220 and 222; or combinations thereof (residue numbers as determined from SEQ ID NO: 70). Alternatively, the specific citrullinated residue(s) may be selected from any of the group comprising residues 55, 72, 120, 169, 173, 209, 214, 219, and 220; or combinations thereof (residue numbers as determined from SEQ ID NO: 70). The specific citrullinated residues may comprise CIT55, CIT209, CIT214, CIT219 and/or CIT220. The specific citrullinated residue may comprise CIT50. The specific citrullinated residue may comprise CIT51. The specific citrullinated residue may comprise CIT55. The specific citrullinated residue may comprise CIT209. The specific citrullinated residue may comprise CIT214. The specific citrullinated residue may comprise CIT219. The specific citrullinated residue may comprise CIT220. In another embodiment, the specific citrullinated residues may comprise CIT209 and/or CIT214. Alternatively, the specific citrullinated residues may comprise CIT219 and/or CIT220.

The specific citrullinated residues may be, or be found in, an epitope and may be detected in such an epitope. For example, the epitope described herein.

### Definitions

By "inflammation" we include the meaning of local accumulation of fluid, plasma proteins, and white blood cells that is initiated by tissue injury, infection or a local immune response.

By "acute inflammation" we include the meaning of the initial stages (initiation) of inflammation and the short-term transient inflammatory response immediately after injury, infection or local immune response. Typically, acute inflammation is rapidly resolved, lasting from a matter of minutes to no longer that a few days.

By "chronic inflammation" we include the meaning of persistent and/or non-resolved inflammation. It is often associated with inappropriate destruction of healthy tissue. This may be progressive and last over a period of weeks or longer. Chronic inflammation is typically associated with persistent infection or disease including, but not limited to, automimmune conditions.

By "chronic joint inflammation" we include the meaning of persistent inflammation that is progressive and unremitting over a period of weeks to months, resulting in distortion of the affected joint and radiographic evidence of cartilage and bone destruction as observed in human disease (Kelly, Harris, Ruddy and Sledge, Textbook of Rheumatology 4th Edition).

In experimental murine models, chronic joint inflammation is characterised by inflammation that does not subside and causes inappropriate tissue destruction, even over a relatively short period of time. This is characterized (and can be identified) histologically by the prolonged presence of inflammatory cells in the synovium and joint space, chondrocyte death, and cartilage and bone erosion.

By an "agent" we include all chemical entities, for example oligonucleotides, polynucleotide, polypeptides, peptidomimetics and small compounds.

By "citrullinated" we mean the conversion of one or more arginine amino acids in a protein into the amino acid citrulline.

By "a fragment of citrullinated tenascin-C" or "one or more fragments of citrullinated tenascin-C" we mean a citrullinated peptide or domain derived from citrullinated tenascin-C. The fragment of citrullinated tenascin-C may be a citrullinated FBG domain, a citrullinated TA domain, a citrullinated EGF-L domain, a citrullinated TNIII domain or any other sequence from within citrullinated tenascin-C. Preferably the fragment of citrullinated tenascin-C is antigenic. Preferably the fragment of citrullinated tenascin-C is biologically active.

By "fragment" we mean at least 10 nucleotides, for example at least 15, 16, 17, 18, 19, 20, 21, 22, 23, 24 or 25 nucleotides.

By "variant" we mean that the nucleotide sequence shares at least 90% sequence identity with the full length sequence of interest, for example at least 50%, 55%, 60%, 65%, 70%, 75%, 80%, 85%, 90%, 95%, 96%, 97%, 98% or 99% sequence identity.

The percent sequence identity between two polynucleotides may be determined using suitable computer programs, for example the GAP program of the University of Wisconsin Genetic Computing Group and it will be appreciated that percent identity is calculated in relation to polynucleotides whose sequences have been aligned optimally.

The alignment may alternatively be carried out using the Clustal W program (as described in Thompson et al., 1994, Nuc. Acid Res. 22:4673-4680).

The parameters used may be as follows:
Fast pairwise alignment parameters: K-tuple(word) size; 1, window size; 5, gap penalty; 3, number of top diagonals; 5. Scoring method: x percent.

Multiple alignment parameters: gap open penalty; 10, gap extension penalty; 0.05.

Scoring matrix: BLOSUM.

Alternatively, the BESTFIT program may be used to determine local sequence alignments.

By "antibody" we include substantially intact antibody molecules, as well as chimaeric antibodies, humanised antibodies, human antibodies (wherein at least one amino acid is mutated relative to the naturally occurring human antibodies), single chain antibodies, bispecific antibodies, antibody heavy chains, antibody light chains, homodimers and heterodimers of antibody heavy and/or light chains, and antigen binding fragments and derivatives of the same.

By "autoantibody" we mean any antibody manufactured by a subject's immune system that is directed against one or more of the subject's own proteins.

An "autoantigen" is an endogenous antigen that stimulates the production of autoantibodies.

By "antigen-binding fragment" we mean a functional fragment of an antibody that is capable of binding to citrullinated tenascin-C.

The term "subject" means all animals including humans. Examples of subjects include humans, cows, dogs, cats, goats, sheep, and pigs. The term "patient" means a subject having a disorder in need of treatment.

As used herein, 'pharmaceutical formulation' means a therapeutically effective formulation according to the invention.

A 'therapeutically effective amount', or 'effective amount', or 'therapeutically effective', as used herein, refers to that amount which provides a therapeutic effect for a given condition and administration regimen. This is a predetermined quantity of active material calculated to produce a desired therapeutic effect in association with the required additive and diluent, *i.e.* a carrier or administration vehicle. Further, it is intended to mean an amount sufficient to reduce and most preferably prevent, a clinically significant deficit in the activity, function and response of the host. Alternatively, a therapeutically effective amount is sufficient to cause an improvement in a clinically significant condition in a host. As is appreciated by those skilled in the art, the amount of a compound may vary depending on its specific activity. Suitable dosage amounts may contain a predetermined quantity of active composition calculated to produce the desired therapeutic effect in association with the required diluent. In the methods and use for manufacture of compositions of the invention, a therapeutically effective amount of the active component is provided. A therapeutically effective amount can be determined by the ordinary skilled medical or veterinary worker based on patient characteristics, such as age, weight, sex, condition, complications, other diseases, *etc*., as is well known in the art.

Examples embodying an aspect of the invention will now be described with reference to the following figures:

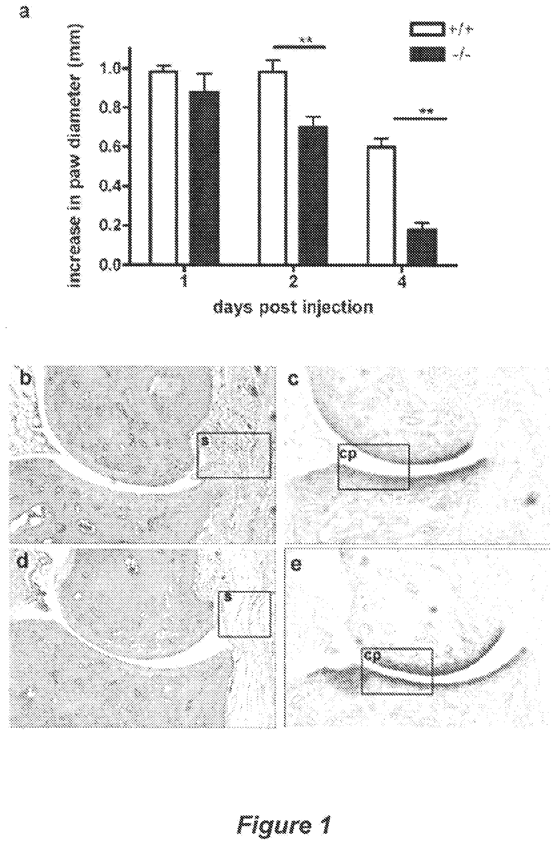

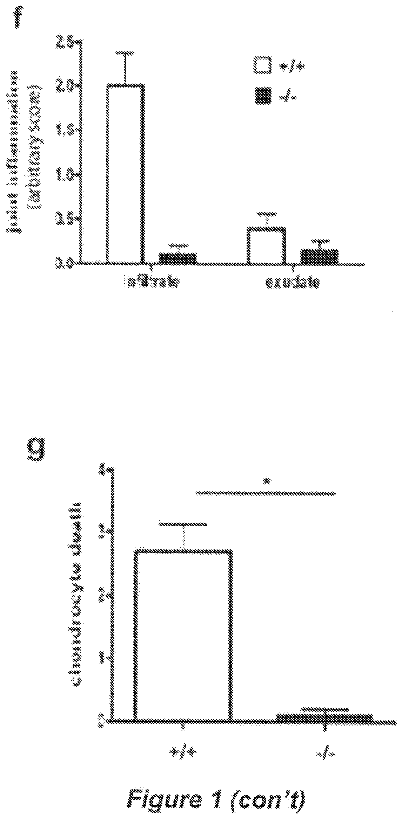

**Figure 1****. Accelerated resolution of acute inflammation in tenascin-C deficient mice.**
   (a) Paw swelling in wild type (+/+)(white bars) and tenascin-C null (-/-)(black bars) mice over time after injection of zymosan. Data are shown as the mean increase in paw diameter compared to paw diameter before injection +/-SEM (n = 24 mice per genotype). **= p<0.01. (b-e) Representative sections of the ankle joint from wild type (b, c) and tenascin-C null (d, e) mice 4 days after zymosan injection, stained with hemotoxylin and eosin (b, d) and safranin-O (c, e). Boxes highlight the joint synovium (s) and cartilage proteoglycan (cp). Magnification x10. Quantification of joint inflammation (f) and chondrocyte death (g) in knee joints 4 days after injection with zymosan from wild type mice (white bars) and tenascin-C null mice (black bars). Data are expressed as the mean (+/- SD) (n = 24 mice per genotype). *= p <0.05.

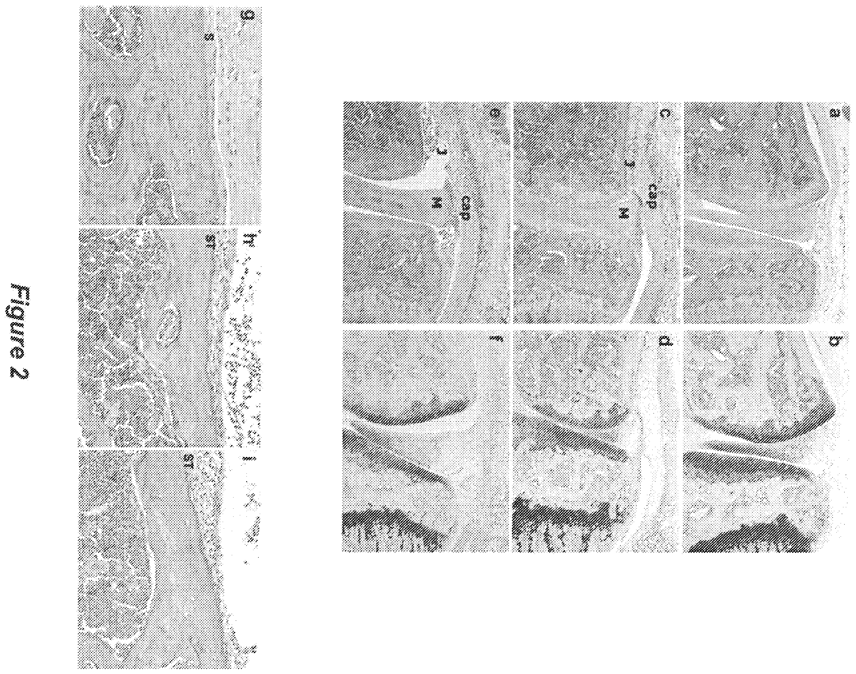

**Figure 2****. Synovial inflammation is induced in tenascin-C deficient mice upon injection of antigen.**
   (a-b, g) Representative sections of the knee joint of sham injected wild type mice. (c-f, h-i) Representative sections of the knee joint of wild type (c, d, h) or tenascin-C null (e, f, i) mice 24 hours after intra-articular injection of mBSA. Inflammatory cell infiltration in the capsule, meniscus and the joint space of both wild type and tenascin-C null mice is highlighted by (cap), (M) and (J) respectively. (S) highlights the healthy synovium of sham injected mice that is no more than 1-3 cells thick along the entire bone surface and (ST) highlights the synovia of wild type and tenascin-C null mice which are both significantly thickened. Sections are stained with hemotoxylin and eosin (a, c, e, g, h, i) and safranin-O (b, d, f). Magnification x10 (a-f) or x40 (g-i). (n = 5 mice per genotype).

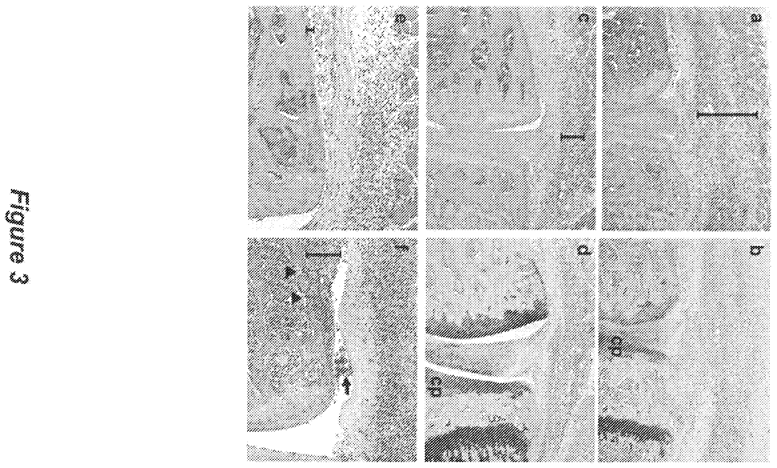

**Figure 3****. Synovial inflammation subsides rapidly in tenascin-C deficient mice.**
   Representative sections of the knee joint of wild type (a, b, f) or tenascin-C null (c, d, e) mice 3 days after intra-articular injection of mBSA. (a, c) The line highlights increased inflammation of the capsule in wild type mice compared to tenascin-C null mice. (b, d) (cp) highlights increased cartilage proteoglycan loss in wild type mice compared to tenascin-C null mice. (e, f) Significant synovial hyperplasia (line), cell and fibrin deposits in the joint space (arrow) and pannus invasion (arrow heads) are observed in wild type mice compared to tenascin-C null mice. Sections are stained with hemotoxylin and eosin (a, c, e, f) and safranin-O (b, d) Magnification x10 (a-d) or x20 (e-f). (n = 5 mice per genotype).

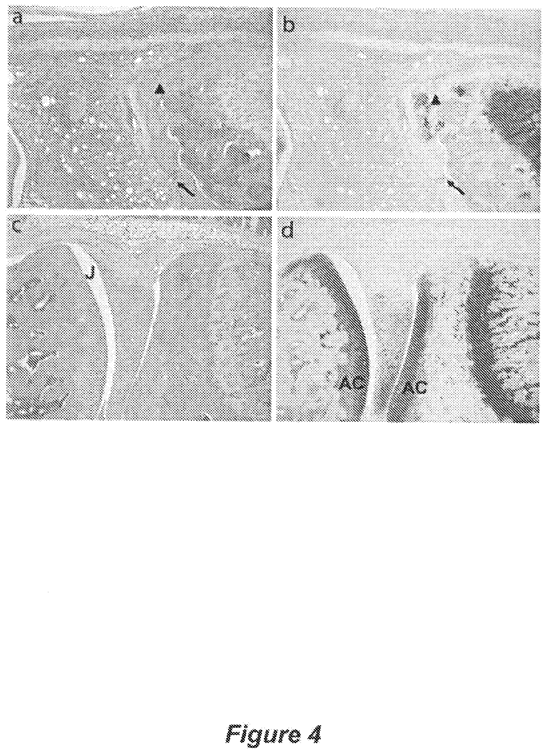

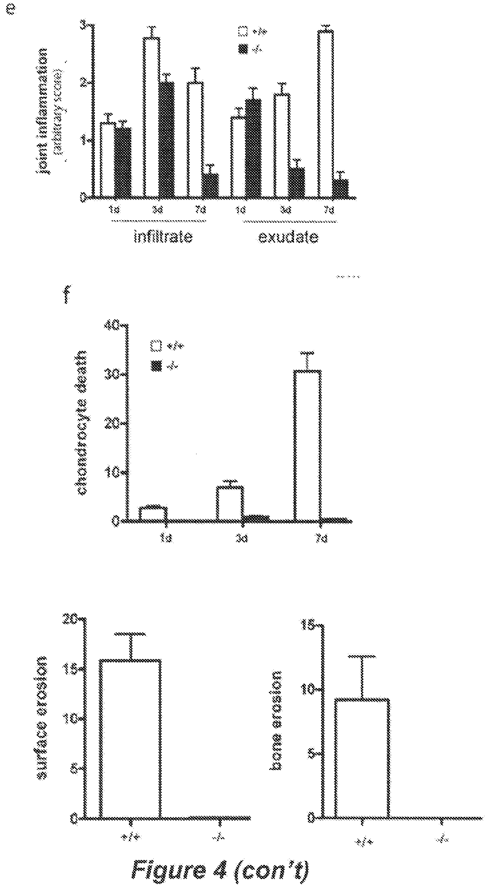

**Figure 4****. tenascin-C deficient mice are protected from tissue destruction during antigen induced arthritis.**
   (a-b) Representative sections of the knee joint of wild type mice 7 days after intra-articular injection of mBSA, stained with hemotoxylin and eosin (a) and safranin-O (b). Magnification x10. (n = 24 mice per genotype). Arrowhead highlights area of bone erosion. Arrow highlights pannus invasion into articular cartilage. (c-d) Representative sections of the knee joint of tenascin-C null type mice 7 days after intra-articular injection of mBSA, stained with hemotoxylin and eosin (c) and safranin-O (d). Magnification x10. (n = 24 mice per genotype). J highlights the joint space and AC the intact articular cartilage. (e) Histological score of knee joint inflammation 24 hours, 3 days and 7 days after injection with mBSA from wild type mice (white bars) and tenascin-C null mice (black bars). Data represent the mean +/- SD (n = 5 per genotype (24h, 3d) or 24 per genotype (7d)). (f) Quantification of chondrocyte death, cartilage surface erosion and bone erosion after injection with mBSA in knee joints from wild type mice (white bars) and tenascin-C null mice (black bars). Chondrocyte death is shown at 24 hours, 3 days and 7 days, and cartilage surface erosion and bone erosion at 7d. Data represent the mean +/- SD (n = 5 per genotype (24h, 3d) or 24 per genotype (7d)).

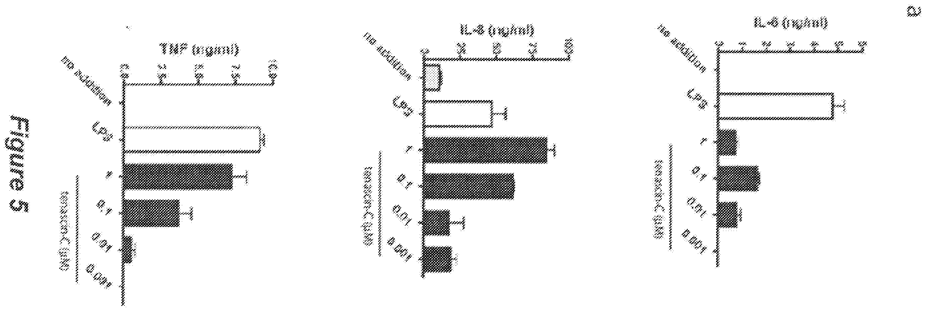

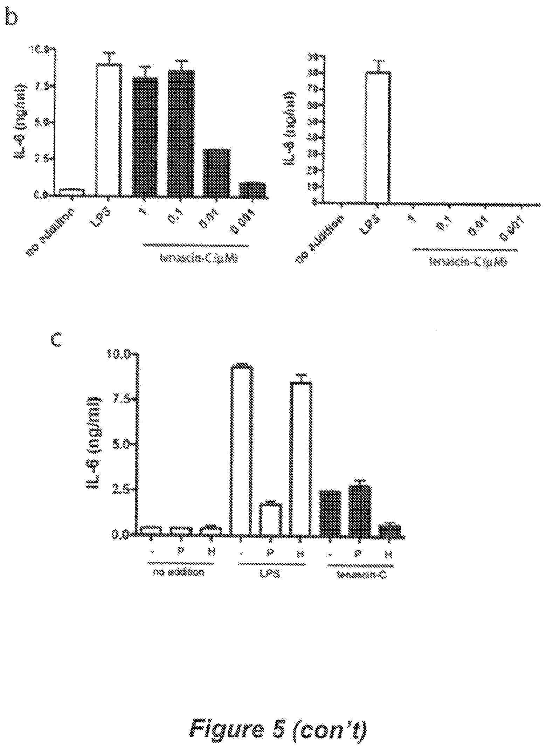

**Figure 5****. tenascin-C induces TNF-α, IL-6 and IL-8 synthesis in primary human macrophages and RA synovial fibroblasts.**
   (a-b) Primary human macrophages (a) and RA synovial fibroblasts (b) were unstimulated (no addition) or stimulated with LPS (1 ng/ml (a) or 10 ng/ml (b)) or recombinant tenascin-C (1.0 µM - 1.0 nM) for 24h. Data shown are the mean of triplicate values (+/-SD) from one of three representative experiments. (c) Primary human macrophages were unstimulated (no addition) or stimulated with LPS (1 ng/ml) or recombinant tenascin-C (1.0 µM) for 24h. (-) indicates cells were pre-incubated with medium alone. (P) Cells were pre-incubated with 25 µg/ml polymyxin B for 30 min before stimulation. (H) Cells were incubated with medium with no addition or containing LPS or tenascin-C that was boiled for 15 minutes before addition to cells. Data shown are the mean of triplicate values (+/- SD) from one of three representative experiments.

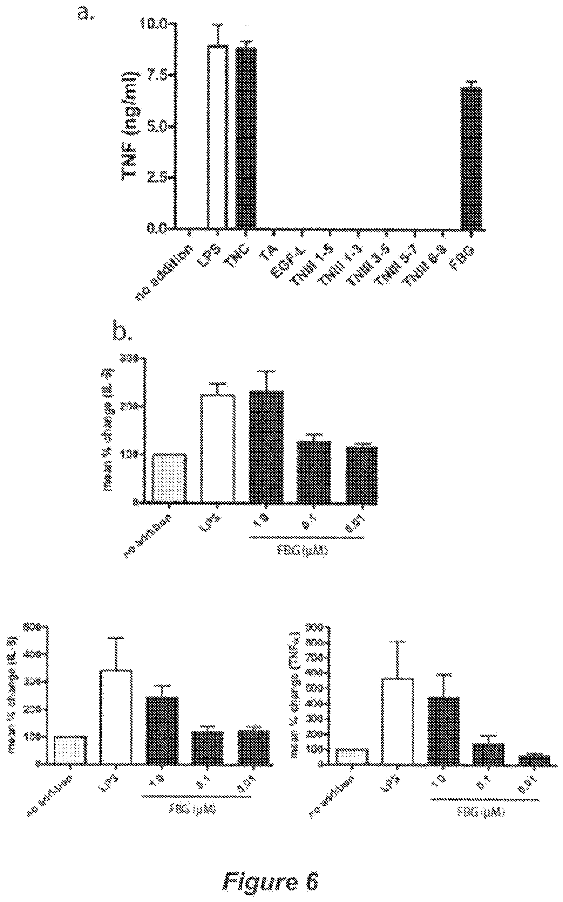

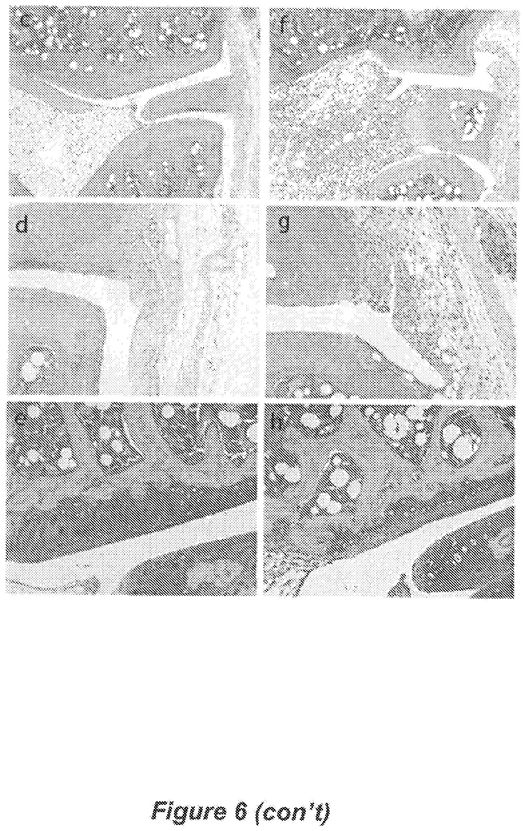

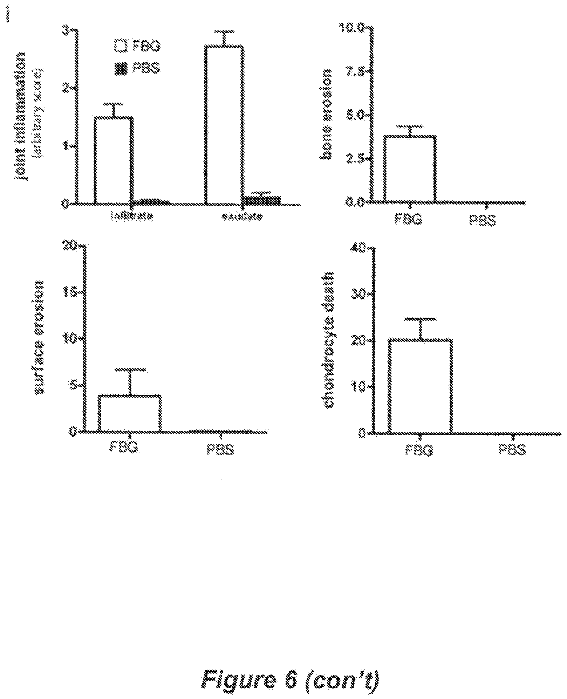

**Figure 6****. The FBG domain of tenascin-C mediates stimulation of cytokine synthesis *in vivo* and *in vitro.***
   (a) Primary human macrophages were unstimulated (no addition) or stimulated with LPS (1 ng/ml), recombinant tenascin-C (TNC) or 1.0 µM tenascin-C domains (TA, EGF-L, TNIII1-5, TNIII1-3, TNIII3-5, TNIII5-7, TNIII6-8 and FBG) for 24h. Data shown are the mean of triplicate values (+/- SD) from one of three representative experiments. (b) RA synovial membrane cells were unstimulated (no addition) or stimulated with LPS (10 ng/ml) or recombinant FBG (1.0 - 0.01 µM) for 24h. Data shown are the mean % change in cytokine levels compared to unstimulated cells (+/- SEM) from five different patients. (c-h) Representative sections of the knee joint of wild type mice 3 days after intra-articular injection of PBS (c-e) or 1 µg FBG (f-h). Sections are stained with hemotoxylin and eosin (c,d,f,g) or Safranin-O (e, h). Magnification x10 (c, f) or x25 (d,e,g,h) (n = 5 mice per genotype). (i) Quantification of joint inflammation, bone erosion, cartilage surface erosion and chondrocyte death in the knee joints of wild type mice 3 days after intra-articular injection of PBS (black bars) or 1 µg FBG (white bars). Data represent the mean +/- SD (n = 5 per genotype).

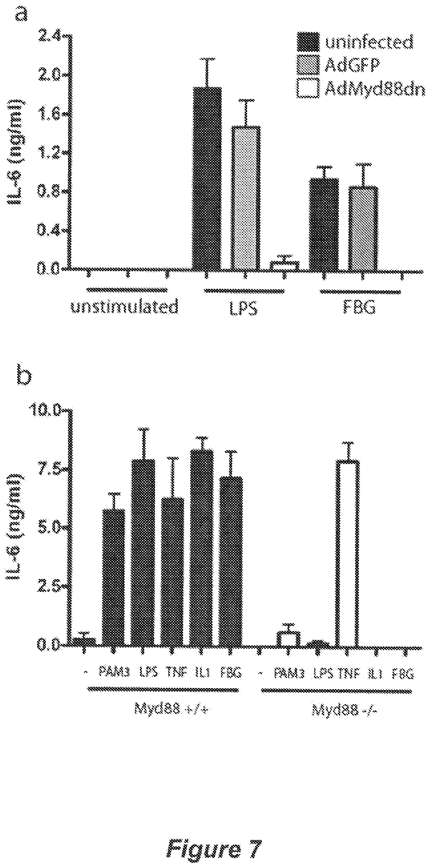

**Figure 7****. FBG mediated cytokine synthesis is MyD88 dependent.**
   (a) Human RA synovial fibroblasts were either uninfected, infected with adenovirus expressing GFP alone (AdGFP) or infected with adenovirus expressing dominant negative MyD88 (AdMyD88dn). Cells were unstimulated, stimulated with LPS (10 ng/ml) or stimulated with FBG (1µM) for 24h. Data shown are the mean of three independent experiments (+/- SEM). (b) Mouse embryonic fibroblasts isolated from wild type (+/+) or MyD88 deficient (-/-) mice were unstimulated (-) or stimulated with PAM3 (100 ng/ml), LPS (100 ng/ml), TNFα (100 ng/ml), IL-1 (5 ng/ml) and FBG (1 µM) for 24h. Data shown are the mean of three independent experiments (+/-SEM).

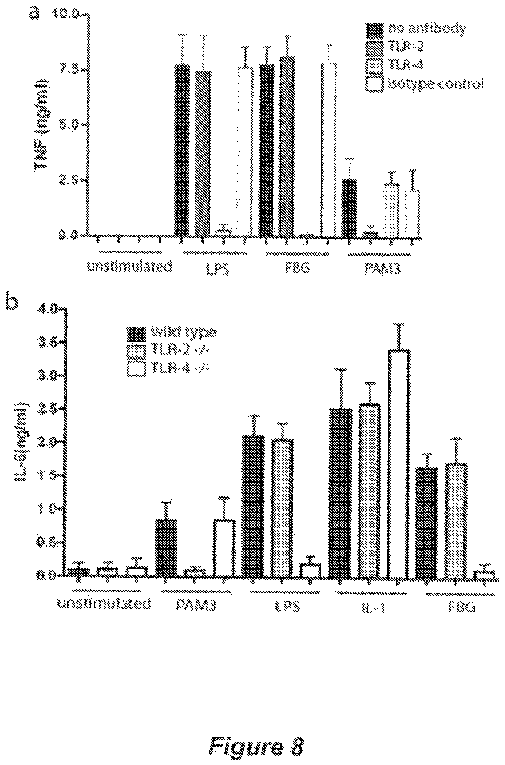

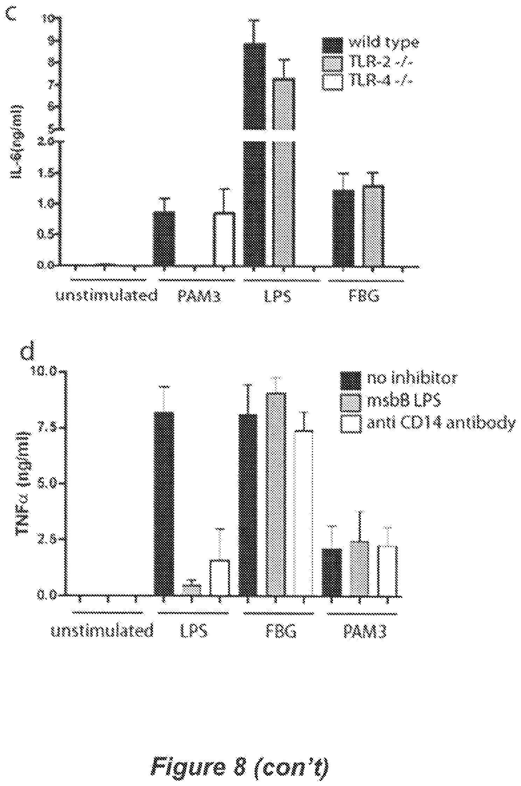

**Figure 8****. FBG mediated cytokine synthesis is TLR4 dependent but does not require CD14 or MD-2.**
   (a) Primary human macrophages were pre-incubated with medium alone or medium containing function blocking antibodies to TLR2 (10 µg/ml), TLR4 (25 µg/ml) or isotype control antibodies (25 µg/ml) for 30 min before stimulation. Cells were unstimulated, or stimulated with LPS (1 ng/ml), FBG (1 µM) or PAM3 (10 ng/ml) for 24h. Data shown are the mean of three independent experiments (+/-SEM). (b) Mouse embryonic fibroblasts isolated from wild type, TLR2 (TLR2 -/-) or TLR4 (TLR4 -/-) deficient mice were unstimulated or stimulated with PAM3 (100 ng/ml), LPS (100 ng/ml), IL-1 (5 ng/ml) and FBG (1 µM) for 24h. Data shown are the mean of three independent experiments (+/-SEM). (c) Bone marrow derived macrophages isolated from wild type, TLR2 (TLR2 -/-) or TLR4 (TLR4 -/-) deficient mice were unstimulated or stimulated with PAM3 (100 ng/ml), LPS (100 ng/ml) or FBG (1 µM) for 24h. Data shown are the mean of three independent experiments (+/-SEM). (d) Human macrophages were pre-incubated with no inhibitor, 1µg/ml msbB LPS or 10µg/ml anti-CD14 antibody for 30 min before stimulation with LPS (1 ng/ml), FBG (1 µM) or PAM3 (10 ng/ml) for 24h. Data shown are the mean of three independent experiments (+/-SEM).
**Figure 9****. Paw swelling over time after injection of zymosan.**
   Representative images of the paws of non-injected tenascin-C null mice (a, e) (diameter 1.6 mm), tenascin-C null mice 24h (d, f) (diameter 2.5 mm) and 4d (b, h) (diameter 1.7 mm) after zymosan injection and from wild type mice 4d after zymosan injection (c, g) (diameter 2.1 mm).
**Figure 10****. Synthesis of recombinant proteins.**
   (a) Domain structure of the tenascin-C monomer comprising different domains, including the assembly domain (TA), 14 and a half EGF-like repeats (EGF-L), 17 fibronectin type III-like repeats (TNIII) (8 constitutively expressed (1-8) and 9 that can be alternatively spliced, and a fibrinogen-like globe (FBG). (b) The regions covered by the recombinant proteins that were synthesized, the corresponding amino acid residues and the molecular weight of each protein.

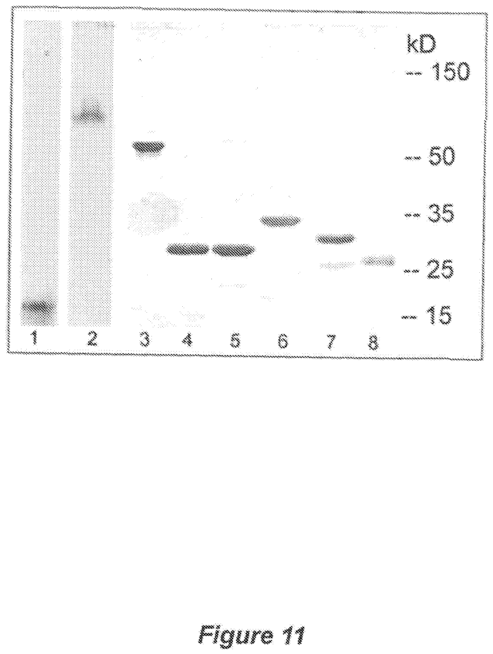

**Figure 11****. Analysis of protein purity.**
   Silver stained gel showing 1 µg of each recombinant protein analysed by SDS-PAGE under reducing conditions. Lanes: 1 (TA), 2 (EGF-L), 3 (TNIII1-5), 4 (TNIII5-7), 5 (TNIII6-8), 6 (TNIII1-3), 7 (TNIII3-5) and 8 (FBG).

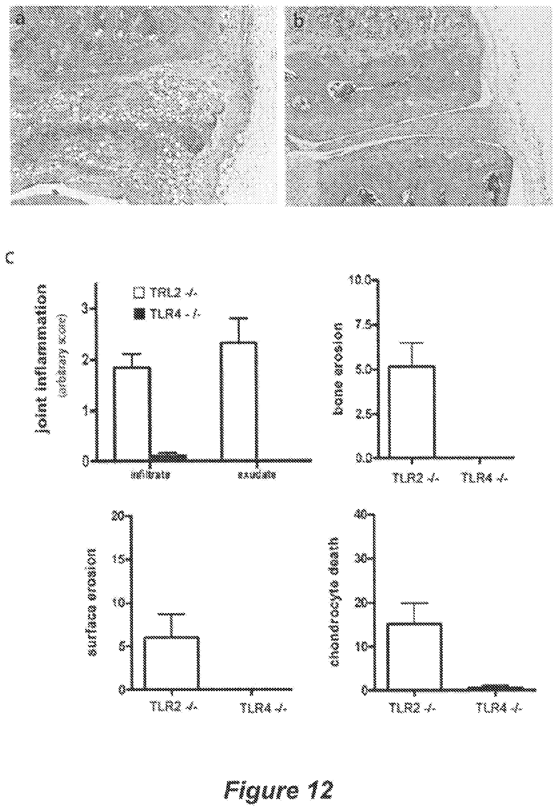

**Figure 12****. FBG-mediated joint inflammation in vivo requires expression of TLR4.**
   Representative sections of the knee joint of TLR2 (a) and TLR4 (b) null mice 3 days after intra-articular injection of 1 µg FBG. Sections are stained with hemotoxylin and eosin. Magnification x10 (n = 5 mice per genotype). (c) Quantification of joint inflammation, bone erosion, cartilage surface erosion and chondrocyte death in the knee joints of TLR2 (white bars) and TLR4 (black bars) null mice 3 days after intra-articular injection of 1 µg FBG. Data represent the mean +/- SD (n = 5 per genotype).
**Figure 13****. Amino acid sequence of human tenascin-C and its domains**

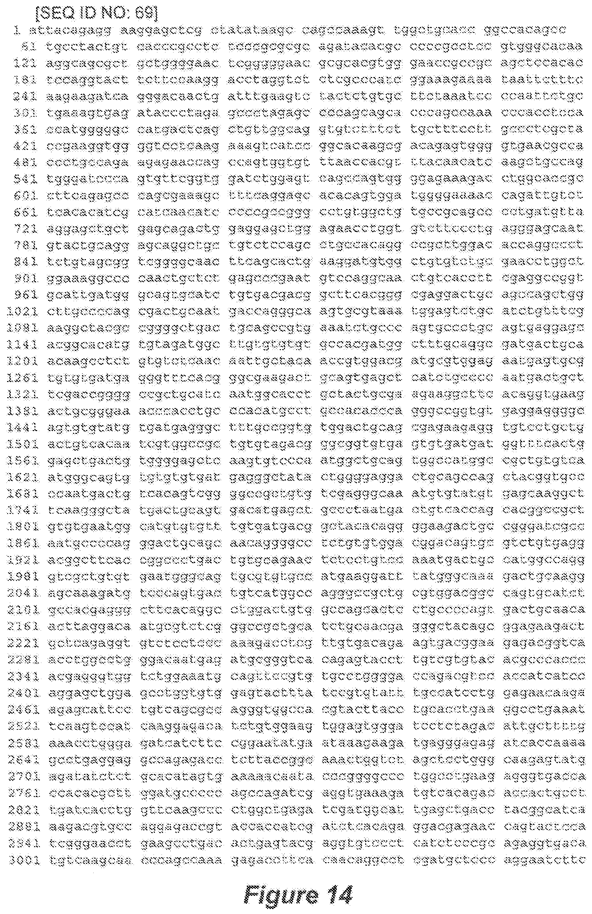

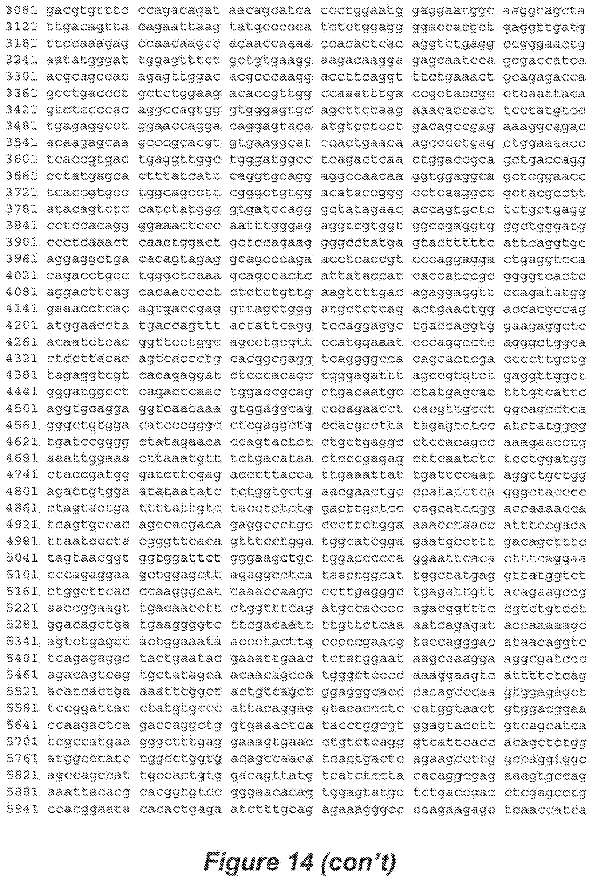

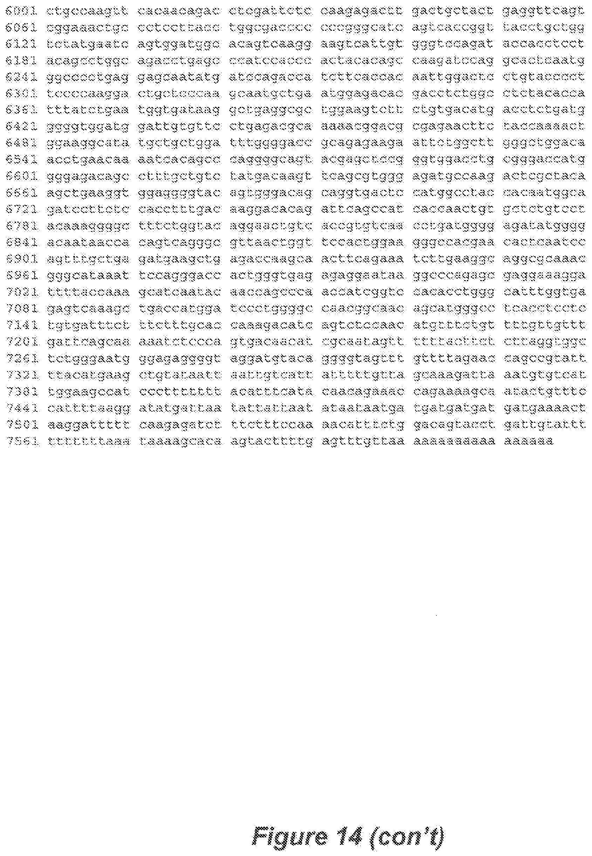

**Figure 14****. Nucleotide sequence of human tenascin-C**

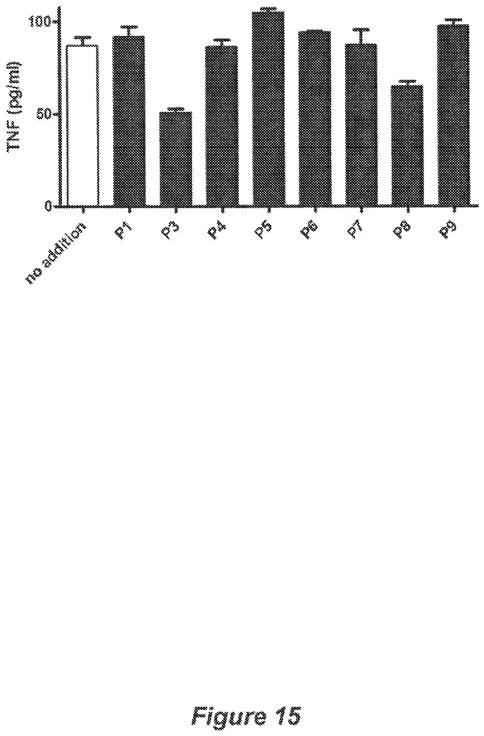

**Figure 15****. TNF synthesis in response to specific FBG peptides.**
   TNF synthesis by RA membrane cultures incubated for 24h with no addition or 100 µM of each FBG peptide (P1, P3-P9).

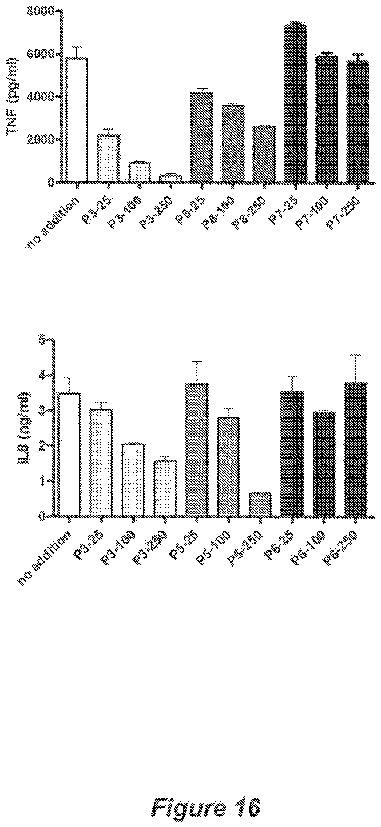

**Figure 16****. TNF and IL8 synthesis in response to varying concentrations of specific FBG peptides.**
   TNF and IL8 synthesis by RA membrane cultures incubated for 24h with no addition or 25, 100 or 250 µM of FBG peptide.

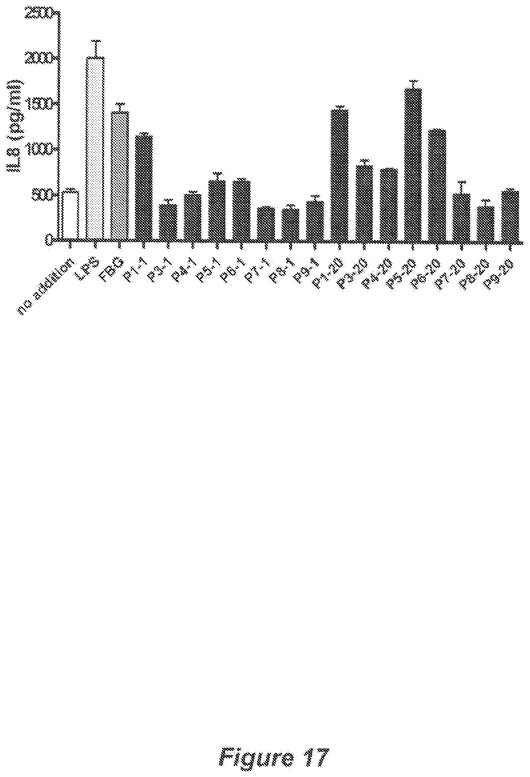

**Figure 17****. IL8 synthesis in response to LPS, whole FBG domain or specific FBG peptides.**
   IL8 synthesis by macrophages after 24h incubation with no addition, 1 ng/ml LPS, 1 µM whole FBG domain (FBG) or 1 or 20 µM of FBG peptides (P1, P3-P9).

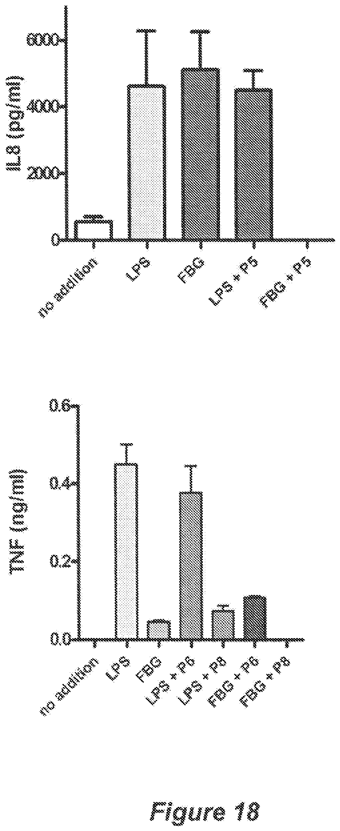

**Figure 18****. IL8 and TNF synthesis in response to LPS and FBG following pre-incubation with FBG peptides.**
   TNF and IL8 synthesis by macrophages after 24h incubation with no addition, 1 ng/ml LPS or 1 µM whole FBG domain (FBG), either with or without pre-incubation with 20 µM of FBG peptides.

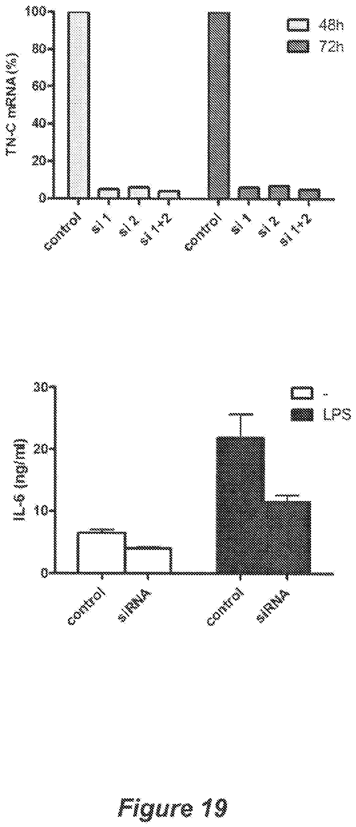

**Figure 19****. IL8 and TNF synthesis in response to tenascin-C targeted siRNAs.**
   Tenascin-C mRNA levels in RA fibroblasts transfected with luciferase specific siRNA (control), or with tenascin-C targeted siRNAs: oligo 1 (si 1), oligo 2 (si 2) or a combination of oligos 1+2 (si 1+2). IL6 synthesis in RA fibroblasts transfected with luciferase siRNA (control) or with a combination of tenascin-C targeted oligos 1+2 (siRNA) in the presence or absence of 10 ng/ml LPS for 24h.

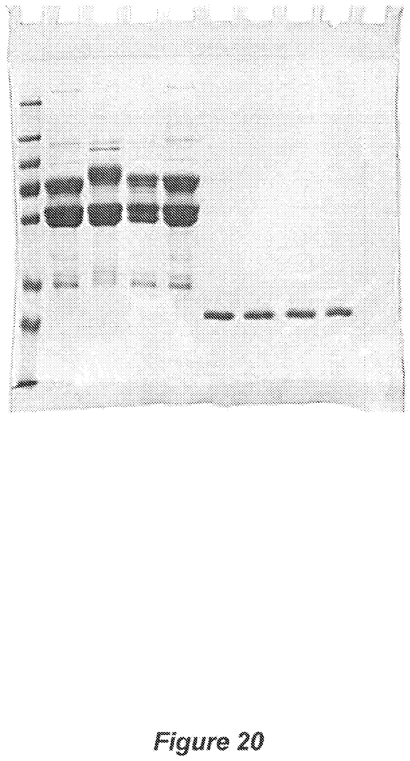

**Figure 20****. Purified fibrinogen and FBG can be citrullinated *in vitro***
   Coomassie stained gel showing purified fibrinogen (lanes 2-5) and FBG (lanes 6-9) that have been left unmodified (lanes 2, 6) or citrullinated by incubation with citrullination buffer, PAD and CaCI (lanes 3, 7). Citrullination of fibrinogen was confirmed by the observation of an increase in MW of this protein. However, changes in the size of FBG were less apparent. Incubation of proteins with PAD in the absence of CaCI or with CaCI in the absence of PAD had no effect on protein size (lanes 4, 5 and 8, 9).

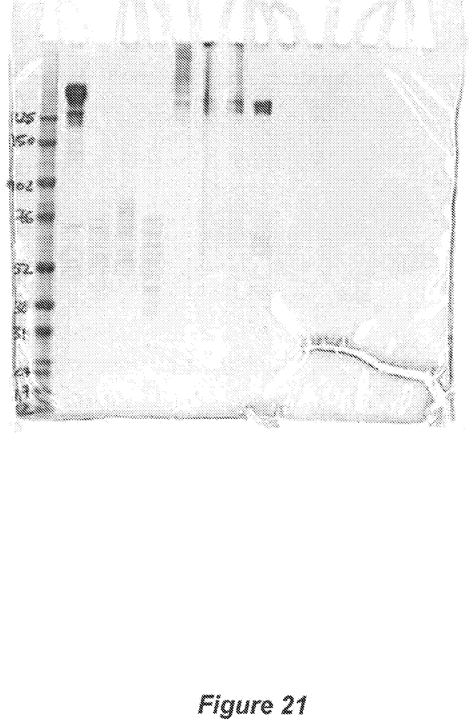

**Figure 21****. Purified full length tenascin-C can be citrullinated *in vitro***
   Coomassie stained gel showing purified full length tenascin-C that has been left unmodified (lane 9) or citrullinated by incubation with citrullination buffer, PAD and CaCI (lane 6). Citrullination of tenascin-C was confirmed by the observation of an increase in MW of this protein. Incubation of tenascin with PAD in the absence of CaCI or with CaCI in the absence of PAD had no effect on protein size (lanes 7,8). Purified fibronectin was included as a loading control (lane 2).

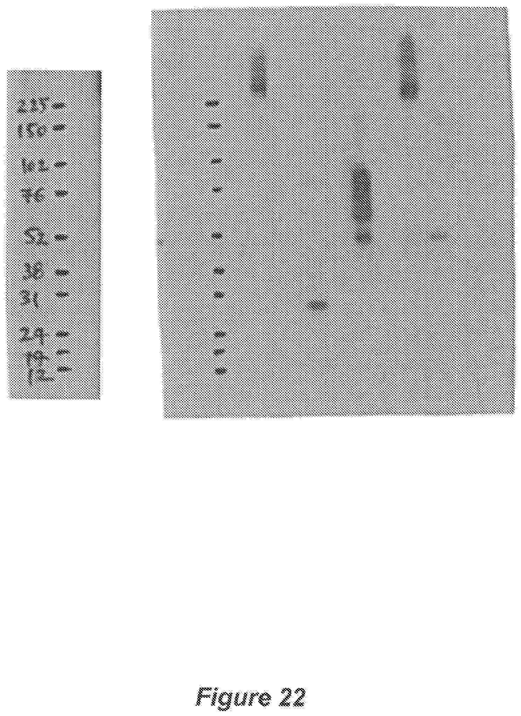

**Figure 22****. Western blot confirmation of citrullination of FBG and TNC**
   Western blot of native and citrullinated TNC (lanes 8, 9) and native and citrullinated FBG (lanes 4, 5) probed using the AMC detection kit. Native and citrullinated fibronectin (lanes 2, 3), native and citrullinated fibrinogen (lanes 6,7) and native and citrullinated enolase (lanes 11,10) were included as positive controls known to be citrullinated.

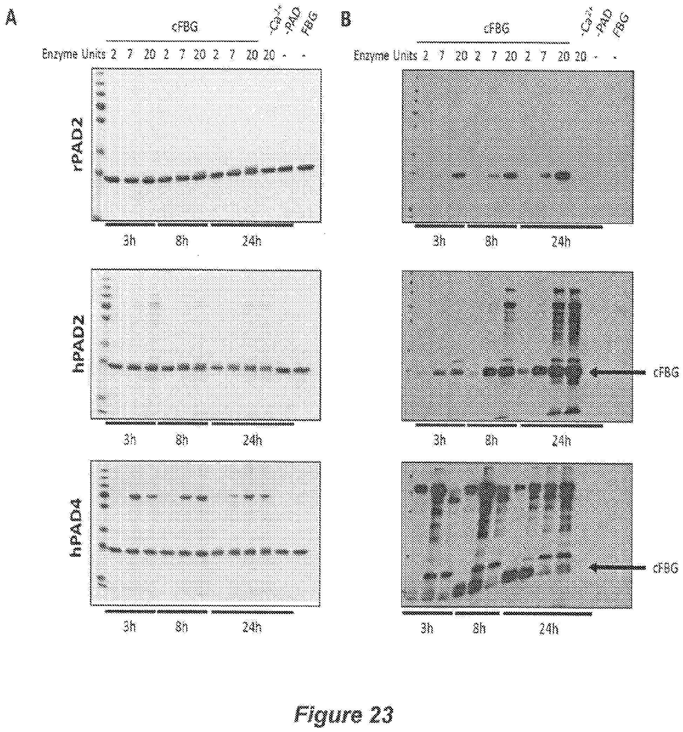

**Figure 23** **FBG is citrullinated by PAD in vitro**
   Purified recombinant FBG was citrullinated *in vitro* by incubating with different concentrations (2, 7 and 20 Units per mg protein) of rabbit PAD2, human PAD2 and human PAD4 in citrullination buffer (100 mM Tris pH 7.4, 10 mM CaCl₂, 5 mM DTT) for 3h, 8h and 24h at 37°C. As a negative control FBG-C was incubated in citrullination buffer without Calcium (-Ca²⁺) or without enzyme (-PAD). **(A)** 1 ug of each sample were resolved on SDS-PAGE and stained with Coomassie Blue. FBG citrullinated by PAD migrates at a slightly higher molecular weight than non-citrullinated FBG. **(B)** Proteins were transferred on nitrocellulose membranes and incubated in a chemical modification mix (0.0125 % FeCl₃, 2.3M H₂SO₄, 1.52 M H₃PO₄, 0.25 M Acetic Acid, 0.25% 2, 3-butanedione monoxime, 0.125% antipyrine). Citrullinated proteins were detected with an anti-modified citrulline specific antibody.

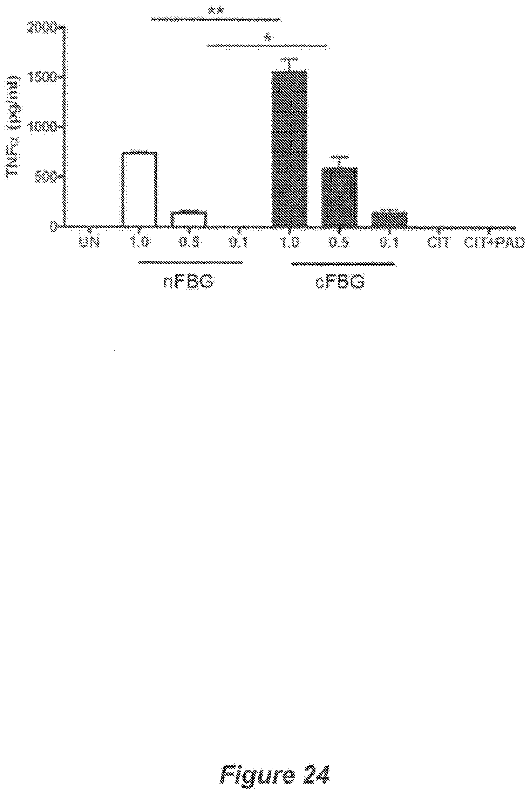

**Figure 24****. Citrullination enhances cytokine production stimulated by FBG**
   TNF synthesis in primary human macrophages left unstimulated (UN) or stimulated with 0.1 - 1.0 µM non-citrullinated (nFBG) or citrullinated (cFBG) FBG. Citrullination buffer alone (CIT) and buffer with PAD (CIT+PAD) were included as controls. FBG incubated with citrullination buffer in the absence of PAD or with PAD in the absence of calcium was not citrullinated and exhibited no enhancement of cytokine synthesis (not shown).

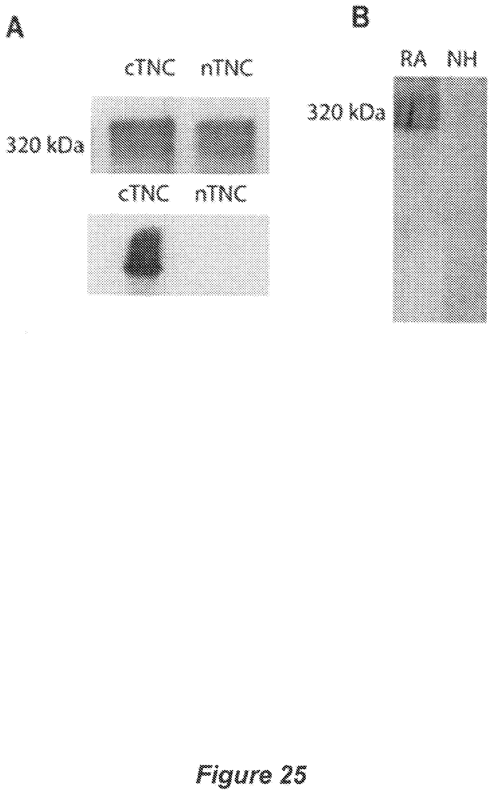

**Figure 25****. Ten of 50 RA patients (20%) and none of 50 controls react with citrullinated tenascin-C by Western blot.**
   (A) Coomassie stained gel of native (nTNC) and citrullinated (cTNC) purified human recombinant tenascin-C (top panel). Citrullination of tenascin-C demonstated by western blot with the AMC kit (bottom panel). (B) Representative western blot of cTNC probed with serum from RA patients (RA) or normal healthy controls (NH) (n=50). No reactivity was observed with any sera in blots of nTNC (not shown).

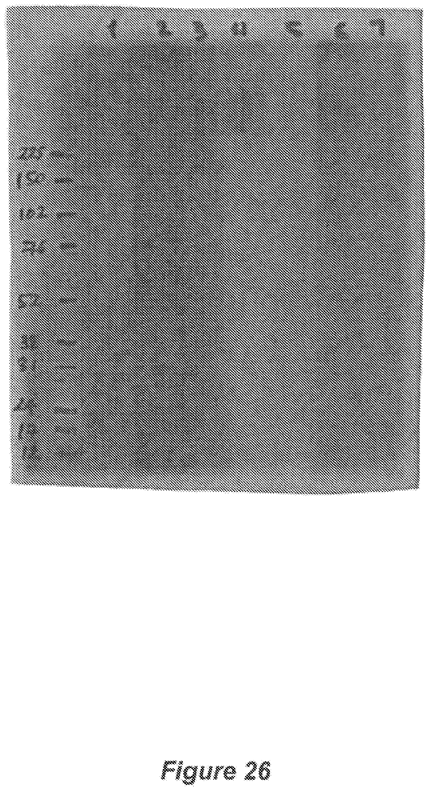

**Figure 26****. Serum from a subset of RA patients exhibits reactivity with citTNC**
   Western blot of cTNC probed with serum from 7 different RA patients showing one positive patient (lane 4).

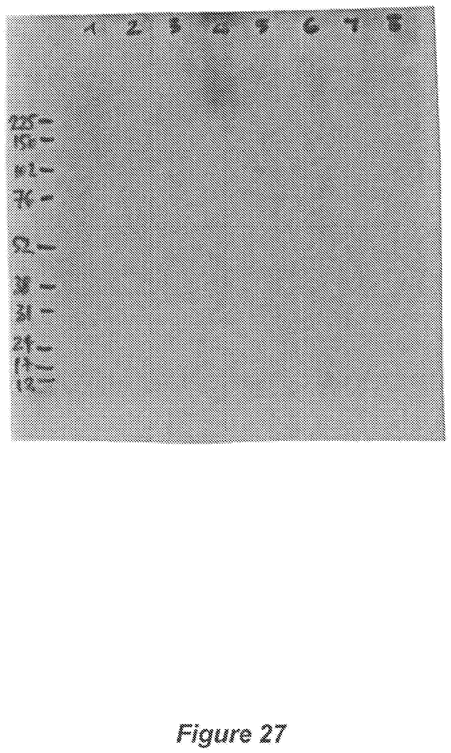

**Figure 27****. Serum from a subset of RA patients exhibits reactivity with citTNC**
   Western blot of cTNC probed with serum from 8 further RA patients showing one positive patient (lane 4).

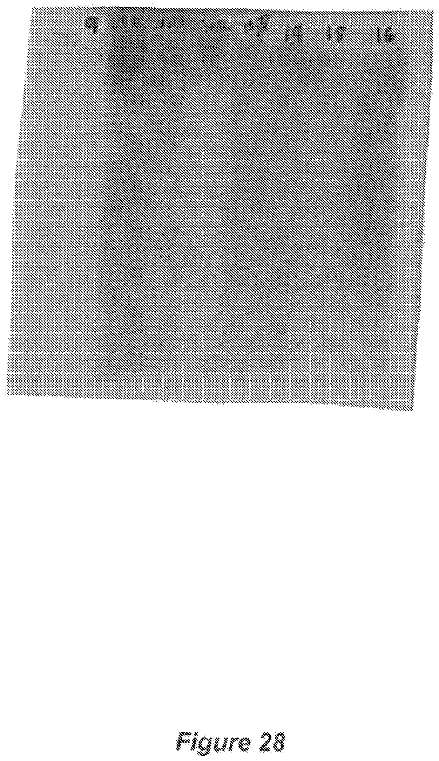

**Figure 28****. Serum from normal healthy controls exhibit no reactivity with citTNC**
   Western blot of cTNC probed with serum from 8 different controls showing no positive patients.

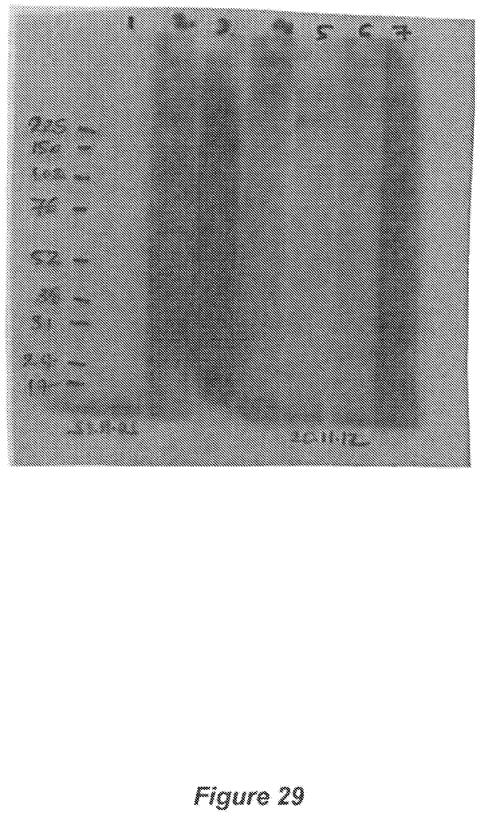

**Figure 29****. Shows RA serum against citrullinated TNC plus citrullinated FBG**
   Western blot of cTNC plus cFBG run together in the same well, probed with serum from 7 different RA patients. Patient subsets were observed that reacted with full length TNC (320kD) but not FBG (lanes 4, 5) or patients that reacted with cit FBG (27kd) but not full length TNC (lane 6).

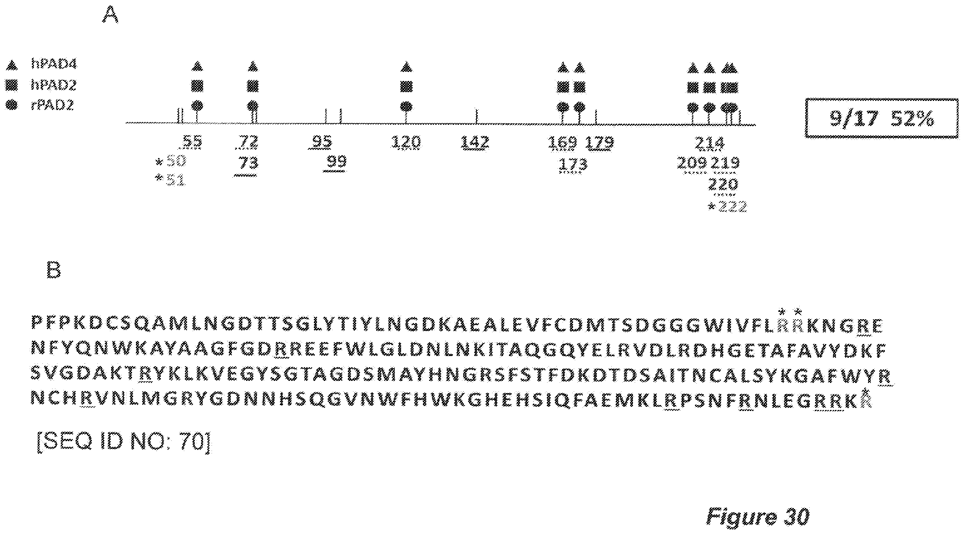

**Figure 30****. Defining the sites of citrullination by LC-MS/MS**
   (A) Arginine residues citrullinated by rPAD2 (circle), hPAD2 (rectangle) and hPAD4 (triangle) were determined by LC-MS/MS. Citrullinated sites are underlined, non-citrullinated sites are dashed-underlined, and sites that were not covered by the LC-MS/MS analysis are marked with *. (B) Sequence of FBG domain. All arginine residues are shown, arginine residues that were modified to citrulline residues are underlined. Arginines marked with * were not covered by the LC-MS/MS analysis.

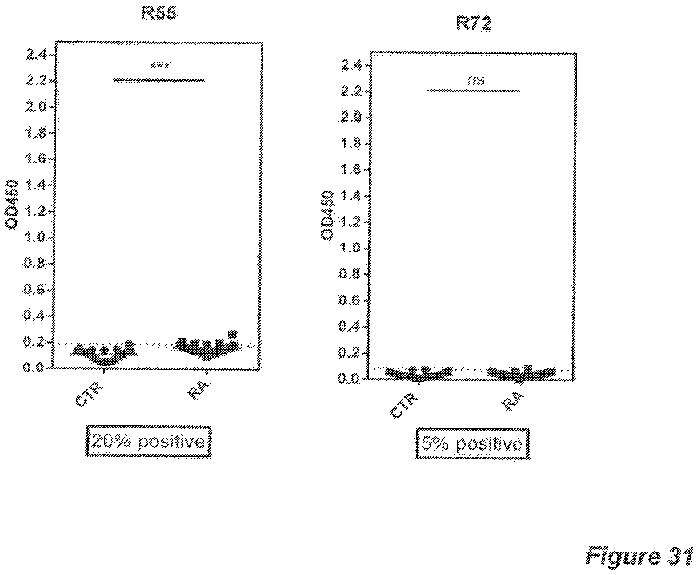

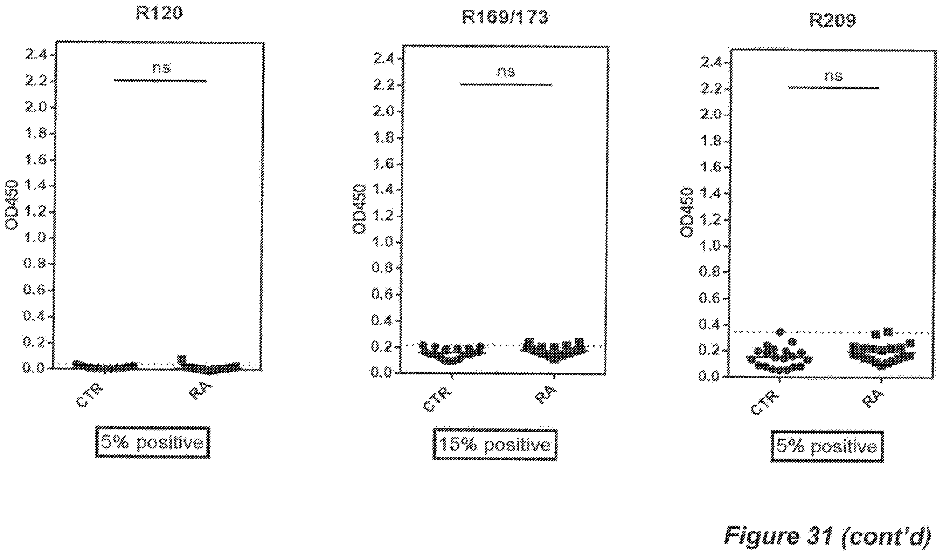

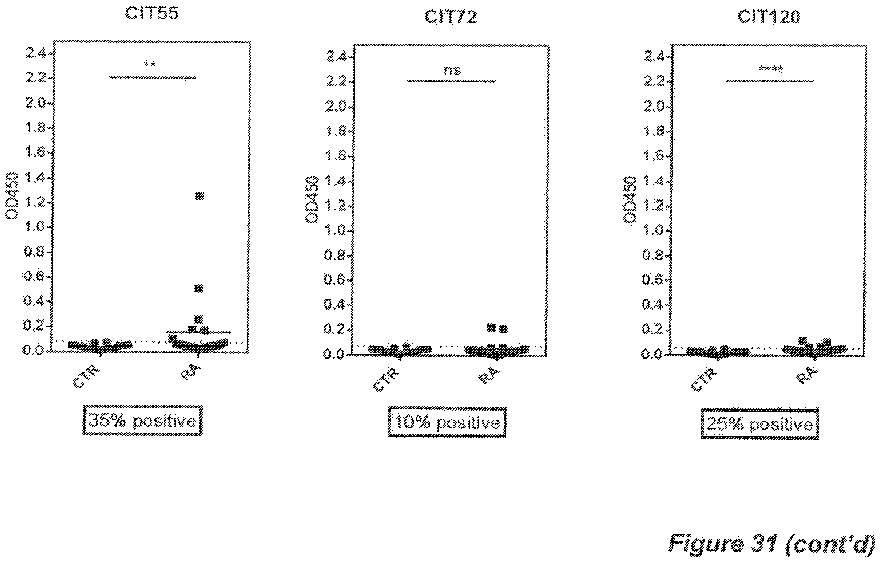

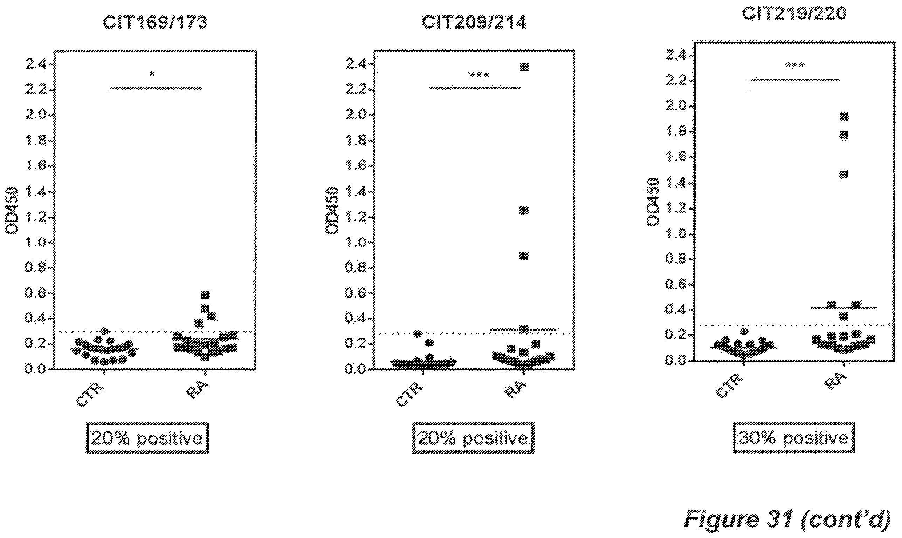

**Figure 31****. Identifying the citrullinated antibody epitope**
   (A) Peptides with sequences corresponding to the amino acid sequence of FBG were designed, with the addition of cysteine residues at the amino and carboxy termini and the exchange of arginine for citrulline residues at positions identified by LC-MS/MS. (B) IgG response to citrullinated FBG peptides and arginine containing control peptides in patients with rheumatoid arthritis (RA; n = 20) and healthy controls (n = 20). The 95th percentile of the control sera was used to determine positivity (dashed line). Mann-Whitney U test was used to calculate p values for differences between groups (n.s.=no significant difference, *=p<0.05 and **=p<0.01, ***=p<0.001, ****=p<0.001).

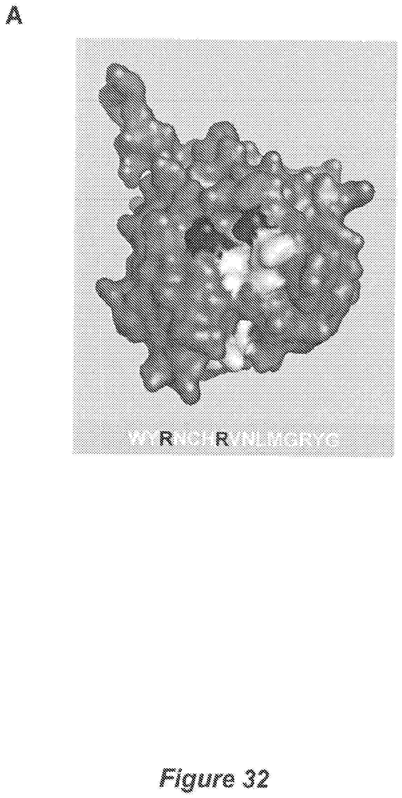

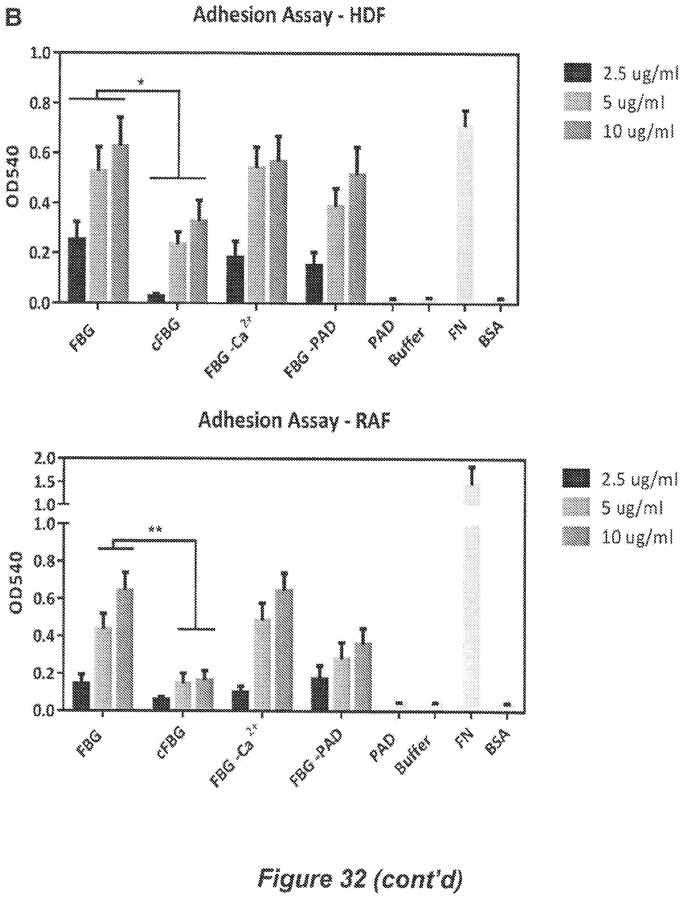

**Figure 32****. Citrullination of FBG reduces cell adhesion of HDF and RAF**
   (A) The sequence identified to bind integrin αvβ3 is shown in white (Yokoyama et al., 2000). Within this sequence two Arginines (shown in black) were identified to be citrullinated. (B) Wells of a 96 well plate were coated with different concentrations of FBG, citrullinated FBG (cFBG), FBG incubated in buffer without Calcium (FBG -Ca²⁺), or FBG incubated without PAD (FBG -PAD). As a positive control wells were coated with Fibronectin (FN) (1 ug/ml), and coating with BSA (10 mg/ml) served as negative control. Plates were incubated with human dermal fibroblasts (HDF) or synovial fibroblasts from RA patients (RAF) for 45 minutes and adhesion was measured by determining absorbance of attached cells after staining with crystal violet (0.1%). Data are shown as the mean of at least four independent experiments + s.e.m., *= p<0.05, ** = p<0.01.

### Example 1 -General methods

### Reagents

Zymosan, methylated BSA and Freund's complete adjuvant, anti-FLAG M2 antibody (mouse monoclonal antibody), blasticidin, and isotype control antibodies (Mouse IgG2a, IgG1) were from Sigma-Aldrich (Dorset, UK). Hypnorm was from VetaPharma Ltd. (Leeds, UK). The *Limulus amaebocyte* lysate assay was from Associates of Cape Cod (Liverpool, UK). Wild type human embryonic kidney (HEK293-EBNA) cells were from Invitrogen (Groningen, Netherlands). M-CSF and murine IL-1β were from PeproTech (Neuilly-Sur-Seine, France). DMEM, RPMI 1640, fetal bovine serum (FBS), penicillin/streptomycin, antibiotic-antimycotic solution PSA and β-Mercaptoethanol were from PAA Laboratories (Yeovil, UK). HEK293 cell lines stably expressing human TLR2 and TLR4/CD14/MD-2, polymyxin B, msbB LPS and the function blocking TLR2 (*Clone*: TL2.1 *Isotype:* Mouse IgG2a) and TLR4 antibodies (*Clone:* HTA125 *Isotype:* Mouse IgG2a) were from Invivogen (Calne, UK). Phenol-chloroform-purified *Escherichia coli* LPS (rough and smooth) and Pam3Cys-Ser-Lys4 (Pam3C) were from Alexis (Birmingham, UK). Murine TNF-α and IL-1 receptor antagonist (IL-1ra-IL-1F3) were from R&D Systems (Abingdon, UK). Function blocking anti-CD14 antibodies (*Isotype:* Mouse IgG1) were from Abcam (Cambridge, UK). Human and murine TNF-α, IL-6, and IL-8 ELISAs were from Pharmingen (Oxford, UK).

### Purification of full-length tenascin-C

To ensure that cytokine production was not attributed to bacterial contaminants such as LPS and LPS-associated molecules we purified recombinant full-length human tenascin-C from the conditioned medium of the mammalian cell line HEK293 transfected with his-tagged human tenascin-C in the pCEP-pu vector as described (Lange (2007)). tenascin-C was purified to homogeneity as described (Lange (2007) and determined to be free of LPS contamination using the *Limulus amaebocyte* lysate assay according to the manufacturer's instructions.

### Synthesis of recombinant proteins

Proteins corresponding to each domain of tenascin-C were synthesized (TA, EGF-L, various TNIII repeats and FBG) and purified. See Example 2.

### Measurement of LPS Contamination in Recombinant Proteins

To ascertain the levels of LPS in each recombinant protein the *Limulus amaebocyte* lysate assay was used according to the manufacturer's instructions (sensitivity ∼0.7 ± 0.5 pg LPS per mg protein). All recombinant proteins used in this study had levels of LPS that were less than 10pg/ml.

### Adenoviral Vectors and Their Propagation

Recombinant, replication-deficient adenoviral vectors encoding wild type MyD88 (AdMyD88wt), dominant-negative forms of MyD88 (AdMyD88dn) and the GFP control (AdGFP) were constructed in-house. A description of the synthesis of these viruses is in Andreakos (2004). All viruses used in this study are E1/E3 deleted, belong to the Ad5 serotype. Viruses were propagated in 293 human embryonic kidney cells, purified by ultracentrifugation through two cesium chloride gradients, and viral titers determined by plaque assay as previously described (Sacre (2007)).

### Animals

Homozygous tenascin-C deficient mice from the original stock described by Saga (1992) on a 129/sv an inbred strain of mice with a white bellied and agouti appearance background were provided by Prof. Charles French-Constant (University of Edinburgh, UK). Age matched congenic inbred wild type 129/sv mice were obtained from Charles River (Margate, UK). All tenascin-C deficient and wild type 129/sv mice were male and between 8-10 weeks of age at the time of experimentation.

Homozygous TLR2 and TLR4 deficient mice on a C57BL/6 background (an inbred strain of mice with a black coat) were obtained from B&K Universal (Hull, UK) Hoshino (1999) and Takeuchi (1999). Homozygous MyD88 deficient mice on a C57BL/6 background were provided by the Sanger Institute (Cambridge, UK). Age matched congenic inbred wild type C57B/L6 mice were obtained from Charles River (Margate, UK). For isolation of mouse embryo fibroblasts one female aged 8-10 weeks was mated with two males aged 8-10 weeks. For isolation of bone marrow derived macrophages mice were female and between 10-12 weeks of age at the time of experimentation.

All animals were fed standard rodent chow and water ad libitum, and were housed (<6 mice/cage) in sawdust-lined cages in an air-conditioned environment with 12-hour light/dark cycles. All animal procedures were approved by the institutional ethics committee.

### Statistical Methods

Mean, SD, SEM, and statistical tests were calculated using GraphPad version 3 (GraphPad Software Inc., San Diego, CA). Multiple group means were analyzed by one-way analysis of variance, followed by the Dunnett Multiple Comparisons test, where appropriate. Unpaired t-test was used for experiments involving only two groups.

### Example 2 - Synthesis of recombinant proteins

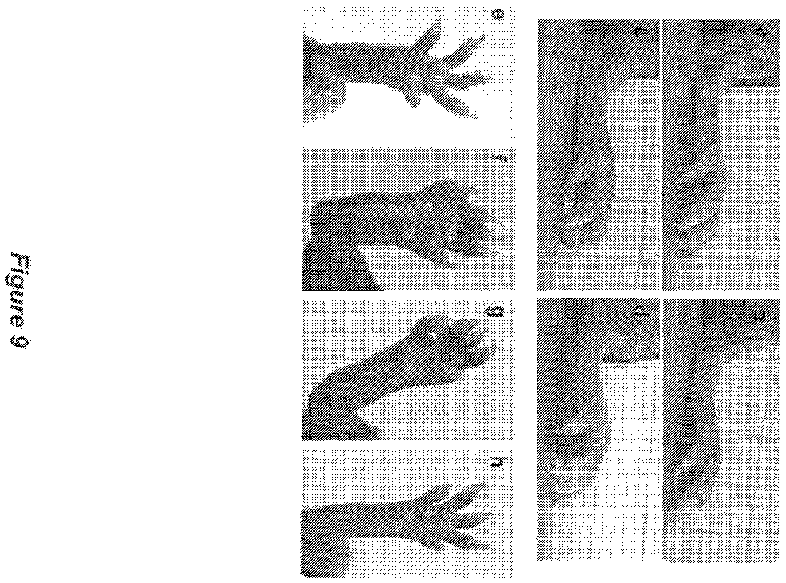

Proteins corresponding to each domain of tenascin-C were synthesized (TA, EGF-L, various TNIII repeats and FBG) and purified. The recombinant proteins synthesized are depicted in Figure 9.

### Reagents

Pfu Turbo polymerase was from Stratagene (Amsterdam, Netherlands). Easy mix 50 PCR tubes were from Molecular Bioproducts (Lutterworth, UK). RNeasy kits and Ni²⁺-NTA-agarose columns were from Qiagen (Crawley, UK). pCR Blunt vector, pCEP4 plasmid vector, human embryonic kidney (HEK293-EBNA) cells and 4-12% Bis-Tris gradient gels were from Invitrogen (Groningen, Netherlands). pET32b vector and BL21 (DE3) Rosetta cells were from Novagen (Kent, UK). HiTrap Q columns, HiTrap S columns, Sephacryl S500 HR column and heparin sepharose columns were from Amersham (Buckinghamshire, UK).

Restriction enzymes were obtained from New England BioLabs (Hitchin, UK). DMEM, fetal bovine serum (FBS) and penicillin/streptomycin were from PAA laboratories (Yeovil, UK). FuGENE6 transfection reagent was from Roche Applied Science (Basel, Switzerland).

Anti-FLAG M2 antibody (mouse monoclonal antibody), anti-FLAG M2-agarose, FLAG peptide were from Sigma-Aldrich (Dorset, UK). Anti-tetra-his antibody (mouse monoclonal antibody) was from Qiagen (Crawley, UK). Alkaline phosphatase-conjugated goat anti-(mouse IgG) IgG and Western Blue stabilized substrate for alkaline phosphatase were from Promega (Southampton, UK). Precision Protein Standards for SDS-PAGE were from BioRad (Hemel Hempstead, UK).

### Primer design

Domain boundaries were determined using alignments published in the human tenascin-C sequence (Siri (1991) accession number P24821 (Swiss-Prot)). To clone each domain we designed PCR primers where both the forward and reverse primers contained 18-21 bases corresponding to the 5' and 3' terminal sequences of the requisite coding sequence. The forward primer contained an Nde1 restriction site, followed by an N terminal his tag, immediately before the coding sequence. The final 3 bases of the Nde1 site form the ATC methionine initiation code. The reverse primer included a TTA stop codon immediately after the coding sequence, followed by a BamH1 or a Kpn1 site to allow unidirectional cloning into pET32b expression vectors.

**Table 1**

| Protein name | Forward primer Reverse primer |
|---|---|
| TA | FW: ATA***CATATG***CATCATCATCATCATCATGGGGTCCTCAAG AAAGTCATCCGG [SEQ ID NO: 1] |
| | RV: GCC***GGATCC***TTAGCCTGCTCCTGCAGTACATTG [SEQ ID NO: 2] |
| EGF-L | PCR1 |
| | FW: ACAGT***GGTACC***ACCATGGGGGCCATGGGGGCCATGACT CAGCTGTTG [SEQ ID NO: 3] |
| | RV: **CTTGTCATCGTCGTCCTTGTAGTCACC**TTCGGTAGCGAG GGCAAG [SEQ ID NO: 4] |
| | |
| | PCR2 |
| | FW: **GACTAGAAGGACGACGATGACAAG**TGCTGTCTCCAGCC TGCCAC [SEQ ID NO: 5] |
| | RV: GACAGC***GGATCC***TTAATGATGATGATGATGATGTGAGCA GTCTTCTCCGCTGTAGC [SEQ ID NO: 6] |
| TN1-5 | FW: ATA***CATATG***CATCATCATCATCATCATGAGGTGTCTCCTCC CAAAGA [SEQ ID NO: 7] |
| | RV: GCC***GGTACC***TTAAGTGGATGCCTTCACACGTGC [SEQ ID NO: 8] |
| TN1-3 | FW: ATA***CATATG***CATCATCATCATCATCATGAGGTGTCTCCTC CCAAAGA [SEQ ID NO: 9] |
| | RV: GCC***GGTACC***TTATGTTGTGAAGGTCTCTTT GGC [SEQ ID NO: 10] |
| TN3-5 | FW: ATA***CATATG***CATCATCATCATCATCATCGCTTGGATGCC CCCAGCCAGAT [SEQ ID NO: 11] |
| | RV: GCC***GGTACC***TTAAGTGGATGCCTTCACACGTGC [SEQ ID NO: 12] |
| TN5-7 | FW: ATA***CATATG***CATCATCATCATCATCATGAGTTGGACACG CCCAAGGAC [SEQ ID NO: 13] |
| | RV: GCC***GGATCC***TTATGTTGTGAACTTGGCAGTGATGGTTG [SEQ ID NO: 14] |
| TN6-8 | FW: ATA***CATATG***CATCATCATCATCATCATGCCATGGGCTCCCC AAAGGAA [SEQ ID NO: 15] |
| | RV: GCC***GGATCC***TTATGTGGTGAAGATGGTCTGGATCAT [SEQ ID NO: 16] |
| FBG | FW: ATA***CATATG***CATCATCATCATCATCATATTGGACTCCTGTAC CCCTTCC [SEQ ID NO: 17] |
| | RV: GCC***GGATCC***TTATGCCCGTTTGCGCCTGCCT TCAA [SEQ ID NO: 18] |

All primers above are written 5' to 3'. Flag sequences are in bold, His tags (CATCATCATCATCATCAT [SEQ ID NO: 19]) are underlined, and restriction enzyme cleavage sites (CATATG = Nde1 site, GGATCC = BamH1, GGTACC = Kpn1 site) are in bold italics.

### PCR

PCR amplification was carried out using 10 pmol/µl of each primer, 1µg template, 5µl DMSO, and 1.25 units Pfu Turbo polymerase in a final volume of 25µl. This was added to buffer and dNTPs in Easy mix 50 tubes. The template used for all reactions was cDNA prepared from U87MG human glioma cells using RNA isolated with RNeasy kits. The reaction was cycled 40 times with denaturing, annealing and elongation temperatures of 95°C, 55-65°C (depending on melting temperature (Tm) of primers) and 72°C respectively.

### Cloning

PCR products were ligated into pCR Blunt vectors and sequenced to ensure no errors had been introduced by PCR. Clones were selected that had no errors or silent mutations. Inserts were then ligated into pET32b using Nde1 and BamH1 restriction sites engineered into primers (TN5-7 and TN6-8). Human tenascin-C has internal BamH1 sites within the TA domain (position 494) and TNIII2 (position 2509). TA and TN1-8 were therefore cloned using the Nde1 site in the FW primer and the Kpn1 site in the cloning site of pCRBIunt. Human tenascin-C contains no internal Kpn1 sites. TN1-5, TN1-3 and TN3-5 were cloned using Nde1 and Kpn1 sites in the primers. FBG contains an internal Nde1 site (position 6439) and was therefore cloned using a two step ligation of Nde1 and BamH1 digestion, followed by Nde1 digestion. (Positions refer to sites within the full length nucleotide sequence of tenascin-C, given in figure 14)

### Bacterial growth, induction and lysis

The plasmids were transformed into BL21 (DE3) Rosetta cells, cultured in 3L of Luria-Bertani medium containing 50 µg/ml carbenicillin and induced with 1 mM isopropyl-β-D-thiogalactopyranoside. After 3 hours, the cells were harvested by centrifugation at 4,000 rpm for 20 min, washed twice with ice-cold wash buffer (50 mM Tris-HCI, pH 8.0, 100 mM NaCl, and 1 mM EDTA), and lysed with a French press. Inclusion bodies were collected by centrifugation at 12,000 rpm for 20 min at 4 °C. With the exception of TA and FBG the proteins were located entirely in the supernatant. Recombinant TA and FBG proteins were extracted from inclusion bodies with 6 M guanidine hydrochloride, 50 mM Tris-HCI, pH 8.0, and 10 mM β-mercaptoethanol at room temperature with constant stirring for 2 hours.

### Purification of bacterial proteins

The solution containing recombinant protein was applied to a Ni²⁺-NTA-agarose column and washed with 50 mM Tris-HCI, pH 8.0 containing 20 mM imidazole. The column was subsequently washed with 50 mM Tris-HCI, pH 8.0 and the protein was eluted with 50 mM Tris-HCI, pH 8.0 containing 60 mM imidazole. For TA and FBG each washing and elution buffer contained 6 M guanidine hydrochloride. Following Ni chromatography TA and FBG required no subsequent purification. TN1-3 and TN6-8 were further purified by anion exchange chromatography using a HiTrap Q column, TN1-5, TN3-5 and TN5-7 by cation exchange chromatography using a HiTrap S column, and TN1-8 using a HiTrap S column followed by gel filtration using a Sephacryl S500 HR column.

### Refolding of insoluble proteins

TA and FBG were refolded by diluting to 20 µg/ml with 50 mM Tris-HCI, pH 8.0 containing 6 M guanidine hydrochloride and then treating with 20 mM cystamine with stirring for 16 hours at 4 °C. The solution was then dialyzed twice against 15 volumes of 50 mM Tris-HCI, pH 8.0 containing 150 mM NaCl, 10 mM CaCl₂, 5 mM β-mercaptoethanol, and 1 mM 2-hydroxyethyl disulfide for 24 hours at 4 °C, twice against 20 mM Tris-HCI, pH 8.0 for 8 hours at 4 °C and then centrifuged at 12,000 rpm for 30 min at 4 °C. Refolding was assessed by size shifts using SDS PAGE under reducing and non reducing conditions. Protein activity was confirmed by TA domain polymerization and FBG binding to heparin sepharose columns.

### Synthesis of EGF-L domain using mammalian cells

Initial attempts to express and purify the EGF-L repeats region using an *E.coli* expression system were unsuccessful. This is most likely to be attributable to difficulty in achieving protein folding due to a total of 91 cysteines in this region. Therefore, the EGF-like domains of TN-C were expressed using HEK293 cells.

Two PCR reactions were carried out. The first PCR product consisted of a restriction enzyme *Kpn*I site, a Kozak sequence followed by the TN-C signal sequence. The second PCR product consisted of a FLAG peptide, the EGF-like domain sequence, followed by a histidine tag and a BamH1 restriction enzyme sequence.

The two PCR products were ligated together as described by Ho (1989). PCR reactions were carried out as described above. The entire construct was cloned into the PCR blunt vector and sequenced. It was then subcloned into the pCEP4 vector. The DNA was transfected into HEK293 cells using Fugene and cells were selected for hygromycin resistance (200 µg/ml) in Dulbecco's modified Eagle's medium (DMEM) containing 10% (v/v) fetal calf serum, penicillin (100 units/ml) and streptomycin (100 units/ml). 2 litres conditioned medium (collected after cells have been cultured in medium) from stably transfected cells was collected and pooled. The pooled conditioned medium (2 litres) was centrifuged at 3000 rpm to separate cell debris from the medium.

The medium was then applied to an anti-FLAG column. Material was collected in 50 ml fractions for the flow-through. The column was washed with 10 column volumes of 1M NaCl, 50 mM Tris-HCI, pH 7.5 and then washed with 10 column volumes of 60% isopropanol to ensure removal of LPS. The column was then washed with 50 mM Tris-HCl buffer, pH 7.5 and finally the protein was eluted using 200 µg/ml FLAG peptide in 50 mM Tris-HCl buffer, pH 7.5.

### Analysis of protein purity

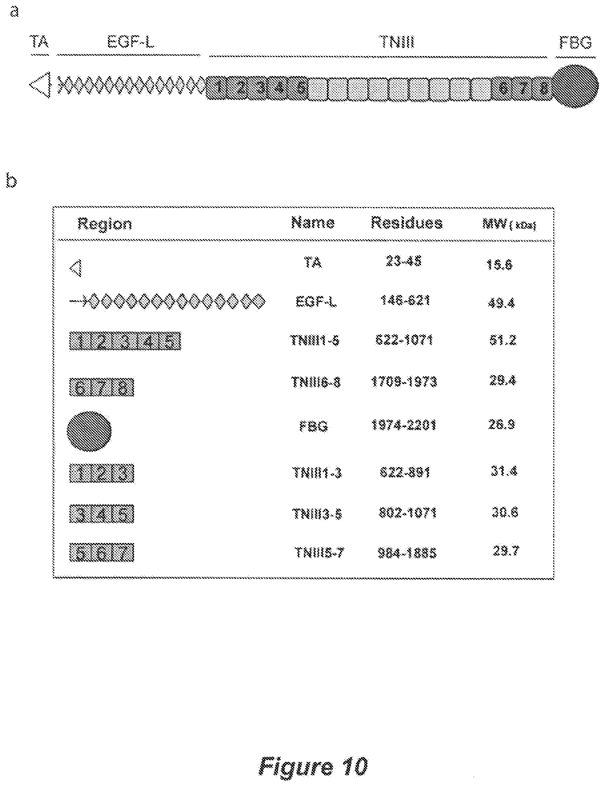

Each protein was dialysed against 1000 volumes of 150 mM NaCl and 50mM Tris pH 7.5. Protein purity was analyzed by SDSPAGE under reducing conditions. To do this 1 µg of each purified recombinant protein was run on a 4-12% Bis-Tris gradient gel and the gel was subsequently silver stained to demonstrate a single band (figure 10). Western blotting analyses were also carried out. Proteins separated by SDS-PAGE were electrotransferred to polyvinylidene difluoride membranes. The membranes were blocked with 5% BSA in Tris-buffered saline and then incubated with primary antibodies recognizing FLAG M2 (1:2000 dilution)(EGF-L) or tetra-his antibodies (1:2000)(all other proteins). The blot was then incubated with secondary antibody conjugated to alkaline phosphatase and the protein bands visualized using Western Blue stabilized substrate whereby the gels show a single specific band recognised by each antibody at the expected Mw (not shown)

### Example 3 - Animal models

### Zymosan-induced arthritis

Zymosan-induced arthritis (ZIA) was induced in tenascin-C deficient and wild type mice by injection of zymosan (*Saccharomyces cerevisiae*), as described in Keystone (1977). Zymosan was prepared by dissolving 15 mg of zymosan in 1ml of sterile PBS. The solution was boiled twice and sonicated. Mice were anesthetized by intraperitoneal injection of 150 µl of Hypnorm diluted 1:10 in sterile water, then injected with zymosan (10 µl) into the right footpad (d=0).

Control mice received an injection of 10 µl PBS alone or were not injected. For macroscopic assessment of arthritis, the thickness of each hind paw was measured daily with microcalipers (Kroeplin, Schluchlem, Germany) and the diameter expressed as an average for each inflamed hind paw per mouse.

Following completion of the experiment (day=4), mice were euthanized and hind paws fixed in 10% (v/v) buffered formalin, decalcified with 10% EDTA and processed to paraffin.

### Antigen-induced arthritis

Antigen-induced arthritis (AIA) was induced in tenascin-C-deficient and wild-type mice as described previously by Brackertz (1977). Briefly, at day 0 mice were anesthetized by intraperitoneal injection of 150 µl of Hypnorm diluted 1:10 in sterile water, then immunized with 200 µg of methylated BSA. mBSA was emulsified in 0.2 ml of Freund's complete adjuvant and injected intra-dermally at the base of the tail.

At day 7, arthritis was induced by intra-articular injection of mBSA (100 µg in 10 µl of sterile PBS) into the right knee joint using a sterile 33-gauge microcannula. Control mice received an injection of 10 I PBS alone or were not injected.

On day 14, mice were euthanized, the knee joints were excised and fixed in 10% (volume/volume) buffered formalin, decalcified, with 10% EDTA and processed to paraffin.

### Injection of FBG

Wild type mice were anesthetized by intraperitoneal injection of 150 µl of Hypnorm diluted 1:10 in sterile water and then injected with 100 ng, 1 or 3 µg FBG in 10 µl of sterile PBS into the right knee joint using a sterile 33-gauge microcannula. Control mice received an injection of 10 µl PBS alone or were not injected.

On days 3 and 7, mice were euthanized, the knee joints were excised and fixed in 10% (volume/volume) buffered formalin, decalcified, with 10% EDTA and processed to paraffin.

### Histology of knee joints

Coronal tissue sections (4 µm) were cut at 7 depths throughout the joint; 80 µm apart and stained with hematoxylin and eosin or Safranin-O to assess joint pathology. Histopathologic changes were scored using the following parameters as described in Van Lent (2006).

Inflammation (the influx of inflammatory cells into synovium (infiltrate) and the joint cavity (exudates), was graded using an arbitrary scale from 0 (no inflammation) to 3 (severe inflammation). Chondrocyte death was determined as the percentage of cartilage area containing empty lacunae in relation to the total area. Cartilage surface erosion was determined as the amount of cartilage lost in relation to the total cartilage area. Bone destruction was determined in 10 different areas of the total knee joint section. Destruction was graded on a scale of 0 (no damage) to 3 (complete loss of bone structure). Histological analysis was performed by an investigator who was blinded to the experimental groups. The mean score for each animal in an experimental group was calculated by averaging the histopathologic scores in at least 5 section depths per joint.

### Results

### Zymosan induced joint inflammation is not sustained in tenascin-C deficient mice

Zymosan injection into the footpad was used to induce acute synovitis in mice. Wild type mice exhibited rapid paw swelling reaching maximal paw diameter by 24 hours (2.56mm, an increase of 62% of the starting paw diameter). This was maintained for a further 24 hours. After 2 days paw diameter decreased but paws remained swollen by 4 days (2.08mm, an increase of 32%) (figure 1a). tenascin-C deficient mice exhibited a similar degree of paw swelling to wild type mice 24 hours post injection (2.41mm, an increase of 57% of starting paw diameter). However, swelling in the tenascin-C null mice subsided faster than in the wild type mice; paw diameter was significantly reduced at 2 days and had declined to 1.7mm (an increase of only 11%) by 4 days (figure 1a). By day 4 post injection the paws of wild type mice were still visibly swollen and red, whereas the paws of tenascin-C null mice were not visibly swollen or red and resembled non-injected paws (Figure 9).

This difference was reflected histologically at 4 days. The synovia of wild type mice were significantly inflamed and exhibited cellular infiltration and cartilage proteoglycan loss was observed (Figure 1b, c). In contrast, the synovium of tenascin-C deficient mice exhibited no synovitis, cellular infiltrate or cartilage proteoglycan loss (figure 1d, e) and resembled the joints of sham injected and non injected mice (not shown). Quantification of joint inflammation revealed whilst there was little exudate (cellular mass in the joint cavity) in either wild type or tenascin-C null mice, levels of infiltrate (cellular mass in the synovial layer) were significantly reduced in tenascin-C null mice (figure 1f). No erosion of cartilage or bone occurred in mice of either genotype (not shown), however a low level of chondrocyte death occurred in wild type mice, that was not observed in tenascin-C null mice (figure 1g). Thus tenascin-C expression appears to promote the maintenance of acute inflammation.

### Tenascin-C null mice are protected from persistent inflammation and structural damage during antigen induced arthritis

To determine whether tenascin-C also contributes to more destructive inflammatory joint disease, erosive arthritis was induced by intra-articular injection of mBSA into the knee joint following immunization with mBSA. This model involves both cellular and humoral immune responses and induces pathological changes similar to human RA (Brackertz (1977)). Injection of mBSA induced a similar inflammatory response in both tenascin-C null and wild type mice. Cell infiltration and synovial thickening is apparent by 24 hours in mice of both genotypes (figure 2c-f, h,i) compared to sham injected (figure 2a, b, g) or non injected (not shown) mice.

However, this does not persist in tenascin-C null mice as it does the wild type mice. By 3 days post injection wild type mice exhibit increased inflammation of the meniscus and capsule, synovial hyperplasia, cells and fibrin deposits in the joint space, pannus formation and localized cartilage proteoglycan loss (figure 3a, b, f). In contrast, by 3 days in tenascin-C null mice inflammation is limited to the capsule, synovial inflammation has subsided and there are no fibrin/cell aggregates present in the joint space, no pannus formation and no cartilage proteoglycan loss (figure 3c, d, e).

By 7 days wild type mice exhibited persistent inflammatory cell infiltration and joint space exudate, extensive synovitis and pannus formation and destruction of articular cartilage and bone erosion (figure 4a, b). Sham injected knees and knees from mice that had undergone no injection were healthy and exhibited no inflammation or joint destruction (not shown). tenascin-C deficient mice also had healthy joints that exhibited only mild inflammatory cell infiltration, with no joint space exudate, synovitis, pannus formation, destruction of articular cartilage or bone erosion (figure 4c, d). Joints from tenascin-C deficient mice that had been sham injected and or that had undergone no injection were also healthy (not shown).

These histological data are reflected upon scoring of joint disease as described in materials and methods. Levels of cellular infiltrate and exudate observed in both wild type mice and tenascin-C null mice 24 hours post injection were not significantly different. However, whilst cellular mass continued to increase in wild type mice over time, this response was attenuated in tenascin-C null mice and cell numbers in the joint decreased over time (figure 4e). Increasingly high levels of chondrocyte death occurred in the cartilage of wild type mice over time, but no significant death was observed in tenascin-C null mice (figure 4f). No cartilage surface erosion and bone erosion was evident in wild type mice at 24 hours or 3 days (not shown) but significant tissue destruction had occurred by 7 days. In contrast tenascin-C null mice exhibited no tissue destruction at 24 hours, 3 days (not shown) or 7 days (figure 4f). These data indicate that whilst the initiation of joint inflammation (cell influx into the synovium and joint space) is unaffected in tenascin-C null mice, unlike in wild type mice disease does not progress to tissue destruction and cell death. These results demonstrate that expression of tenascin-C is required for persistent synovial inflammation and joint destruction in this model.

### Example 4 - Cell culture

### Patient Specimens

Human monocytes were isolated from peripheral blood (London Blood Bank) and macrophages were derived from monocytes after differentiation for 4 days with 100 ng/ml of M-CSF as previously described (Foxwell (1998)).

RA membrane cells (representing a mixed population of all synovial cell types) were isolated from synovial membranes obtained from patients undergoing joint replacement surgery as previously described (Brennan(1989)). RA synovial fibroblasts were isolated from the mixed population of RA membrane cells as previously described (Brennan(1989)). The study was approved by the local Trust ethics committee (Riverside NHS Research Committee), and waste tissue (synovium after joint replacement surgery) was obtained only after receiving signed informed consent from the patient and anonymyzing the tissue to protect patient identity.

Immediately after isolation, RA membrane cells and macrophages were cultured at 1x10⁵ cells/well in RPMI 1640 containing 10% (v/v) FBS and 100 U/ml (Units/ml) penicillin/streptomycin in 96-well tissue culture plates for 24 hours before stimulation. Synovial fibroblasts (used only at either passage number 2 or 3) were cultured at 1x10⁴ cells/well in DMEM containing 10% (v/v) FBS and 100 U/ml penicillin/streptomycin in 96-well tissue culture plates for 24 hours before stimulation.

### Mouse embryonic fibroblasts (MEFs) and bone marrow derived macrophages (BMDMs)

MEFs express high levels of mRNA of all 9 murine TLRs and are specifically and highly responsive to TLR ligand activation. MEFs from mice with targeted deletions of TLR2, TLR4 and MyD88 demonstrate profound defects in their IL-6 response to specific ligands (Kurt-Jones (2004)). MEFs were isolated from d13 embryos harvested from age-matched, pregnant female wild type, TLR2, TLR4 and null mice (as described in Todaro (1963)). Fibroblasts were cultured at 2x10⁴ cells/well in DMEM containing 10% (v/v) FBS and 100 U/ml penicillin/streptomycin in 96-well tissue culture plates for 24 hours before stimulation.

BMDMs were derived by aspirating the femurs of age matched female wild type, TLR2 and TLR4 null mice as described in Butler (1999)) and culturing the cells for 7 days in DMEM, 20% (v/v) FBS, 10ml/L (v/v) antibiotic-antimycotic solution PSA, 50µM β-Mercaptoethanol and 10ng/ml M-CSF. Macrophages were then cultured at 1x10⁵ cells/well in DMEM, 20% (v/v) FBS, 10ml/L (v/v) antibiotic-antimycotic solution PSA, 50µM β-Mercaptoethanol in 96-well tissue culture plates for 24 hours before stimulation.

### HEK293 cell lines

HEK293 cell lines expressing TLR2 and TLR4/CD14/MD-2 were cultured at 1x10⁴ cells/well in DMEM containing 10% (v/v) FBS and 10 µg/ml blasticidin in 96-well tissue culture plates for 24 hours before stimulation.

### Cell stimulation and assessment of cytokine synthesis

Cells were incubated for 24 hours at 37°C with the indicated doses of tenascin-C and recombinant tenascin-C fragments (1.0 µM - 1.0 nM). Cells were also stimulated where indicated with LPS (1 ng/ml for human macrophages, 10ng/ml for human fibroblasts, RA membrane cells and HEKs, 100ng/ml for MEFS and BMDMs and 10ng/ml for HEKS), PAM3 (10 ng/ml for human macrophages, human fibroblasts, and HEKs, 100ng/ml for MEFs and BMDMs), murine IL-1 (5ng/ml for MEFS) and murine TNF-α (100ng/ml for MEFS). Unless specifically stated otherwise rough LPS was used for *in vitro* studies.

For adenoviral gene transfer experiments, human RA synovial fibroblasts were incubated with adenoviral vectors at a multiplicity of infection of 100, washed after 2 hours, cultured in complete medium for 24 hours, then stimulated for 24 hours, after which time supernatants were collected.

Where stated, cells were pre-incubated with 10µg/ml anti-CD14 antibody, 10µg/ml IL1 receptor antagonist, 10µg/ml anti-TLR2 antibody, 25µg/ml anti-TLR4 antibody, 10 or 25µg/ml isotype control antibody, 25µg/ml polymyxin B, or 1µg/ml msbB LPS, for 30 minutes at 37°C before stimulation. Where stated, recombinant tenascin-C and FBG, and LPS were boiled for 15 minutes before addition to cells

In all cases, viability of the cells was not significantly affected throughout the experimental time period when examined by the MTT cell viability assay (Sigma, Poole, UK).

Supernatants were subsequently examined for the presence of the cytokines TNF-α, IL-6, and IL-8 by enzyme-linked immunosorbent assay (ELISA) according to the manufacturer's instructions. Absorbance was read on a spectrophotometric ELISA plate reader (Labsystems Multiscan Biochromic, Vantaa, Finland) and analyzed using the Ascent software program (Thermo Labsystems, Altrincham, UK).

### Results

### Tenascin-C induces TNF-α, IL-6 and IL-8 synthesis in primary human RA synovial fibroblasts and macrophages

We next investigated whether tenascin-C might activate the innate immune response. tenascin-C was used to stimulate primary human macrophages and RA synovial fibroblasts and the production of the pro-inflammatory cytokines TNF-α, IL-6 and IL-8 examined. The bacterial cell wall component LPS was used as a positive control. tenascin-C induced a cell type specific cytokine profile which was significantly different from LPS. It dose dependently stimulated the production of TNF-α, IL-6 and IL-8 in human macrophages (figure 5a). However, tenascin-C only induced IL-6 synthesis in synovial fibroblasts, whereas LPS induced both IL-6 and IL-8 (figure 5b). Neither LPS nor tenascin-C induced TNF-α synthesis in fibroblasts (data not shown). tenascin-C stimulation of IL-6 (Figure 5c), IL-8 and TNF-α by human macrophages and IL-6 by synovial fibroblasts (not shown) was heat sensitive and unaffected by the LPS inhibitor, polymyxin B. Together these results provide strong evidence that cytokine induction by tenascin-C is not due to LPS contamination.

### The fibrinogen-like globe (FBG) mediates tenascin-C activation of cells.

Tenascin-C is a large hexameric molecule, each domain of which binds to different cell surface receptors (reviewed in Orend (2005)). Understanding the mechanism of action of tenascin-C will require identification of which domain(s) are critical for promoting cytokine production. We synthesized recombinant proteins comprising different domains of the molecule (figure 10). Each domain was made in *E.coli*, purified (figure 11), and found to contain <10 pg/ml LPS by subjecting neat protein to the *Limulus amaebocyte* lysate assay. Only one domain of tenascin-C was active. The fibrinogen-like globe (FBG) stimulated TNF-α synthesis in human macrophages (Figure 6a), IL-6 and IL-8 synthesis in human macrophages (not shown) and IL-6 in RA synovial fibroblasts (not shown) to an equal extent to full-length tenascin-C. Like full-length tenascin-C, FBG did not induce IL-8 synthesis in RA synovial fibroblasts where LPS did (data not shown). FBG induced cytokine synthesis was also heat sensitive and unaffected by polymyxin B (data not shown).

### The FBG domain of tenascin-C induces cytokine production in human RA synovium and joint inflammation in mice.

We investigated whether FBG could promote expression of inflammatory cytokines in synovial membranes from RA patients. This tissue model of RA (comprising a mixed population of all synovial cell types) spontaneously produces high levels of IL-6, IL-8 and TNF-α (Brennan (1989)) (figure 6b). FBG further enhanced synthesis of all these cytokines (figure 6b). To determine whether FBG could induce inflammation *in vivo*, wild type mice were injected intra-articularly with FBG. We observed a transient and dose dependent stimulation of joint inflammation. No inflammation or proteoglycan loss occurred in non-injected mice or in mice injected with PBS (figure 6c-e) or 100ng FBG (data not shown). In mice injected with 1 µg FBG inflammatory cell infiltration (figure 6f), mild synovitis, pannus formation (figure 6g) and proteoglycan loss (figure 6h) was observed. A similar response was seen in mice injected with 3 µg FBG (data not shown). Upon histological quantification, high levels of cellular infiltrate and exudate and chondrocyte death were observed in mice injected with FBG, together with a modest amount of cartilage surface erosion and bone damage (figure 6i).

### FBG mediated cytokine synthesis is dependent on Myd88

Many DAMPs, including fibrinogen (Smiley (2001)), have been shown to stimulate the innate immune response by activation of TLRs. Therefore, we investigated whether TLRs might also mediate tenascin-C induced cytokine production. Myeloid differentiation factor 88 (MyD88) is required for signalling by all TLRs, except TLR3 (O'Neill (2008)). Infection of synovial fibroblasts with adenovirus expressing dominant negative MyD88, but not GFP control virus, abolished FBG induction of IL-6 (figure 7a). These data suggest that FBG induced inflammation is dependent on functional MyD88. This effect of FBG did not appear to be mediated by IL-1 as addition of IL-1 receptor antagonist did not inhibit induction of cytokines (data not shown). To confirm that FBG action is MyD88 dependent we demonstrated that FBG does not stimulate cytokine synthesis in embryonic fibroblasts isolated from mice with targeted deletions in the MyD88 gene. The TLR2 ligand PAM3, TLR4 ligand LPS and IL-1 all signal via MyD88. Stimulation with these was also abolished in MEFs from deficient mice. However, TNF-α, which does not signal via MyD88, was unaffected (figure 7b). Re-transfection of wild type MyD88 restored the responsiveness of these cells to FBG, PAM3, LPS and IL-1 (data not shown).

### FBG signals via TLR4

TLRs exhibit specificity for endogenous ligands; proteins are recognised by one or both of TLR2 and 4 (reviewed in O'Neill (2008)). Neutralising antibodies to TLR4 inhibited both FBG and LPS induced IL-6, IL-8 and TNF-α synthesis in human macrophages and IL-6 synthesis in RA synovial fibroblasts but had no effect on the function of the TLR2 ligand, PAM3. Antibodies to TLR2 inhibited PAM3 mediated cytokine synthesis but had no effect on LPS or FBG induced cytokine synthesis. Isotype matched controls had no effect on cytokine synthesis induced by any ligand (TNF-α synthesis by human macrophages is shown in figure 8a). To confirm that FBG action is TLR4 dependent we demonstrated that FBG does not stimulate cytokine synthesis in embryonic fibroblasts or macrophages isolated from mice with targeted deletions in the TLR4 gene. FBG mediated cytokine synthesis was unaffected in embryonic fibroblasts or macrophages isolated from mice with targeted deletions in the TLR2 gene. Cells isolated from TLR2 deficient mice were unresponsive to PAM3 but responsive to LPS and IL-1. Cells isolated from TLR4 deficient mice were unresponsive to LPS but did respond to PAM3 and IL-1 (figure 8b, c). In addition, expression of TLR4 was required for the arthritogenic action of FBG in vivo; FBG was able to induce joint inflammation in TLR2 null mice but not in TLR4 null mice (figure 12).

### Different co-receptor requirements for FBG and LPS

LPS signalling via TLR4 is mediated by a receptor complex including the soluble protein MD-2 and GPI-linked cell surface or soluble CD14 (reviewed in Fitzgerald (2004)). We next examined whether CD14 and MD-2 are required for FBG activation of TLR4. As a positive control here we examined the activity of smooth glycosylated LPS which requires both MD-2 and CD14 (Jiang (2005)). LPS mediated IL-6, IL-8 and TNF-α synthesis by human macrophages and IL-6 synthesis by RA synovial fibroblasts was inhibited by anti-CD14 antibodies and an antagonistic LPS derived from the msbB mutant E.coli which competes for LPS binding to MD-2 (Coats(2007)). Conversely, both PAM3, which does not require these co-receptors for activation of TLR2, and FBG-mediated cytokine synthesis was unaffected by anti CD14 antibodies or msbB mutant LPS (figure 8d shows TNF-α synthesis by human macrophages). These data suggest **t**hat neither CD14 nor MD-2 is required for FBG mediated cytokine synthesis. Therefore, whilst LPS and FBG both signal via activation of TLR4, they may have different co-receptor requirements.

### Example 5 - Inhibition of Tenascin-C action and synthesis in human tissue

This example studies the effect of (1) prevention of the pro-inflammatory action of tenascin-C and (2) inhibition of tenascin-C expression in the human RA synovium.

### Methods

### Peptide synthesis

Nine overlapping peptides comprising the entire FBG domain (table 2) were synthesized by Biogenes, Germany. Peptides were cleaved at room temperature (cleavage mixture: 90% trifluoroacetate, 5% thioanisol, 3% ethanedithiol, 2% anisole), purified by reverse phase high performance liquid chromatography, and characterized by MALDI TOF mass spectral analysis. The purity of the peptides was >85% as determined high performance liquid chromatography.

The facility was unable to synthesize peptide 7, presumably due to the formation of secondary structure that prevented elongation of the peptide chain (as previously reported (LaFleur (1997)).

**Table 2. Overlapping peptides that span the entire FBG domain of human tenascin-C**

| **Peptide #** | **Amino acid sequence** |
|---|---|
| 1 | TIGLLYPFPKDCSQAMLNGDTTSGLYTIYL [SEQ ID NO: 20] |
| 2 | YTIYLNGDKAEALEVFCDMTSDGGGWIVFL [SEQ ID NO: 21] |
| 3 | WIVFLRRKNGRENFYQNWKAYAAGFGDRRE [SEQ ID NO: 22] |
| 4 | GDRREEFWLGLDNLNKITAQGQYELRVD [SEQ ID NO: 23] |
| 5 | ELRVDLRDHGETAFAVYDKFSVGDAKTRYK [SEQ ID NO: 24] |
| 6 | KTRYKLKVEGYSGTAGDSMAYHNGRSFST [SEQ ID NO: 25] |
| 7 | RSFSTFDKDTDSAITNCALSYKGAFWYRN [SEQ ID NO: 26] |
| 8 | WYRNCHRVNLMGRYGDNNHSQGVNWFHWKG [SEQ ID NO: 27] |
| 9 | FHWKGHEHSIQFAEMKLRPSNFRNLEGRRKRA [SEQ ID NO: 28] |

### Patient specimens and cell culture

RA membrane cells (representing a mixed population of all synovial cell types) were isolated from synovial membranes obtained from patients undergoing joint replacement surgery (Brennan (1989)). Synovial membrane tissue was digested in RPMI 1640 (GIBCO) containing 5% fetal calf serum (FCS) (GIBCO), 5 mg/ml collagenase type IV (Sigma) and 0 15 mg/ml DNAse type I (Sigma) and incubated at 37°C for 2 h.

After incubation the tissue was pipetted through a nylon mesh into a sterile beaker. The cells were then washed three times in complete medium (RPMI 1640 supplemented with 10% FCS). RA synovial fibroblasts were isolated from the mixed population of RA membrane cells by selection in DMEM (Bio-Whittaker) supplemented with 10% FBS, 1 µM glutamine, 100 U/ml penicillin, and streptomycin. Human monocytes were isolated from peripheral blood (London Blood Bank) and macrophages were derived from monocytes after differentiation for 4 days with 100 ng/ml of M-CSF.

The study was approved by the local Trust ethics committee, and waste tissue (synovium after joint replacement surgery) was obtained only after receiving signed informed consent from the patient and anonymising the tissue to protect patient identity.

### Cell stimulation and assessment of cytokine synthesis

Immediately after isolation, RA membrane cells were cultured at 1x10⁵ cells/well in RPMI 1640 containing 10% (v/v) FBS and 100 U/ml penicillin/streptomycin in 96-well tissue culture plates. Cells were incubated for 24h at 37°C with no addition, buffer control (PBS, 1% BSA, 0.01% NaN₃), or with 25 µm, 100 µM or 250 µM of each FBG spanning peptide.

Synovial fibroblasts (used only at either passage number 2 or 3) were seeded at a concentration of 5 × 10⁴ cells in a 3.5-cm dish. siRNA was transfected at a final concentration of 10 nM using Lipofectamine 2000 (Invitrogen) for 4 h in serum-free OptiMEM I. Two different siRNAs against human tenascin-C were used (s7069 and s229491) (Applied Biosystems).

siRNA sequences of s7069 are: (sense 5' CGCGAGAACUUCUACCAAAtt 3' [SEQ ID NO: 29], antisense 5' UUUGGUAGAAGUUCUCGCGtc 3' [SEQ ID NO: 30]) and of s229491 are (5' GGAAUAUGAAUAAAGAAGAtt 3' [SEQ ID NO: 31], antisense 5' UCUUCUUUAUUCAUAUUCCgg 3' [SEQ ID NO: 32]). siRNA against luciferase (Dharmacon) was transfected as a non-targeting control.

Four hours after transfection, medium was changed with pre-equilibrated Dulbecco's modified Eagle's medium containing 10% FBS (v/v) and cells were incubated for a further 48h and 72h. Cells were then stimulated with 10 ng/ml LPS for 24h at 37°C. Tenascin-C mRNA and protein levels were quantitated by PCR and western blotting respectively. Total RNA was extracted from cells using a QiaAmp RNA Blood mini kit (Qiagen, Germany). cDNA was synthesised from equivalent amounts of total RNA using SuperScript® III Reverse Transcriptase (Invitrogen) and 18-mer oligo dTs (Eurofins MWG Operon).

Gene expression was analysed by delta-delta ct methods based on quantitative real-time PCR with TaqMan primer set human tenascin-C (Hs01115663-m1) and human ribosomal protein endogenous control (RPLPO) (4310879E) (Applied Biosystems) in a Corbett Rotor-gene 6000 machine (Corbett Research Ltd). Tenascin-C protein was detected in cell supernatants and cell lysates by by SDS PAGE and western blotting using antibody MAB1908 (Millipore).

Macrophages were cultured at 1x10⁵ cells/well in RPMI 1640 containing 5% (v/v) FBS and 100 U/ml penicillin/streptomycin in 96-well tissue culture plates for 24h before stimulation. Cells were incubated for 24h at 37°C with no addition, 1.0 µM FBG, 1 ng/ml LPS or 1 or 20 µM FBG peptide. Where stated, cells were pre-incubated with 20 µM FBG peptides for 15 min.

The viability of the cells was not significantly affected throughout the experimental time period when examined by the MTT cell viability assay (Sigma, Poole, UK). Supernatants were examined for the presence of the cytokines TNF-α, IL-6, and IL-8 by enzyme-linked immunosorbent assay (ELISA) according to the manufacturer's instructions (R&D systems). Absorbance was read on a spectrophotometric ELISA plate reader (Labsystems Multiscan Biochromic, Vantaa, Finland) and analyzed using the Ascent software program (Thermo Labsystems, Altrincham, UK).

### Statistical Methods

Mean, SD and SEM were calculated using GraphPad (GraphPad Software Inc., San Diego, CA).

### Results

### Blockade of cytokine synthesis in RA membrane cultures by specific FBG peptides

The approach of peptide inhibition has been used successfully to pinpoint the αvβ3 integrin binding site in the FBG domain of tenascin-C and to prevent cell adhesion in response to this domain of tenascin-C (Lafleur (1997) and Yokoyama (2000)).

We synthesized a series of 8 overlapping peptides of ∼30 amino acids that span the entire sequence of FBG (Table 2). Peptides were tested for the ability to block spontaneous cytokine synthesis in RA synovial membrane cultures. TNF and IL8 synthesis was inhibited by peptides 3 and 8, but not by any other peptide (TNF shown in figure 15). Peptides 3 and 8 dose dependently inhibited cytokine synthesis with the highest concentrations achieving 95% and 56% inhibition respectively (figure 16). Whilst peptide 5 had no effect on TNF synthesis, it dose dependently blocked IL8 synthesis in RA membrane cells with a maximal inhibition of 81% (figure 16).

To map the active domain within FBG responsible for inducing cytokine production we stimulated primary human macrophages with each FBG peptide. Peptides 1, 5 and 6 all induced cytokine synthesis in a dose dependent manner (figure 17).

To determine if any peptide could block FBG induced cytokine synthesis in human macrophages, cells were pre-incubated with each FBG peptide before stimulation with either whole FBG or LPS. Peptide 5 specifically blocked FBG mediated cytokine synthesis, whilst peptide 8 blocked cytokine synthesis in response to both LPS and FBG (figure 18).

Peptide 8 therefore non-specifically blocks cytokine production induced by any stimuli. This domain is the integrin binding domain of FBG that mediates cell adhesion and thus may be acting to prevent cell attachment to tissue culture plates. Peptide 5 specifically blocks FBG-induced cytokine synthesis suggesting that targeting this domain may be useful in preventing tenascin-C induced inflammation.

### Silencing tenascin-C gene expression inhibits cytokine synthesis in RA synovial fibroblasts

Examination of the effect of inhibiting tenascin-C expression in the human RA synovium has identified synovial fibroblasts as the major source of tenascin-C in RA (figure 1C) (in Goh 2010).

siRNA mediated knockdown of tenascin-C expression in these cells has been shown with a maximal efficiency between 94-96% (figure 19). In cells transfected with tenascin-C siRNA, both the basal level of cytokine synthesis and LPS induced cytokine production was inhibited by 38% and 44% respectively compared to control cells (figure 19)

These data reveal that silencing tenascin-C in RA synovial fibroblasts reduces the synthesis of pro-inflammatory cytokines and suggest that ablation of tenascin-C expression is a viable strategy to inhibit inflammation in the synovium.

This work has established that blocking tenascin-C activity (with peptides) and tenascin-C expression (with siRNA) reduces inflammatory cytokine synthesis in human RA synovia. These data shows that tenascin-C blockade is of potential clinical benefit in treating RA and other inflammatory diseases.

### Example 6 - In vitro citrullination

Equal volumes of protein and 2x citrullination buffer (200 mM Tris HCI, pH 7.4, 20 mM CaCI2, 10 mM DTT) were mixed. 8.75 U rabbit skeletal PAD (product number P1584 from Sigma) per mg substrate protein was added and incubated for 3 hours at 37°C or 2 hours at 50°C. Citrullination was confirmed by size shift visualized by coomassie blue staining of SDS PAGE or by AMC detection.

The results shown in Figure 20 demonstrate that purified fibrinogen and FBG can be citrullinated *in vitro*.

*In vitro* citrullination of purified full length tenascin-C is shown by the results in Figure 21.

### Example 7 - AMC (anti-modified citrulline) detection of citrullinated proteins

Citrullination was detected using a protocol from an anti-citrulline (Modified) Detection Kit (Millipore (catalogue: 17-347)).
A. Nitrocellulose Blot Preparation
   1. Run SDS-PAGE and transfer to nitrocellulose. Wash blot with water 2 x 5 min.
   2. Incubate blot in 10ml 0.1% ovalbumin in TBS 15 min RT
   3. Wash with water 2 x 10 min
B. Modification of Citrulline Residues
   1. Mix 3ml Reagent A and 3ml Reagent B. Prepare just before use.
   2. Add modification buffer to blot in light-proof container, incubate at 37°C 5-7hrs.
   3. Rinse blot 4-5 times in water.
C. Detection of modified Citrulline Residues
   1. Block blot in freshly prepared 3% non-fat dried milk in TBS for 30 min-1hr.
   2. Incubate blot with 5-8 ml of 1:1000 dil of anti-modified citrulline antibody diluted in TBS-MLK overnight with agitation at 4°C (Seems to work with a 2-3hr incubation RT).
   3. Rinse blot 3x with water, then wash 1 x 15 min, then 3x5 min.
   4. Incubate the blot with 5-8 ml of 1:5000 dil of goat anti-rabbit HRP-conjugated IgG in TBS-MLK for 1 hr at RT with agitation.
   5. Wash blot as in step 3.
   6. Wash blot in TBS-0.05% Tween 20 for 3-5 min.
   7. Rinse blot in 4-5 changes of water.
   8. Use ECL-plus for detection.

Figure 22 shows confirmation of the citrullination of FBG and tenascin-C by western blot.

### Example 8 - FBG is citrullinated by PAD in vitro

Figure 23 shows FBG is citrullinated by PAD *in vitro*. In particular, an increased molecular weight of FBG in Coomassie-blue stained SDS PAGE and Western Blotting with an anti-modified citrulline specific antibody demonstrates that FBG is citrullinated by rPAD2, hPAD2 and hPAD4 in a dose- and time dependent manner.

### Example 9 - Demonstration that citrullination modulates the pro-inflammatory activity of tenascin-C

Primary human macrophages were isolated as described above and cultured at 1x10⁵ cells/well in RPMI 1640 containing 5% (v/v) FBS and 100 U/ml penicillin/streptomycin in 96-well tissue culture plates for 24h before stimulation. Cells were incubated for 24h at 37°C with no addition (UN), different concentrations (uM) of native FBG (nFBG) or cit FBG (cFBG) or citrullination buffer alone (CIT) or cit buffer plus PAD enzyme (CIT+PAD).

The viability of the cells was not significantly affected throughout the experimental time period when examined by the MTT cell viability assay (Sigma, Poole, UK). Supernatants were examined for the presence of the cytokines TNF-α by enzyme-linked immunosorbent assay (ELISA) according to the manufacturer's instructions (R&D systems). Absorbance was read on a spectrophotometric ELISA plate reader (Labsystems Multiscan Biochromic, Vantaa, Finland) and analyzed using the Ascent software program (Thermo Labsystems, Altrincham, UK).

Figure 24 shows that citrullination enhances cytokine production stimulated by FBG.

### Example 10 - Serum from RA patients react with citrullinated tenascin-C and citrullinated FBG unlike healthy controls

### SDS PAGE of citrullinated proteins

33.33ul of citrullination reaction (cit-TNC alone or cit-TNC combined with cit-FBG) was loaded into NuPAGE® Novex 4-12% Bis-tris gel using a 1.0 mm 2D single lane well.

### Western blot detection with RA serum

After electrophoresis proteins were transferred to nitrocellulose and the membrane blocked and then cut into strips (up to 8 strips ∼ 0.7 cm wide). Strips were incubated with serum from RA patients or normal healthy individuals diluted 1:100 in 5% milk TBS-tween. Strips were incubated in 15 ml falcon tubes on rollers 1 h room temperature, then washed 3 x 5 min, 1 x 15 min in tubes with TBS-Tween. Strips were then incubated with seondary mouse anti-human Ig 1:5000 1 h in room temperature, then washed 3 x 5 min, 1 x 15 min in the dish with TBS-tween. Strips were finally incubated with ECL reagents and exposed to film.

Figure 25A shows a coomassie stained gel of native (nTNC) and citrullinated (cTNC) purified human recombinant tenascin-C (top panel) and citrullination of tenascin-C demonstated by western blot with the AMC (Anti-modified citrulline) kit (bottom panel).

Figure 25B shows a representative western blot of cTNC probed with serum from RA patients (RA) or normal healthy controls (NH) (n=50). No reactivity was observed with any sera in blots of nTNC (not shown).

Figures 26 and 27 shows that serum from a subset of RA patients exhibits reactivity with citTNC (see RA patient samples in lane 4 on each gel).

Figure 28 shows that serum from normal healthy controls exhibit no reactivity with citTNC.

Figure 29 shows RA serum reactivity against citrullinated TNC and citrullinated FBG. A western blot of cTNC plus cFBG was run together in the same well, probed with serum from 7 different RA patients. Patient subsets were observed that reacted with full length TNC (320kD) but not FBG (lanes 4, 5) or patients that reacted with citrullinated FBG (27kd) but not full length TNC (lane 6).

Gels run with full length citrullinated TNC and with citrullinated FBG in the same lane show some patients react with full length TNC. Of those that do react with TNC only a subset also react with FBG thus they must by definition recognize a different domain.

### Example 11 - Defining the sites of citrullination by LC-MS/MS

With reference to figure 30 and table 3, citrullinated arginine residues were identifed by LC-MS/MS. From 17 Arginines, 5 are not citrullinated, 3 arginines are not covered by this LC-MS/MS analysis, and 9 arginine residues are modified to citrulline residues, independent of the enzyme used for citrullination.

**Table 3**

| **Position R** | **Sequence** | **m/z** | **Charge** |
|---|---|---|---|
| **55** | NGR(+.98)ENFYQNWK [SEQ ID NO: 33] | 728.8331 | 2+ |
| **72** | AYAAGFGDR(+.98)R [SEQ ID NO: 34] | 542.7619 | 2+ |
| **120** | TR(+.98)YKLK [SEQ ID NO: 35] | 405.2478 | 2+ |
| **169** | GAFWYR(+.98)RNC(+57.02)HR [SEQ ID NO: 36] | 684.3063 | 2+ |
| **173** | NC(+57.02)HR(+.98)VNLM(+15.99)GR [SEQ ID NO: 37] | 637.2995 | 2+ |
| **173** | NC(+57.02)HR(+.98)VNLMGR [SEQ ID NO: 38] | 629.3023 | 2+ |
| **173** | NC(+57.02)HR(+.98)VNLM(+15.99)GR [SEQ ID NO: 39] | 425.2011 | 3+ |
| **173** | N(+27.99)C(+57.02)HR(+.98)VNLMGR [SEQ ID NO: 40] | 643.2988 | 2+ |
| **209** | L(+57.02)R(+.98)PSNFR [SEQ ID NO: 41] | 474.2563 | 2+ |
| **214** | L(+27.99)RPSNFR(+.98)NLEGR [SEQ ID NO: 42] | 744.3896 | 2+ |
| **214** | LRPSNFR(+.98)NLEGR [SEQ ID NO: 43] | 730.3915 | 2+ |
| **214** | LRPSN(+.98)FR(+.98)NLEGR [SEQ ID NO: 44] | 487.5948 | 3+ |
| **214** | L(+27.99)RPSNFR(+.98)NLEGR [SEQ ID NO: 45] | 496.5952 | 3+ |
| **214** | LRPSNFR(+.98)NLEGRR [SEQ ID NO: 46] | 404.7247 | 4+ |
| **214** | LRPSNFR(+.98)NLEGR [SEQ ID NO: 47] | 487.2641 | 3+ |
| **219** | N(+27.99)LEGR(+.98)RK [SEQ ID NO: 48] | 451.2462 | 2+ |
| **219** | L(+27.99)RPSNFRNLEGR(+.98)R [SEQ ID NO: 49] | 548.6292 | 3+ |
| **214/219** | LRPSN(+.98)FR(+.98)NLEGR(+.98)R [SEQ ID NO: 50] | 539.9565 | 3+ |
| **214/219** | LRPSNFR(+.98)NLEGR(+.98) [SEQ ID NO: 51] | 730.8856 | 2+ |
| **214/219** | LRPSNFR(+.98)NLEGR(+.98)R [SEQ ID NO: 52] | 808.9345 | 2+ |
| **214/219** | LRPSNFR(+.98)NLEGR(+.98)R [SEQ ID NO: 53] | 539.6262 | 3+ |

### Example 12 - Identifying the citrullinated antibody epitope

With reference to figure 31, the antibody response in 20 RA and 20 control sera to 6 cyclic FBG peptides, encompassing citrulline residues was evaluated.

The peptides evaluated where as follows:
**R55:** CVFLRRKNG-R-ENFYQNWC [SEQ ID NO: 54]
**CIT55:** CVFLRRKNG-Cit-ENFYQNWC [SEQ ID NO: 55]
**R72:** CAYAAGFGD-R-REEFWLGLC [SEQ ID NO: 56]
**CIT72:** CAYAAGFGD-Cit-REEFWLGLC [SEQ ID NO:57]
**R120:** CFSVGDAKT-R-YKLKVEGYC [SEQ ID NO: 58]
**CIT120:** CFSVGDAKT-Cit-YKLKVEGYC [SEQ ID NO: 59]
**R169/173:** CKGAFWY-R-NCH-R-VNLMGRC [SEQ ID NO: 60]
**CIT169/173:** CKGAFWY-Cit-NCH-Cit-VNLMGRC [SEQ ID NO: 61]
**R209/214/219/220:** CEMKL-R-PSNF-R-NLEG-R-R-KRC [SEQ ID NO: 62]
**CIT209/214:** CEMKL-Cit-PSNF-Cit-NLEGRRKRC [SEQ ID NO: 63]
**CIT219/220:** CEMKLRPSNFRNLEG-Cit-Cit-KRC [SEQ ID NO: 64]

A strong antibody response towards citrulline containing peptides CIT55, CIT209/214 and CIT219/220 was detected in RA sera but not control sera, and no response was observed against arginine containing control peptides. Data from this small cohort shows that about 35% of RA patients will be positive for the CIT55 epitope, 20% for the CIT 209/214 and 30% for the CIT219/220 whilst none of the healthy controls exhibited positivity, defined using the 95th percentile of the normal group. The same calculations may be used to diagnose RA compared to a group of healthy controls and a similar percentage in these groups may be expected.

These data will enable the stratification of patients based on citFBG antibody positivity or on citFBG positivity. Those patients that were positive would be candidates for treatment with any agents designed to target the activity of citrullinated FBG. The presence of different epitopes also informs how to target citFBG - e.g. if CIT55 was present treatment may comprise blocking the immunogenic activity of FBG, but if the integrin binding site was citrullinated treatment may comprise blocking the action of this domain.

### Example 13 - Citrullination of FBG reduces cell adhesion of HDF and RAF

With reference to figure 32, FBG is proadhesive for human dermal fibroblasts (HDF) and synovial fibroblasts from RA joints (RAF). Citrullination of FBG reduces adhesion of HDF and RAF. Two arginines within the sequence known to bind integrin αvβ3 are citrullinated, therefore reduced adhesion may be due to reduced binding of cFBG to integrin αvβ3. Yokoyama et al. determined that this peptide sequence within FBG mediates cell adhesion via binding to cell surface avb3 integrins. Here it is shown 1) citrullination occurs *in vitro* in the integrin binding site, 2) this reduces cell adhesion, and 3) this happens in RASF meaning that, in an RA joint, citrullination of FBG at this site may reduce the adhesion of RASF. This can have many implications for disease progression, for example, it may promote cell migration (e.g. invasion into and degradation of healthy joint tissue), and it may facilitate RASF proliferation (thus perpetuating synovial hyperplasia).

### References

1. Smolen, J.S. & Maini, R.N. Interleukin-6: a new therapeutic target. Arthritis Res Ther 8 Suppl 2, S5 (2006).
2. Williams, R.O., Paleolog, E. & Feldmann, M. Cytokine inhibitors in rheumatoid arthritis and other autoimmune diseases. Curr Opin Pharmacol7, 412-417 (2007).
3. Brentano, F., Kyburz, D., Schorr, O., Gay, R. & Gay, S. The role of Toll-like receptor signalling in the pathogenesis of arthritis. Cell Immunol 233, 90-96 (2005).
4. O'Neill, L.A. Primer: Toll-like receptor signaling pathways-what do rheumatologists need to know? Nat Clin Pract Rheumatol (2008).
5. Matzinger, P. The danger model: a renewed sense of self. Science 296, 301-305 (2002).
6. Bianchi, M.E. DAMPs, PAMPs and alarmins: all we need to know about danger. J Leukoc Biol 81, 1-5 (2007).
7. Gordon, S. Pattern recognition receptors: doubling up for the innate immune response. Cell 111, 927-930 (2002).
8. Medzhitov, R. & Janeway, C.A., Jr. Decoding the patterns of self and nonself by the innate immune system. Science 296, 298-300 (2002).
9. Radstake, T.R., et al. Expression of toll-like receptors 2 and 4 in rheumatoid synovial tissue and regulation by proinflammatory cytokines interleukin-12 and interleukin-18 via interferon-gamma. Arthritis Rheum 50, 3856-3865 (2004).
10. Roelofs, M.F., et al. The expression of toll-like receptors 3 and 7 in rheumatoid arthritis synovium is increased and costimulation of toll-like receptors 3, 4, and 7/8 results in synergistic cytokine production by dendritic cells. Arthritis Rheum 52, 2313-2322 (2005).
11. Sacre, S.M., et al. The Toll-like receptor adaptor proteins MyD88 and Mal/TIRAP contribute to the inflammatory and destructive processes in a human model of rheumatoid arthritis. Am J Pathol 170, 518-525 (2007).
12. Choe, J.Y., Crain, B., Wu, S.R. & Corr, M. Interleukin 1 receptor dependence of serum transferred arthritis can be circumvented by toll-like receptor 4 signaling. J Exp Med 197, 537-542 (2003).
13. Lee, E.K., Kang, S.M., Paik, D.J., Kim, J.M. & Youn, J. Essential roles of Toll-like receptor-4 signaling in arthritis induced by type II collagen antibody and LPS. Int Immunol 17, 325-333 (2005).
14. Abdollahi-Roodsaz, S., et al. Inhibition of Toll-like receptor 4 breaks the inflammatory loop in autoimmune destructive arthritis. Arthritis Rheum 56, 2957-2967 (2007).
15. Vanags, D., et al. Therapeutic efficacy and safety of chaperonin 10 in patients with rheumatoid arthritis: a double-blind randomised trial. Lancet 368, 855-863 (2006).
16. Chiquet-Ehrismann, R. & Chiquet, M. Tenascins: regulation and putative functions during pathological stress. J Pathol 200, 488-499 (2003).
17. Cutolo, M., Picasso, M., Ponassi, M., Sun, M.Z. & Balza, E. Tenascin and fibronectin distribution in human normal and pathological synovium. J Rheumatol 19, 1439-1447 (1992).
18. McCachren, S.S. & Lightner, V.A. Expression of human tenascin in synovitis and its regulation by interleukin-1. Arthritis Rheum 35, 1185-1196 (1992).
19. Salter, D.M. Tenascin is increased in cartilage and synovium from arthritic knees. Br J Rheumatol 32, 780-786 (1993).
20. Chevalier, X., Groult, N., Larget-Piet, B., Zardi, L. & Hornebeck, W. Tenascin distribution in articular cartilage from normal subjects and from patients with osteoarthritis and rheumatoid arthritis. Arthritis Rheum 37, 1013-1022 (1994).
21. Hasegawa, M., et al. Expression of large tenascin-C splice variants in synovial fluid of patients with rheumatoid arthritis. J Orthop Res 25, 563-568 (2007).
22. Orend, G. Potential oncogenic action of tenascin-C in tumorigenesis. Int J Biochem Cell Biol 37, 1066-1083 (2005).
23. Brackertz, D., Mitchell, G.F. & Mackay, I.R. Antigen-induced arthritis in mice. I. Induction of arthritis in various strains of mice. Arthritis Rheum 20, 841-850 (1977).
24. Brennan, F.M., Chantry, D., Jackson, A., Maini, R. & Feldmann, M. Inhibitory effect of TNF alpha antibodies on synovial cell interleukin-1 production in rheumatoid arthritis. Lancet 2, 244-247 (1989).
25. Smiley, S.T., King, J.A. & Hancock, W.W. Fibrinogen stimulates macrophage chemokine secretion through toll-like receptor 4. J Immunol 167, 2887-2894 (2001).
26. Fitzgerald, K.A., Rowe, D.C. & Golenbock, D.T. Endotoxin recognition and signal transduction by the TLR4/MD2-complex. Microbes Infect 6, 1361-1367 (2004).
27. Jiang, Z., et al. CD14 is required for MyD88-independent LPS signaling. Nat Immunol 6, 565-570 (2005).
28. Coats, S.R., Do, C.T., Karimi-Naser, L.M., Braham, P.H. & Darveau, R.P. Antagonistic lipopolysaccharides block E. coli lipopolysaccharide function at human TLR4 via interaction with the human MD-2 lipopolysaccharide binding site. Cell Microbiol 9, 1191-1202 (2007).
29. Siri, A., et al. Human tenascin: primary structure, pre-mRNA splicing patterns and localization of the epitopes recognized by two monoclonal antibodies. Nucleic Acids Res 19, 525-531 (1991).
30. Gondokaryono, S.P., et al. The extra domain A of fibronectin stimulates murine mast cells via toll-like receptor 4. J Leukoc Biol 82, 657-665 (2007).
31. Taylor, K.R., et al. Recognition of hyaluronan released in sterile injury involves a unique receptor complex dependent on Toll-like receptor 4, CD44, and MD-2. J Biol Chem 282, 18265-18275 (2007).
32. Kim, H.M., et al. Crystal structure of the TLR4-MD-2 complex with bound endotoxin antagonist Eritoran. Cell 130, 906-917 (2007).
33. Schaefer, L., et al. The matrix component biglycan is proinflammatory and signals through Toll-like receptors 4 and 2 in macrophages. J Clin Invest 115, 2223-2233 (2005).
34. Foell, D., Wittkowski, H. & Roth, J. Mechanisms of disease: a 'DAMP' view of inflammatory arthritis. Nat Clin Pract Rheumatol 3, 382-390 (2007).
35. Taniguchi, N., et al. High mobility group box chromosomal protein 1 plays a role in the pathogenesis of rheumatoid arthritis as a novel cytokine. Arthritis Rheum 48, 971-981 (2003).
36. Pullerits, R., et al. High mobility group box chromosomal protein 1, a DNA binding cytokine, induces arthritis. Arthritis Rheum 48, 1693-1700 (2003).
37. Kokkola, R., et al. Successful treatment of collagen-induced arthritis in mice and rats by targeting extracellular high mobility group box chromosomal protein 1 activity. Arthritis Rheum 48, 2052-2058 (2003).
38. Gutowski, N.J., Newcombe, J. & Cuzner, M.L. Tenascin-R and C in multiple sclerosis lesions: relevance to extracellular matrix remodelling. Neuropathol Appl Neurobiol 25, 207-214 (1999).
39. Amin, K., et al. Inflammation and structural changes in the airways of patients with primary Sjogren's syndrome. Respir Med 95, 904-910 (2001).
40. Loots, M.A., et al. Differences in cellular infiltrate and extracellular matrix of chronic diabetic and venous ulcers versus acute wounds. J Invest Dermatol 111, 850-857 (1998).
41. Lange, K., et al. Endothelin receptor type B counteracts tenascin-C-induced endothelin receptor type A-dependent focal adhesion and actin stress fiber disorganization. Cancer Res 67, 6163-6173 (2007).
42. Saga, Y., Yagi, T., Ikawa, Y., Sakakura, T. & Aizawa, S. Mice develop normally without tenascin. Genes Dev 6, 1821-1831 (1992).
43. Hoshino, K., et al. Cutting edge: Toll-like receptor 4 (TLR4)-deficient mice are hyporesponsive to lipopolysaccharide: evidence for TLR4 as the Lps gene product. J Immunol 162, 3749-3752 (1999).
44. Takeuchi, O., et al. Differential roles of TLR2 and TLR4 in recognition of gram-negative and gram-positive bacterial cell wall components. Immunity 11, 443-451 (1999).
45. Keystone, E.C., Schorlemmer, H.U., Pope, C. & Allison, A.C. Zymosan-induced arthritis: a model of chronic proliferative arthritis following activation of the alternative pathway of complement. Arthritis Rheum 20, 1396-1401 (1977).
46. van Lent, P.L., et al. Fcgamma receptors directly mediate cartilage, but not bone, destruction in murine antigen-induced arthritis: uncoupling of cartilage damage from bone erosion and joint inflammation. Arthritis Rheum 54, 3868-3877 (2006).
47. Foxwell, B., et al. Efficient adenoviral infection with IkappaB alpha reveals that macrophage tumor necrosis factor alpha production in rheumatoid arthritis is NF-kappaB dependent. Proc Natl Acad Sci USA 95, 8211-8215 (1998).
48. Kurt-Jones, E.A., et al. Use of murine embryonic fibroblasts to define Toll-like receptor activation and specificity. J Endotoxin Res 10, 419-424 (2004).
49. Todaro, G.J. & Green, H. Quantitative studies of the growth of mouse embryo cells in culture and their development into established lines. J Cell Biol 17, 299-313 (1963).
50. Butler, D.M., Malfait, A.M., Maini, R.N., Brennan, F.M. & Feldmann, M. Anti-IL-12 and anti-TNF antibodies synergistically suppress the progression of murine collagen-induced arthritis. Eur J Immunol 29, 2205-2212 (1999).
51. Ho, S.N., Hunt, H.D., Horton, R.M., Pullen, J.K. & Pease, L.R. Site-directed mutagenesis by overlap extension using the polymerase chain reaction. Gene 77, 51-59 (1989).
52. Clark, R.A., Erickson, H.P. & Springer, T.A. Tenascin supports lymphocyte rolling. J Cell Biol 137, 755-765 (1997).
53. El-Karef, A., et al. Deficiency of tenascin-C attenuates liver fibrosis in immune-mediated chronic hepatitis in mice. J Pathol 211, 86-94 (2007).
54. Loike, J.D., Cao, L., Budhu, S., Hoffman, S. & Silverstein, S.C. Blockade of alpha 5 beta 1 integrins reverses the inhibitory effect of tenascin on chemotaxis of human monocytes and polymorphonuclear leukocytes through three-dimensional gels of extracellular matrix proteins. J Immunol 166, 7534-7542 (2001).
55. Talts, J.F., Wirl, G., Dictor, M., Muller, W.J. & Fassler, R. tenascin-C modulates tumor stroma and monocyte/macrophage recruitment but not tumor growth or metastasis in a mouse strain with spontaneous mammary cancer. J Cell Sci 112 (Pt 12), 1855-1864 (1999).
56. Jones (2000) Matrix Biol., 19, 581-96
57. Harandl (2009) Expert Review of Vaccines, 8, 293-298
58. McIntyre (2006) BMC Biotechnol. 6: 1
59. Paddison (2002) Genes Dev. 16 (8): 948-58
60. Andreakos (2004) Blood, 103, 2229-37
61. Goh, F.G., Piccinini, A.M., Krausgruber, T., Udalova, I.A. & Midwood, K.S. Transcriptional regulation of the endogenous danger signal tenascin-C: a novel autocrine loop in inflammation. J Immunol 184, 2655-2662 (2010).
62. Midwood, K. et al. Tenascin-C is an endogenous activator of Toll-like receptor 4 that is essential for maintaining inflammation in arthritic joint disease. Nat Med 15, 774-780 (2009).
63. LaFleur, D.W. et al. Aortic smooth muscle cells interact with tenascin-C through its fibrinogen-like domain. J Biol Chem 272, 32798-32803 (1997).
64. Taylor, P.C. & Feldmann, M. Anti-TNF biologic agents: still the therapy of choice for rheumatoid arthritis. Nat Rev Rheumatol 5, 578-582 (2009).
65. Yokoyama, K., Erickson, H.P., Ikeda, Y. & Takada, Y. Identification of amino acid sequences in fibrinogen gamma -chain and tenascin C C-terminal domains critical for binding to integrin alpha vbeta 3. J Biol Chem 275, 16891-16898 (2000).
66. Sacre SM, Lo A, Gregory B, Simmonds RE, Williams L, Feldmann M, Brennan FM, Foxwell BM. J Immunol. 2008 Dec 1;181(11):8002-9.
67. Wegner N, Lundberg K, Kinloch A, Fisher B, Malmstrom V, Feldmann M, Venables PJ: Autoimmunity to specific citrullinated proteins gives the first clues to the etiology of rheumatoid arthritis. Immunol Rev 2010, 233:34-54.
68. Fisher BA, Plant D, Brode M, van Vollenhoven RF, Mathsson L, Symmons D, Lundberg K, Ronnelid J, Venables PJ: Antibodies to citrullinated alpha-enolase peptide 1 and clinical and radiological outcomes in rheumatoid arthritis. Ann Rheum Dis 2011, 70:1095-1098.
69. Kinloch A, Tatzer V, Wait R, Peston D, Lundberg K, Donatien P, Moyes D, Taylor PC, Venables PJ: Identification of citrullinated alpha-enolase as a candidate autoantigen in rheumatoid arthritis. Arthritis Res Ther2005, 7:R1421-1429.
70. Kinloch AJ, Alzabin S, Brintnell W, Wilson E, Barra L, Wegner N, Bell DA, Cairns E, Venables PJ: Immunization with Porphyromonas gingivalis enolase induces autoimmunity to mammalian alpha-enolase and arthritis in DR4-IE-transgenic mice. Arthritis Rheum 2011, 63:3818-3823.
71. Lundberg K, Kinloch A, Fisher BA, Wegner N, Wait R, Charles P, Mikuls TR, Venables PJ: Antibodies to citrullinated alpha-enolase peptide 1 are specific for rheumatoid arthritis and cross-react with bacterial enolase. Arthritis Rheum 2008, 58:3009-3019.
72. Snir O, Widhe M, Hermansson M, von Spee C, Lindberg J, Hensen S, Lundberg K, Engstrom A, Venables PJ, Toes RE, Holmdahl R, Klareskog L, Malmstrom V: Antibodies to several citrullinated antigens are enriched in the joints of rheumatoid arthritis patients. Arthritis Rheum 2010, 62:44-52.
73. Snir O, Widhe M, von Spee C, Lindberg J, Padyukov L, Lundberg K, Engstrom A, Venables PJ, Lundeberg J, Holmdahl R, Klareskog L, Malmstrom V: Multiple antibody reactivities to citrullinated antigens in sera from patients with rheumatoid arthritis: association with HLA-DRB1 alleles. Ann Rheum Dis 2009, 68:736-743.
74. Wegner N, Wait R, Sroka A, Eick S, Nguyen KA, Lundberg K, Kinloch A, Culshaw S, Potempa J, Venables PJ: Peptidylarginine deiminase from Porphyromonas gingivalis citrullinates human fibrinogen and alpha-enolase: implications for autoimmunity in rheumatoid arthritis. Arthritis Rheum 2010, 62:2662-2672.
75. Kuhn KA, Kulik L, Tomooka B, Braschler KJ, Arend WP, Robinson WH, Holers VM: Antibodies against citrullinated proteins enhance tissue injury in experimental autoimmune arthritis. J Clin Invest 2006, 116:961-973.
76. Uysal H, Bockermann R, Nandakumar KS, Sehnert B, Bajtner E, Engstrom A, Serre G, Burkhardt H, Thunnissen MM, Holmdahl R: Structure and pathogenicity of antibodies specific for citrullinated collagen type II in experimental arthritis. J Exp Med 2009, 206:449-462.
77. Clavel C, Nogueira L, Laurent L, lobagiu C, Vincent C, Sebbag M, Serre G: Induction of macrophage secretion of tumor necrosis factor alpha through Fcgamma receptor IIa engagement by rheumatoid arthritis-specific autoantibodies to citrullinated proteins complexed with fibrinogen. Arthritis Rheum 2008, 58:678-688. Page 10 of 10
78. Sokolove J, Zhao X, Chandra PE, Robinson WH: Immune complexes containing citrullinated fibrinogen costimulate macrophages via Toll-like receptor 4 and Fcgamma receptor. Arthritis Rheum 2011, 63:53-62.
79. Midwood K, Sacre S, Piccinini AM, Inglis J, Trebaul A, Chan E, Drexler S, Sofat N, Kashiwagi M, Orend G, Brennan F, Foxwell B: Tenascin-C is an endogenous activator of Toll-like receptor 4 that is essential for maintaining inflammation in arthritic joint disease. Nat Med 2009, 15:774-780.
80. Ruhmann M, Piccinini AM, Kong PL, Midwood KS: Endogenous activation of adaptive immunity: tenascin-C drives interleukin-17 synthesis in murine arthritic joint disease. Arthritis Rheum 2012, 64:2179-2190.
81. Goh FG, Piccinini AM, Krausgruber T, Udalova IA, Midwood KS: Transcriptional regulation of the endogenous danger signal tenascin-C: a novel autocrine loop in inflammation. J Immunol 2010, 184:2655-2662.
82. Page TH, Charles PJ, Piccinini AM, Nicolaidou V, Taylor PC, Midwood KS: Raised circulating tenascin-C in rheumatoid arthritis. Arthritis Res Ther 2012, 14:R260.

## Claims

1. An agent for modulation of a chronic inflammatory response wherein the agent modulates the biological activity of citrullinated tenascin-C; wherein the agent is an antibody or antigen binding fragment thereof which binds specifically to citrullinated tenascin-C.

2. An agent according to claim 1 wherein the antibody or antigen-binding fragment thereof is selected from the group consisting of Fv fragments, scFv fragments, Fab, single variable domains and domain antibodies.

3. An agent as claimed in any of claims 1-2 wherein the antibody or antigen-binding fragment thereof is humanised.

4. An agent as claimed in any of claims 1-3 wherein the antibody or antigen-binding fragment thereof has specificity for the FBG domain of citrullinated tenascin-

5. An agent as claimed in any previous claim wherein the citrullinated tenascin-C is citrullinated at the FBG domain.

6. An agent as claimed in any one of claims 1 to 4 wherein the citrullinated tenascin-C is citrullinated at only the FBG domain.

7. An agent as claimed in any previous claim wherein the specific citrullinated residue(s) of the citrullinated tenascin-C comprise one or more of residues 55, 209, 214, 219, 220, 50, 51, 72, 120, 169, 173 and 222 as numbered in SEQ ID NO: 70.

8. An agent as claimed in any of claims 1-5 wherein the citrullinated tenascin-C is citrullinated at one or more domains other than the FBG domain (e.g. the fibronectin type III like repeats).

9. A composition comprising an agent as defined in any of claims 1-8 and a pharmaceutically acceptable carrier, excipient and/or diluent.

10. A composition as claimed in claim 9 further comprising at least one other agent, for example an anti-inflammatory agent, a statin, a biological agent (biologicals), an immunosuppressive agent, a salicylate and/or a microbicidal agent..

11. A composition as claimed in claim 10 wherein the anti-inflammatory agent is selected from the group consisting non-steroidal anti-inflammatories (NSAIDs), corticosteroids, disease-modifying antirheumatic drugs (DMARDs) or immunosuppressants.

12. An agent or composition as defined in claims 1-8 and 9-11 for use in treatment.

13. An agent or composition as defined in claim 12 for use in the treatment of a chronic inflammatory condition.

14. An agent or composition for use as claimed in claim 13 wherein the chronic inflammatory condition is in a subject possessing citrullinated tenascin-C or autoantibodies with specificity for citrullinated tenascin-C.

15. An agent or composition for use as claimed in claim 14 wherein the citrullinated tenascin-C or autoantibodies are to be found in a sample of one or more of blood, synovial fluid or joint tissue derived from the subject.

16. An agent or composition for use as claimed in claims 14-15 wherein the chronic inflammatory response is associated with a condition **characterised by** inappropriate inflammation.

17. An agent, composition for use as claimed in claims 14-16 wherein the chronic inflammatory response is associated with rheumatoid arthritis (RA), autoimmune conditions, inflammatory bowel diseases (including Crohn's disease and ulcerative colitis), non-healing wounds, multiple sclerosis, cancer, atherosclerosis, sjogrens disease, diabetes, lupus erythrematosus (including systemic lupus erythrematosus), asthma, fibrotic diseases (including liver cirrhosis), pulmonary fibrosis, UV damage, psoriasis, ankylosing spondylitis and cardiovascular disease.

## Patentansprüche

1. Agens zur Modulation einer chronischen Entzündungsreaktion, wobei das Agens die biologische Aktivität von citrulliniertem Tenascin-C moduliert; wobei das Agens ein Antikörper oder ein antigenbindendes Fragment davon ist, der bzw. das spezifisch an citrulliniertes Tenascin-C bindet.

2. Agens nach Anspruch 1, wobei der Antikörper oder das antigenbindende Fragment davon aus der Gruppe bestehend aus Fv-Fragmenten, scFv-Fragmenten, Fab, einzelnen variablen Domänen und Domänenantikörpern ausgewählt ist.

3. Agens nach einem der Ansprüche 1-2, wobei der Antikörper oder das antigenbindende Fragment davon humanisiert ist.

4. Agens nach einem der Ansprüche 1-3, wobei der Antikörper oder das antigenbindende Fragment davon Spezifität für die FBG-Domäne von citrulliniertem Tenascin aufweist.

5. Agens nach einem vorhergehenden Anspruch, wobei das citrullinierte Tenascin-C an der FBG-Domäne citrulliniert ist.

6. Agens nach einem der Ansprüche 1 bis 4, wobei das citrullinierte Tenascin-C nur an der FBG-Domäne citrulliniert ist.

7. Agens nach einem vorhergehenden Anspruch, wobei der bzw. die spezifischen citrullinierten Reste des citrullinierten Tenascin-C einen oder mehrere der Reste 55, 209, 214, 219, 220, 50, 51, 72, 120, 169, 173 und 222, wie in SEQ ID Nr. 70 nummeriert, umfasst bzw. umfassen.

8. Agens nach einem der Ansprüche 1-5, wobei das citrullinierte Tenascin-C an einer oder mehreren Domänen, bei der bzw. denen es sich nicht um die FBG-Domäne handelt (z. B. die Fibronektin-III-ähnlichen Wiederholungen), citrulliniert ist.

9. Zusammensetzung, die ein Agens nach einem der Ansprüche 1-8 und einen pharmazeutisch akzeptablen Trägerstoff, einen pharmazeutisch akzeptablen Hilfsstoff und/oder ein pharmazeutisch akzeptables Verdünnungsmittel umfasst.

10. Zusammensetzung nach Anspruch 9, die weiterhin mindestens ein anderes Agens umfasst, beispielsweise ein entzündungshemmendes Agens, ein Statin, ein biologisches Agens (Biologicals), ein immunsuppressives Agens, ein Salicylat und/oder ein mikrobizides Agens.

11. Zusammensetzung nach Anspruch 10, wobei das entzündungshemmende Agens aus der Gruppe bestehend aus nichtsteroidalen Entzündungshemmern (NSAID), Kortikosteroiden, krankheitsmodifizierenden Antirheumatika (DMARD) oder Immunsuppressiva ausgewählt ist.

12. Agens oder Zusammensetzung nach den Ansprüchen 1-8 und 9-11 zur Verwendung bei einer Behandlung.

13. Agens oder Zusammensetzung nach Anspruch 12 zur Verwendung bei der Behandlung eines chronischen Entzündungszustands.

14. Agens oder Zusammensetzung zur Verwendung nach Anspruch 13, wobei der chronische Entzündungszustand in einem Probanden ist, der über citrulliniertes Tenascin-C oder Autoantikörper mit Spezifität für citrulliniertes Tenascin-C verfügt.

15. Agens oder Zusammensetzung zur Verwendung nach Anspruch 14, wobei das citrullinierte Tenascin-C oder die Autoantikörper in einer Probe von einem oder mehreren von Blut, Synovia oder Gelenkgewebe, das bzw. die von dem Probanden stammt, vorzufinden ist bzw. sind.

16. Agens oder Zusammensetzung zur Verwendung nach den Ansprüchen 14-15, wobei die chronische Entzündungsreaktion mit einem Zustand assoziiert ist, der durch eine ungünstige Entzündung gekennzeichnet ist.

17. Agens, Zusammensetzung zur Verwendung nach den Ansprüchen 14-16, wobei die chronische Entzündungsreaktion mit rheumatoider Arthritis (RA), Autoimmunzuständen, entzündlichen Darmerkrankungen (einschließlich Morbus Crohn und Colitis ulcerosa), nicht heilenden Wunden, multipler Sklerose, Krebs, Atherosklerose, Sjögren-Syndrom, Diabetes, Lupus erythematodes (einschließlich systemischem Lupus erythematodes), Asthma, Fibroseerkrankungen (einschließlich Leberzirrhose), Lungenfibrose, UV-Schädigung, Psoriasis, Morbus Bechterew und Herz-Kreislauf-Erkrankung assoziiert ist.

## Revendications

1. Agent pour la modulation d'une réponse inflammatoire chronique, l'agent modulant l'activité biologique de la ténascine-C citrullinée, l'agent étant un anticorps ou un fragment de liaison à l'antigène de celui-ci qui se lie spécifiquement à la ténascine-C citrullinée.

2. Agent selon la revendication 1, dans lequel l'anticorps ou le fragment de liaison à l'antigène de celui-ci est choisi dans le groupe consistant en les fragments Fv, les fragments scFv, Fab, les domaines variables uniques et les anticorps à domaines.

3. Agent selon l'une quelconque des revendications 1 à 2, dans lequel l'anticorps ou le fragment de liaison à l'antigène de celui-ci est humanisé.

4. Agent selon l'une quelconque des revendications 1 à 3, dans lequel l'anticorps ou le fragment de liaison à l'antigène de celui-ci, a une spécificité pour le domaine FBG de la ténascine-C citrullinée.

5. Agent selon l'une quelconque des revendications précédentes, dans lequel la ténascine-C citrullinée est citrullinée au niveau du domaine FBG.

6. Agent selon l'une quelconque des revendications 1 à 4, dans lequel la ténascine-C citrullinée est citrullinée uniquement au niveau du domaine FBG.

7. Agent selon l'une quelconque des revendications précédentes dans lequel le ou les résidus citrullinés spécifiques de la ténascine-C citrullinée comprennent au moins l'un des résidus 55, 209, 214, 219, 220, 50, 51, 72, 120, 169, 173 et 222 tels que numérotés dans la SEQ ID NO : 70.

8. Agent selon l'une quelconque des revendications 1 à 5, dans lequel la ténascine-C citrullinée est citrullinée au niveau d'un ou plusieurs domaines autres que le domaine FBG (par exemple, les répétitions de type fibronectine de type III) .

9. Composition comprenant un agent tel que défini à l'une quelconque des revendications 1 à 8 et un support, excipient et/ou diluant pharmaceutiquement acceptable.

10. Composition selon la revendication 9, comprenant en outre au moins un autre agent, par exemple un agent anti-inflammatoire, une statine, un agent biologique (produits biologiques), un agent immunosuppresseur, un salicylate et/ou un agent microbicide.

11. Composition selon la revendication 10, dans laquelle l'agent anti-inflammatoire est choisi dans le groupe consistant en les anti-inflammatoires non-stéroïdiens (NSAID), les corticostéroïdes, les médicaments antirhumatismaux modificateurs de la maladie (DMARD) ou les immunosuppresseurs.

12. Agent ou composition tel(le) que défini(e)dans les revendications 1 à 8 et 9 à 11, pour l'utilisation en thérapie.

13. Agent ou composition tel(le) que défini(e) dans la revendication 12, pour l'utilisation dans le traitement d'un état inflammatoire chronique.

14. Agent ou composition pour l'utilisation telle que revendiquée dans la revendication 13, dans lequel ou laquelle l'état inflammatoire chronique est chez un sujet possédant de la ténascine-C citrullinée ou des auto-anticorps ayant une spécificité pour la ténascine-C citrullinée.

15. Agent ou composition pour l'utilisation telle que revendiquée dans la revendication 14, dans lequel ou laquelle la ténascine-C citrullinée ou les auto-anticorps doivent être trouvés dans un échantillon d'un ou plusieurs parmi le sang, le liquide synovial ou le tissu d'articulation prélevés à partir du sujet.

16. Agent ou composition pour l'utilisation telle que revendiquée dans les revendications 14 et 15, dans lequel ou laquelle la réponse inflammatoire chronique est associée avec un état **caractérisé par** une inflammation inappropriée.

17. Agent, composition pour l'utilisation telle que revendiquée dans les revendications 14 à 16, dans lequel ou laquelle la réponse inflammatoire chronique est associée à la polyarthrite rhumatoïde (RA), les états auto-immuns, les maladies intestinales inflammatoires (comprenant la maladie de Crohn et la colite ulcéreuse), les plaies qui ne cicatrisent pas, la sclérose en plaques, le cancer, l'athérosclérose, la maladie de Sjögren, le diabète, le lupus érythémateux (comprenant le lupus érythémateux disséminé), l'asthme, les maladies fibrotiques (comprenant la cirrhose hépatique), la fibrose pulmonaire, les dommages causés par les rayons UV, le psoriasis, la spondylarthrite ankylosante et les maladies cardiovasculaires.
